(19) Europäisches Patentamt / European Patent Office / Office européen des brevets

(11) **EP 4 667 469 A1**

(12) **EUROPEAN PATENT APPLICATION**
published in accordance with Art. 153(4) EPC

(43) Date of publication:
**24.12.2025 Bulletin 2025/52**

(21) Application number: **24756346.3**

(22) Date of filing: **16.02.2024**

(51) International Patent Classification (IPC):
***C07D 487/08*** *(2006.01)* ***C07D 491/08*** *(2006.01)*
***A61K 31/395*** *(2006.01)* ***A61P 35/00*** *(2006.01)*

(52) Cooperative Patent Classification (CPC):
**A61K 31/395; A61P 35/00; C07D 487/08; C07D 491/08**

(86) International application number:
**PCT/CN2024/077255**

(87) International publication number:
**WO 2024/169976 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.02.2023 CN 202310134195**
**31.01.2024 CN 202410136766**

(71) Applicant: **Chia Tai Tianqing Pharmaceutical Group Co., Ltd.**
**Lianyungang, Jiangsu 222062 (CN)**

(72) Inventors:
- **WANG, Bin**
**Lianyungang, Jiangsu 222062 (CN)**
- **FENG, Weiwei**
**Lianyungang, Jiangsu 222062 (CN)**
- **LIU, Fei**
**Lianyungang, Jiangsu 222062 (CN)**
- **YAO, Yiyan**
**Lianyungang, Jiangsu 222062 (CN)**
- **XU, Hongjiang**
**Lianyungang, Jiangsu 222062 (CN)**

(74) Representative: **v. Bezold & Partner Patentanwälte - PartG mbB**
**Ridlerstraße 57**
**80339 München (DE)**

(54) **COMPOUND CONTAINING TRIFLUOROMETHYLSULFONYL**

(57) The present disclosure relates to the field of pharmaceutical chemistry, relates to a compound containing trifluoromethylsulfonyl, and specifically relates to a compound of formula (I) or a pharmaceutically acceptable salt thereof, a preparation method therefor, a pharmaceutical composition containing the compound, and a use thereof in treating tumor diseases.

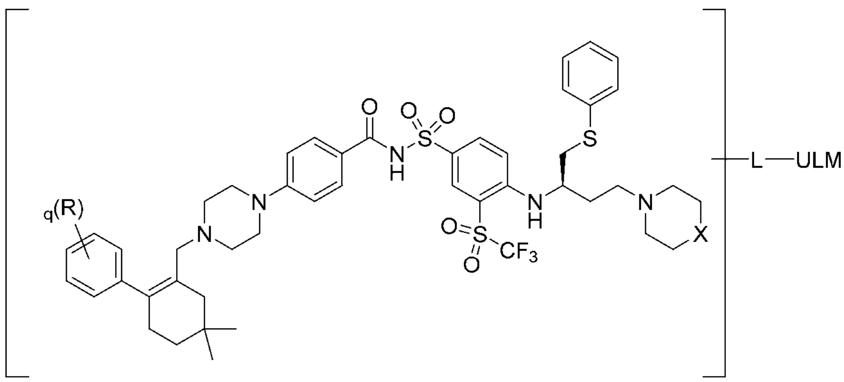

Formula I

EP 4 667 469 A1

## Description

### CROSS-REFERENCE TO RELATED APPLICATIONS

The present application claims priority and benefit to the Chinese Patent Application No. 202310134195.8 filed with China National Intellectual Property Administration on February 17, 2023, and the Chinese Patent Application No. 202410136766.6 filed with China National Intellectual Property Administration on January 31, 2024, which are incorporated herein by reference in their entirety.

### TECHNICAL FIELD

The present disclosure relates to a trifluoromethanesulfonyl-containing compound, a preparation method therefor, a pharmaceutical composition comprising the compound, and use thereof for treating tumor diseases.

### BACKGROUND

The B-cell lymphoma 2 (Bcl-2) protein family, composed of pro-apoptotic and anti-apoptotic members, plays a critical role in determining cell fate by regulating the intrinsic apoptotic pathway. Anti-apoptotic Bcl-2 family proteins (such as Bcl-2, Bcl-xL, Bcl-W, and Mcl-1) are upregulated in many cancers, and are associated with tumor initiation and progression, as well as resistance to chemotherapy and targeted therapy. Since the growth of most solid tumors does not rely on BCL-2 protein, inhibitors that only target BCL-2 have no significant efficacy in the treatment of solid tumors. The expression of BCL-XL protein is significantly increased in many leukemia cells and solid tumors. Studies have shown that BCL-XL expression in tumor tissues is positively correlated with tumor drug resistance, so that targeting BCL-XL is a potential ideal molecular target for anti-tumor therapy.

The proteolysis targeting chimera (PROTAC) molecule is a bifunctional compound capable of binding to a target protein and E3 ubiquitin ligase simultaneously. Such compounds can induce the target protein to be recognized by proteasomes of cells, cause the degradation of the target protein, and effectively reduce the content of the target protein in the cells. The introduction of ligands capable of binding to different target proteins into PROTAC molecules has made it possible to apply the PROTAC technology to the treatment of various diseases, and this technology has also received much attention in recent years.

### SUMMARY

The present disclosure relates to a compound of formula I, a stereoisomer thereof, or a pharmaceutically acceptable salt thereof:

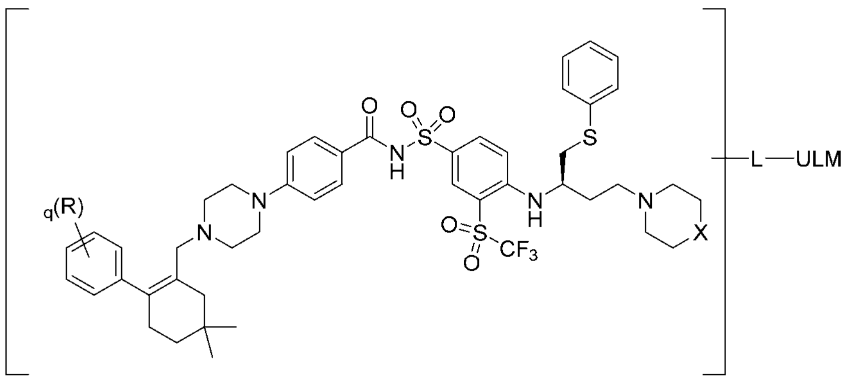

formula I

wherein

R is selected from the group consisting of OH, $NH_2$, CN, halogen, and $C_{1-6}$ alkyl optionally substituted with one or more OH, $NH_2$, CN, or halogens;
X is selected from the group consisting of $CH_2$, NH, and O;
L is a connecting group;
ULM is

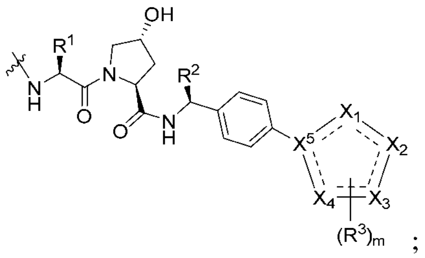

wherein $R^1$ is selected from $C_{1-6}$ alkyl optionally substituted with one or more halogens;
$R^2$ is selected from $C_{1-6}$ alkyl optionally substituted with one or more OH, $NH_2$, CN, or halogens;
$X^1$, $X^2$, $X^3$, $X^4$, and $X^5$ are each independently selected from the group consisting of CH, C, N, NH, O, and S; each $R^3$ is independently selected from the group consisting of OH, $NH_2$, CN, halogen, $C_{1-6}$ alkyl, and $C_{1-6}$ alkoxy, wherein the $NH_2$, $C_{1-6}$ alkyl, or $C_{1-6}$ alkoxy is optionally substituted with one or more OH, $NH_2$, CN, or halogens; m and q are each independently selected from the group consisting of 0, 1, 2, and 3;
the proviso is that ULM is not

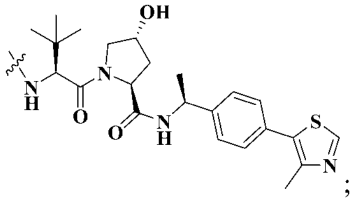

each R, X, L, $R^1$, $R^2$, $X^1$, $X^2$, $X^3$, $X^4$, or $X^5$ is independently optionally substituted with one or more substituents. The present disclosure relates to a compound of formula I, a stereoisomer thereof, or a pharmaceutically acceptable salt thereof:

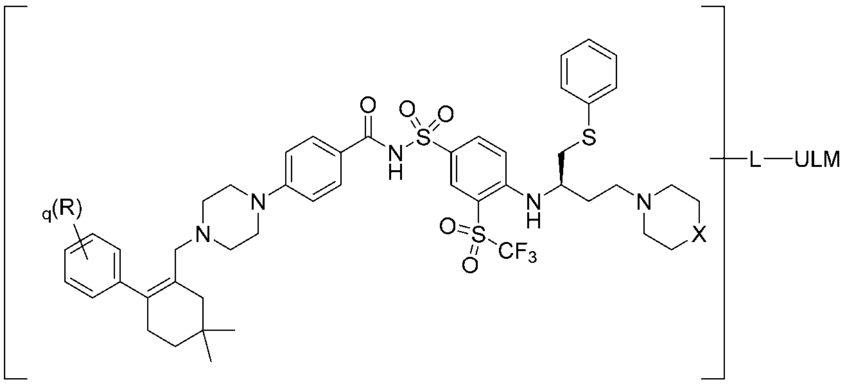

formula I

wherein
R is selected from the group consisting of OH, $NH_2$, CN, halogen, and $C_{1-6}$ alkyl optionally substituted with one or more OH, $NH_2$, CN, or halogens;
X is selected from the group consisting of $CH_2$, NH, and O;
L is a connecting group;
ULM is

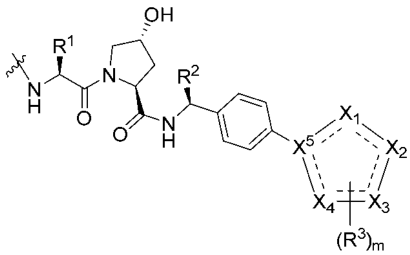
;

wherein $R^1$ is selected from $C_{1-6}$ alkyl optionally substituted with one or more halogens;
$R^2$ is selected from $C_{1-6}$ alkyl optionally substituted with one or more OH, $NH_2$, CN, or halogens;
$X^1$, $X^2$, $X^3$, $X^4$, and $X^5$ are each independently selected from the group consisting of CH, C, N, O, and S;
each $R^3$ is independently selected from the group consisting of OH, $NH_2$, CN, halogen, $C_{1-6}$ alkyl, and $C_{1-6}$ alkoxy, wherein the OH, $NH_2$, $C_{1-6}$ alkyl, or $C_{1-6}$ alkoxy is optionally substituted with one or more OH, $NH_2$, CN, or halogens;
m and q are each independently selected from the group consisting of 0, 1, 2, and 3;
the proviso is that ULM is not

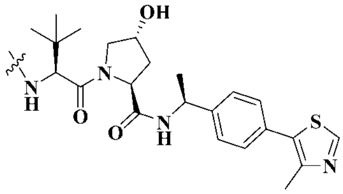
;

each R, X, L, $R^1$, $R^2$, $X^1$, $X^2$, $X^3$, $X^4$, or $X^5$ is independently optionally substituted with one or more substituents. In some embodiments, R is selected from the group consisting of halogen and $C_{1-6}$ alkyl optionally substituted with one or more OH, $NH_2$, CN, or halogens.

In some embodiments, R is selected from the group consisting of F, Cl, Br, and $C_{1-3}$ alkyl optionally substituted with one or more OH, $NH_2$, CN, or halogens.

In some embodiments, R is selected from the group consisting of F, Cl, Br, and $C_{1-3}$ alkyl optionally substituted with one or more F or Cl.

In some embodiments, R is selected from the group consisting of F, Cl, Br, and trifluoromethyl.

In some embodiments, R is selected from Cl.

In some embodiments, q is selected from the group consisting of 0, 1, and 2; or q is selected from the group consisting of 0 and 1.

In some embodiments, R is selected from Cl and q is selected from 1.

In some embodiments, the structural unit

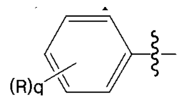

is selected from

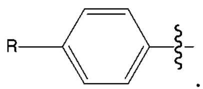
.

In some embodiments, X is selected from the group consisting of NH and O.

In some embodiments, X is selected from NH.

In some embodiments, X is selected from O.

In some embodiments, L is selected from the group consisting of a bond, $-C_{1-20}$ alkyl-, $-C_{2-20}$ alkenyl-, and $-C_{2-20}$ alkynyl-, wherein one or more $-CH_2-$ in the $-C_{1-20}$ alkyl-, $-C_{2-20}$ alkenyl-, or $-C_{2-20}$ alkynyl- are (each independently) optionally replaced by $R^x$; $R^x$ is selected from the group consisting of -O-, $-NR^a-$, $-S(O)_2-$, $-S(O)_2NR^a-$, -S(O)-, $-S(O)NR^a-$, -C(O)-, -C(O)O-, $-C(O)NR^a-$, $-C(O)N(R^a)O-$, -OC(O)-, $-OC(O)NR^a-$, $-N(R^a)C(O)O-$, $-N(R^a)C(O)-$, $-N(R^a)S(O)_2-$, 5- to 12-membered heteroaryl, phenyl, $C_{3-10}$ cycloalkyl, 3- to 10-membered heterocycloalkyl, and -S-; $R^a$ is selected from the

group consisting of hydrogen and $C_{1-6}$ alkyl; each $R^x$ or $R^a$ is independently optionally substituted with one or more substituents.

One or more $-CH_2-$ in the $-C_{1-20}$ alkyl-, $-C_{2-20}$ alkenyl-, or $-C_{2-20}$ alkynyl- described in the present disclosure are optionally replaced by $R^x$, wherein each replacement occurs independently; for example, L is selected from the group consisting of a bond, $-C_{1-20}$ alkyl-, $-C_{2-20}$ alkenyl-, and $-C_{2-20}$ alkynyl-, wherein one or more $-CH_2-$ in the $-C_{1-20}$ alkyl-, $-C_{2-20}$ alkenyl-, or $-C_{2-20}$ alkynyl- are each independently optionally replaced by $R^x$; $R^x$ is selected from the group consisting of -O-, $-NR^a-$, $-S(O)_2-$, $-S(O)_2NR^a-$, -S(O)-, $-S(O)NR^a-$, -C(O)-, -C(O)O-, $-C(O)NR^a-$, - $C(O)N(R^a)O-$, -OC(O)-, $-OC(O)NR^a-$, $-N(R^a)C(O)O-$, $-N(R^a)C(O)-$, $-N(R^a)S(O)_2-$, 5- to 12-membered heteroaryl, phenyl, $C_{3-10}$ cycloalkyl, 3- to 10-membered heterocycloalkyl, and -S-; $R^a$ is selected from the group consisting of hydrogen and $C_{1-6}$ alkyl; each $R^x$ or $R^a$ is independently optionally substituted with one or more substituents.

In some embodiments, $R^a$ is selected from the group consisting of hydrogen and $C_{1-4}$ alkyl, or selected from the group consisting of hydrogen and $C_{1-3}$ alkyl, or selected from the group consisting of hydrogen and $C_{1-2}$ alkyl, or selected from the group consisting of hydrogen and methyl.

In some embodiments, $R^x$ is selected from the group consisting of -O-, -C(O)-, 5- to 12-membered heteroaryl, phenyl, $C_{3-10}$ cycloalkyl, 3- to 10-membered heterocycloalkyl, -NH-, $-N(C_{1-6}$ alkyl)-, and -S-.

In some embodiments, $R^x$ is selected from the group consisting of -O-, -C(O)-, 5- to 6-membered heteroaryl, phenyl, $C_{3-6}$ cycloalkyl, 3- to 6-membered heterocycloalkyl, -NH-, $-N(C_{1-6}$ alkyl)-, and -S-.

In some embodiments, $R^x$ is selected from the group consisting of -O-, -C(O)-, phenyl, $C_{5-6}$ cycloalkyl, 5- to 6-membered heterocycloalkyl, -NH-, $-N(C_{1-3}$ alkyl)-, and -S-.

In some embodiments, $R^x$ is selected from the group consisting of -O-, -C(O)-, phenyl, piperidinyl, piperazinyl, -NH-, $-N(C_{1-3}$ alkyl)-, and -S-.

In some embodiments $R^x$ is selected from the group consisting of -C(O)- and piperazinyl.

In some embodiments, $R^x$ is selected from the group consisting of -C(O)- and

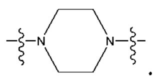.

In some embodiments, $R^x$ is selected from -C(O)-.

Each replacement in L described in the present disclosure occurs independently; for example, L is selected from the group consisting of a bond, $-C_{1-12}$ alkyl-, $-C_{2-12}$ alkenyl-, and $-C_{2-12}$ alkynyl-, wherein one or more $-CH_2-$ in the $-C_{1-12}$ alkyl-, $-C_{2-12}$ alkenyl-, or $-C_{2-12}$ alkynyl- are each independently optionally replaced by $R^x$; $R^x$ is as defined in the present disclosure.

In some embodiments, L is selected from the group consisting of a bond, $-C_{1-12}$ alkyl-, $-C_{2-12}$ alkenyl-, and $-C_{2-12}$ alkynyl-, wherein one or more $-CH_2-$ in the $-C_{1-12}$ alkyl-, $-C_{2-12}$ alkenyl-, or $-C_{2-12}$ alkynyl- are (each independently) optionally replaced by $R^x$; $R^x$ is as defined in the present disclosure.

In some embodiments, L is selected from the group consisting of a bond, $-C_{3-12}$ alkyl-, $-C_{3-12}$ alkenyl-, and $-C_{3-12}$ alkynyl-, wherein one or more $-CH_2-$ in the $-C_{3-12}$ alkyl-, $-C_{3-12}$ alkenyl-, or $-C_{3-12}$ alkynyl- are each independently optionally replaced by $R^x$; $R^x$ is as defined in the present disclosure.

In some embodiments, L is selected from the group consisting of a bond, $-C_{6-12}$ alkyl-, $-C_{6-12}$ alkenyl-, and $-C_{6-12}$ alkynyl-, wherein one or more $-CH_2-$ in the $-C_{6-12}$ alkyl-, $-C_{6-12}$ alkenyl-, or $-C_{6-12}$ alkynyl- are each independently optionally replaced by $R^x$; $R^x$ is as defined in the present disclosure.

In some embodiments, L is selected from the group consisting of a bond, $-C_{8-9}$ alkyl-, $-C_{8-9}$ alkenyl-, and $-C_{8-9}$ alkynyl-, wherein one or more $-CH_2-$ in the $-C_{8-9}$ alkyl-, $-C_{8-9}$ alkenyl-, or $-C_{8-9}$ alkynyl- are each independently optionally replaced by $R^x$; $R^x$ is as defined in the present disclosure.

In some embodiments, L is selected from the group consisting of a bond, $-C_{6-12}$ alkyl-, $-C_{6-12}$ alkenyl-, and $-C_{6-12}$ alkynyl-, wherein one or more $-CH_2-$ in the $-C_{6-12}$ alkyl-, $-C_{6-12}$ alkenyl-, or $-C_{6-12}$ alkynyl- are each independently optionally replaced by $R^x$; $R^x$ is selected from the group consisting of -O-, -C(O)-, phenyl, piperidinyl, piperazinyl, -NH-, $-N(C_{1-3}$ alkyl)-, and -S-.

In some embodiments, L is selected from the group consisting of a bond, $-C_{6-12}$ alkyl-, $-C_{6-12}$ alkenyl-, and $-C_{6-12}$ alkynyl-, wherein one or more $-CH_2-$ in the $-C_{6-12}$ alkyl-, $-C_{6-12}$ alkenyl-, or $-C_{6-12}$ alkynyl- are (each independently) optionally replaced by $R^x$; $R^x$ is selected from the group consisting of -O-, -C(O)-, phenyl, piperidinyl, piperazinyl, -NH-, $-N(C_{1-3}$ alkyl)-, and -S-.

In some embodiments, L is selected from $-C_{3-12}$ alkyl-, wherein one or more $-CH_2-$ in the $-C_{3-12}$ alkyl- are each independently optionally replaced by $R^x$; $R^x$ is selected from the group consisting of -C(O)- and piperazinyl.

In some embodiments, L is selected from $-C_{6-12}$ alkyl-, wherein one or more $-CH_2-$ in the $-C_{6-12}$ alkyl- are each independently optionally replaced by $R^x$; $R^x$ is selected from the group consisting of -C(O)- and piperazinyl.

In some embodiments, L is selected from $-C_{6-10}$ alkyl-, wherein one or more $-CH_2-$ in the $-C_{6-10}$ alkyl- are each

independently optionally replaced by $R^x$; $R^x$ is selected from the group consisting of -C(O)- and piperazinyl.

In some embodiments, L is selected from -$C_{6-10}$ alkyl-, wherein one or more -$CH_2$- in the -$C_{6-10}$ alkyl- are each independently optionally replaced by $R^x$; $R^x$ is selected from the group consisting of -C(O)- and

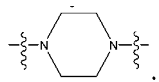.

In some embodiments, L is selected from -$C_{8-10}$ alkyl-, wherein one or more -$CH_2$- in the -$C_{8-10}$ alkyl- are each independently optionally replaced by -C(O)-.

In some embodiments, L is selected from the group consisting of

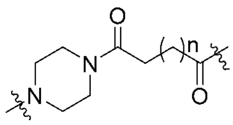, 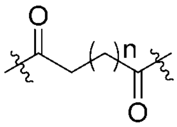,

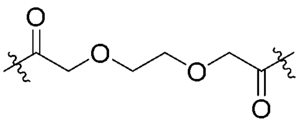, 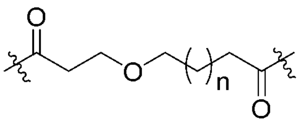, 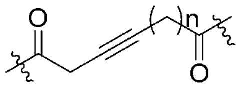, 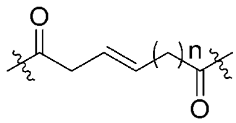,

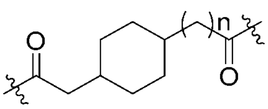, 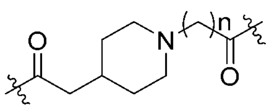, 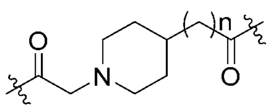, 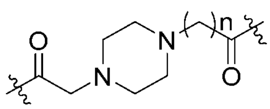,

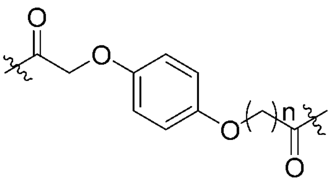, 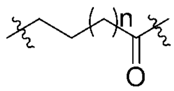, 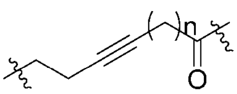, 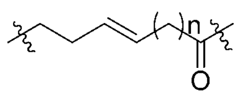,

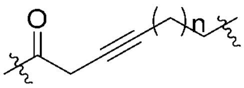, 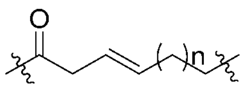, 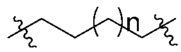, 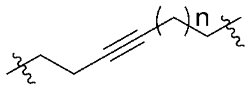,

and

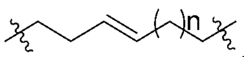,

wherein n is selected from the group consisting of 0-10, or n is selected from the group consisting of 1-9, or n is selected from the group consisting of 1-7.

In some embodiments, L is selected from the group consisting of

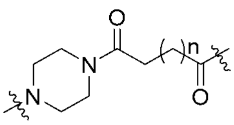, 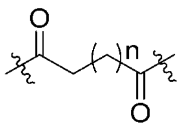,

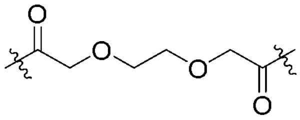, 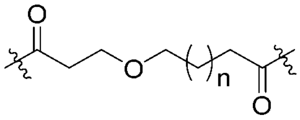, 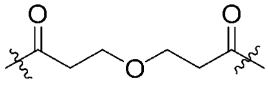, 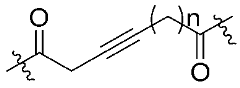,

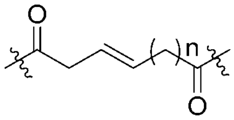, 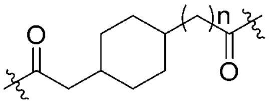, 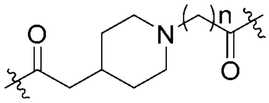, 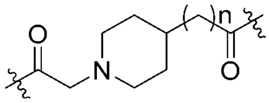,

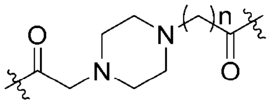, 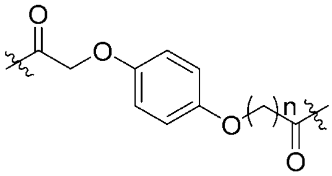, 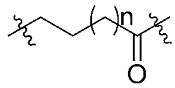, 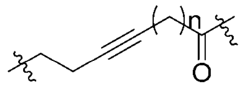,

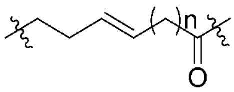, 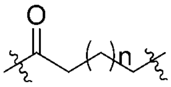, 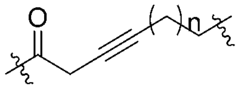, 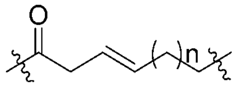, 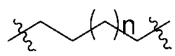,

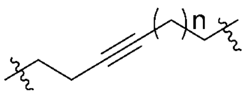, and 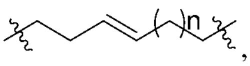,

wherein n is selected from the group consisting of 0-10, or n is selected from the group consisting of 1-9, or n is selected from the group consisting of 1-7.

In some embodiments, L is selected from the group consisting of

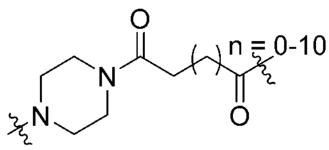,

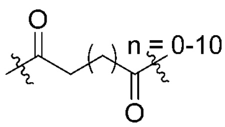, 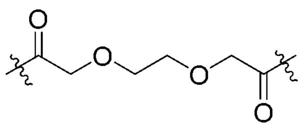, 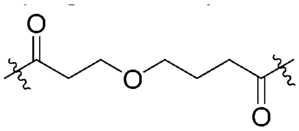, 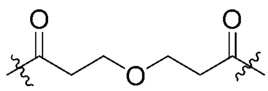,

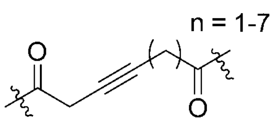, 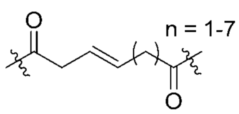, 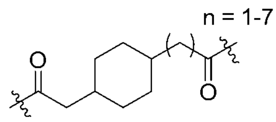, 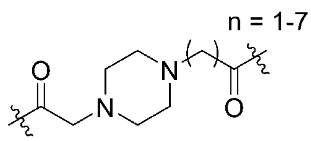,

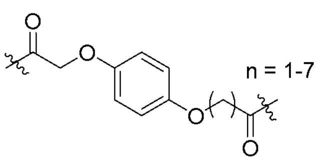, 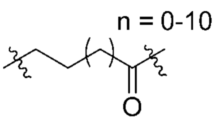, 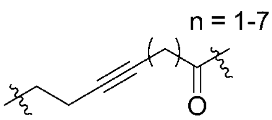, 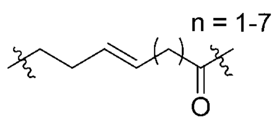,

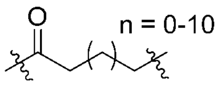, 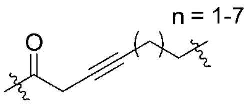, 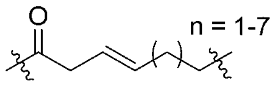, 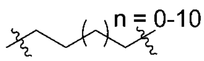,

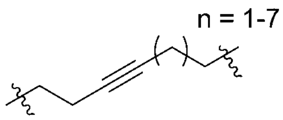, and 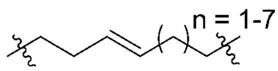.

In some embodiments, L is selected from the group consisting of

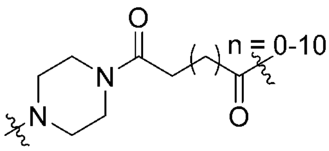

and

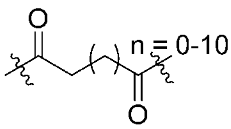

. In some embodiments, L is selected from the group consisting of

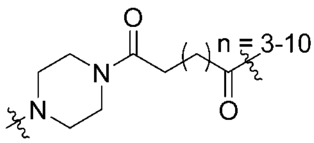

and

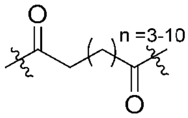

. In some embodiments, L is selected from the group consisting of

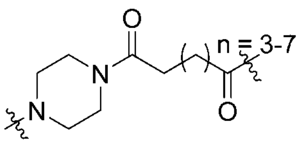

and

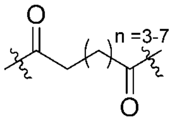.

In some embodiments, L is selected from the group consisting of

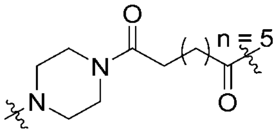

and

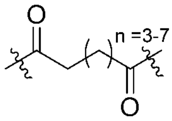

.

In some embodiments, L is selected from the group consisting of

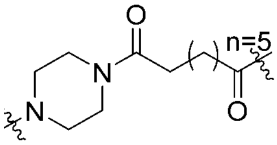

,

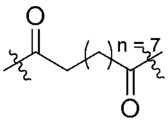

,

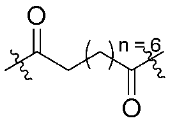

,

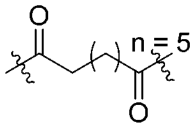

,

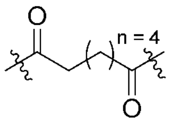

, and

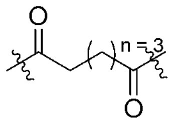

.

In some embodiments, L is selected from the group consisting of

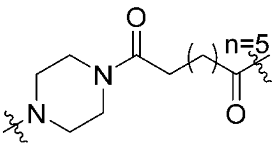

,

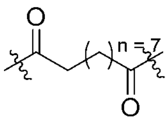

,

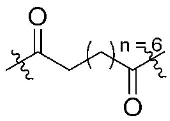

,

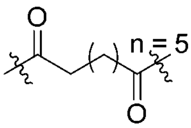

,

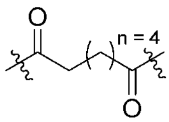

, and

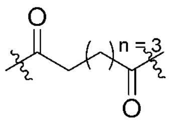

.

In some specific embodiments, L is selected from

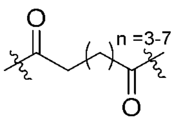

.

In some specific embodiments, L is selected from the group consisting of

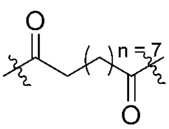

,

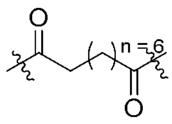

, and

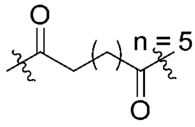

.

. In some specific embodiments, L is selected from

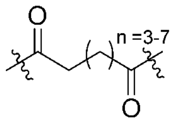

.

In some specific embodiments, L is selected from

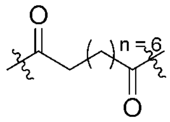

.

In some specific embodiments, L is selected from

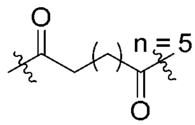

.

In some other embodiments, L is selected from the group consisting of

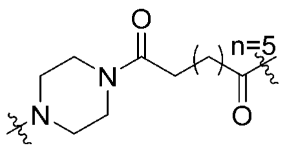

,

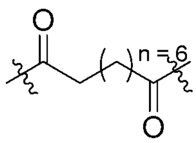

, and

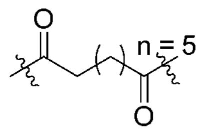

.

In some other embodiments, L is selected from the group consisting of

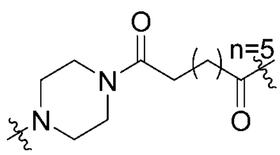

and

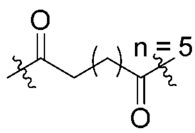

.

In some embodiments, one optional end of L is connected to ULM.

In some embodiments, the right end of L is connected to ULM. For example, the right end of

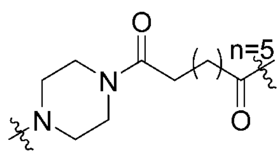

is connected to ULM.

In some embodiments, the left end of L is connected to ULM.

In some embodiments, L is selected from the group consisting of

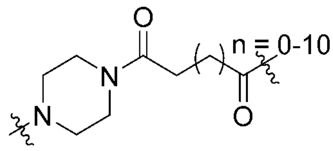

* and

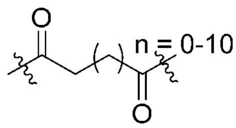

*, wherein * indicates that the end is connected to ULM.

In some embodiments, $X^1$, $X^2$, $X^3$, $X^4$, and $X^5$ are each independently selected from the group consisting of CH, C, N, O, and S.

In some embodiments, $X^1$, $X^2$, $X^3$, $X^4$, and $X^5$ are each independently selected from the group consisting of CH, C, N, NH, O, and S. In some embodiments, $X^1$, $X^2$, $X^3$, $X^4$, and $X^5$ are each independently selected from the group consisting of CH, C, N, NH, and S. In some embodiments, $X^1$, $X^2$, $X^3$, $X^4$, and $X^5$ are each independently selected from the group consisting of CH, C, NH, and N.

In some embodiments, $X^1$ and $X^2$ are each independently selected from the group consisting of N and NH, $X^3$ and $X^4$ are each independently selected from CH, and $X^5$ is selected from C. In some embodiments, $X^1$, $X^2$, and $X^4$ are selected from CH, and $X^3$ and $X^5$ are selected from the group consisting of N and NH. In some embodiments, $X^1$, $X^2$, and $X^4$ are selected from CH, $X^3$ is selected from the group consisting of N and NH, and $X^5$ is selected from N. In some embodiments, $X^1$ is selected from S, $X^2$ and $X^4$ are selected from CH, $X^3$ is selected from the group consisting of N and NH, and $X^5$ is selected from C.

In some embodiments, $X^1$ and $X^2$ are selected from N, $X^3$ and $X^4$ are each independently selected from CH, and $X^5$ is selected from C; or, $X^1$, $X^2$, and $X^4$ are selected from CH, and $X^3$ and $X^5$ are selected from N; or, $X^1$ is selected from S, $X^2$ and $X^4$ are selected from CH, $X^3$ is selected from N, and $X^5$ is selected from C.

In some embodiments, the structural unit

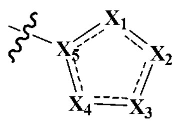

is selected from the group consisting of

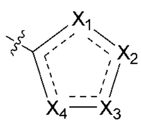

and

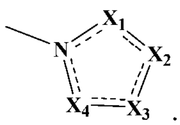

In some embodiments, the structural unit

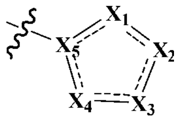

is selected from the group consisting of

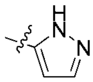, 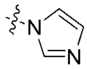,

and

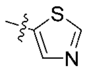.

In some embodiments, the structural unit

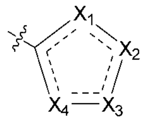

is selected from

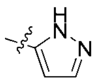

. In some embodiments, the structural unit

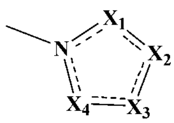

is selected from

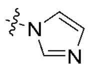

. In some embodiments, the structural unit

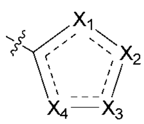

is selected from

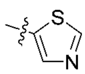.

In some embodiments, $R^1$ is selected from $C_{1-4}$ alkyl optionally substituted with one or more halogens.

In some embodiments, $R^1$ is selected from $C_{3-4}$ alkyl optionally substituted with one or more halogens.

In some embodiments, $R^1$ is selected from the group consisting of isopropyl and tert-butyl optionally substituted with one or more halogens.

In some embodiments, $R^1$ is selected from the group consisting of isopropyl and tert-butyl.

In some embodiments, $R^2$ is selected from $C_{1-4}$ alkyl optionally substituted with one or more OH, $NH_2$, CN, or halogens.

In some embodiments, $R^2$ is selected from methyl optionally substituted with one or more OH, $NH_2$, CN, or halogens.

In some embodiments, $R^2$ is selected from methyl optionally substituted with one or more OH.

In some embodiments, $R^2$ is selected from the group consisting of hydroxymethyl and methyl.

In some embodiments, $X^1$, $X^2$, $X^3$, $X^4$, and $X^5$ are each independently selected from the group consisting of CH, C, N, NH, O, and S, and at least one of them is N or NH.

In some embodiments, $X^1$, $X^2$, $X^3$, $X^4$, and $X^5$ are each independently selected from the group consisting of CH, C, N, NH, O, and S, and at least two of them are heteroatoms.

In some embodiments, $X^1$, $X^2$, $X^3$, $X^4$, and $X^5$ are each independently selected from the group consisting of CH, C, N, NH, O, and S, and at least two of them are selected from the group consisting of N, NH, and S.

In some embodiments, $X^1$, $X^2$, $X^3$, $X^4$, and $X^5$ are each independently selected from the group consisting of CH, C, N, O, and S, and at least one of them is N.

In some embodiments, $X^1$, $X^2$, $X^3$, $X^4$, and $X^5$ are each independently selected from the group consisting of CH, C, N, O, and S, and at least two of them are heteroatoms.

In some embodiments, $X^1$, $X^2$, $X^3$, $X^4$, and $X^5$ are each independently selected from the group consisting of CH, C, N, O, and S, and at least two of them are selected from the group consisting of N and S.

In some embodiments, $X^5$ is C.

In some embodiments, $X^5$ is N.

In some embodiments, the ring formed by $X^1$, $X^2$, $X^3$, $X^4$, and $X^5$ is aromatic. In some embodiments, the ring formed by $X^1$, $X^2$, $X^3$, $X^4$, and $X^5$ is a five-membered heteroaromatic ring.

In some embodiments, the structural unit

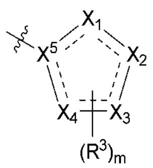

is selected from the group consisting of

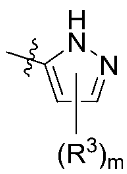

,

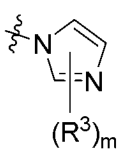

, and

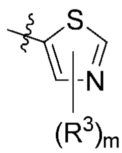

.

In some embodiments, the structural unit

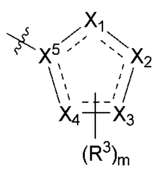

is selected from

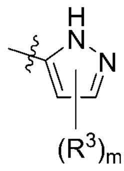.

In some embodiments, the structural unit

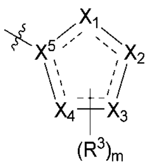

is selected from

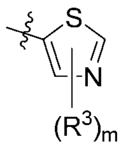.

In some embodiments, the structural unit

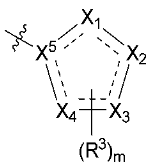

is selected from the group consisting of

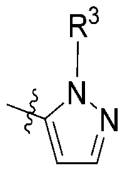,

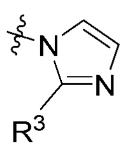, and 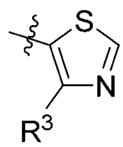.

In some embodiments, the structural unit

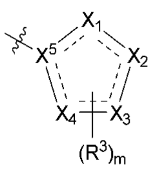

is selected from

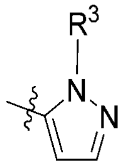.

In some embodiments, the structural unit

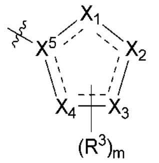

is selected from

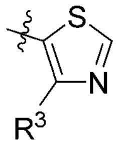.

In some embodiments, each $R^3$ is independently selected from the group consisting of OH, $NH_2$, CN, halogen, $C_{1-3}$ alkyl, and $C_{1-3}$ alkoxy, wherein the OH, $NH_2$, $C_{1-3}$ alkyl, or $C_{1-3}$ alkoxy is optionally substituted with one or more OH, $NH_2$, CN, or halogens.

In some embodiments, each $R^3$ is independently selected from the group consisting of $C_{1-3}$ alkyl and $C_{1-3}$ alkoxy. In some embodiments, each $R^3$ is independently selected from the group consisting of OH, $NH_2$, CN, halogen, methyl, and ethyl.

In some embodiments, each $R^3$ is independently selected from $C_{1-3}$ alkyl. In some embodiments, each $R^3$ is independently selected from the group consisting of methyl and ethyl.

In some embodiments, the structural unit

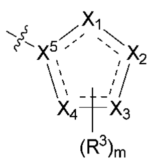

is selected from the group consisting of

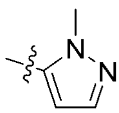,

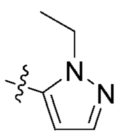, 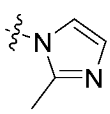, and 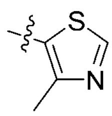

. In some embodiments, the structural unit

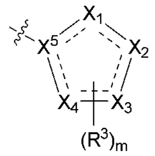

is selected from the group consisting of

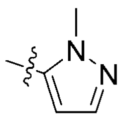 and 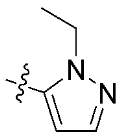.

In some embodiments, ULM is

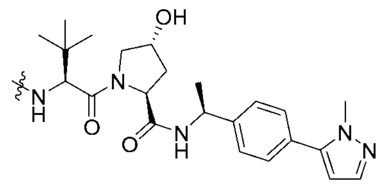, 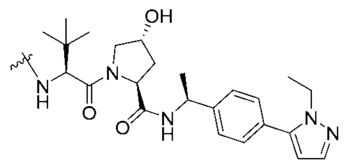,

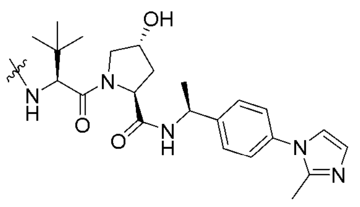, 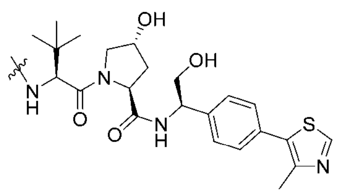, 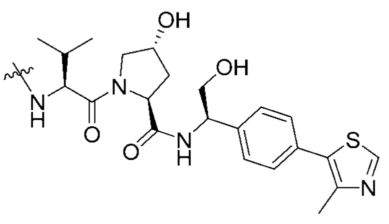,

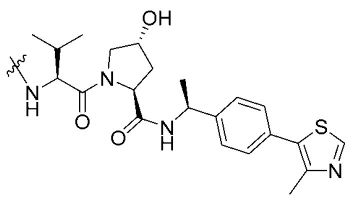, 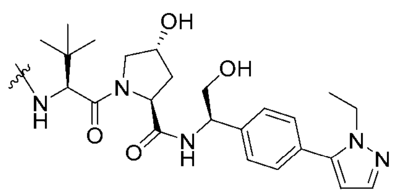, 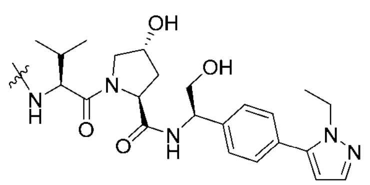,

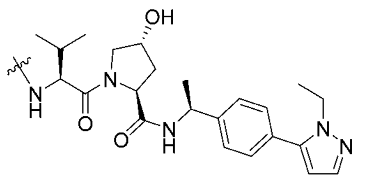, 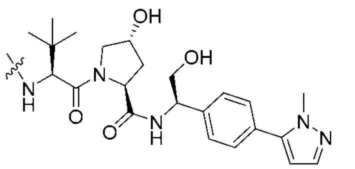, 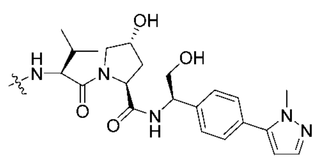

or

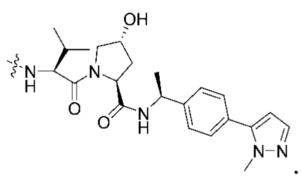.

In some embodiments, ULM is

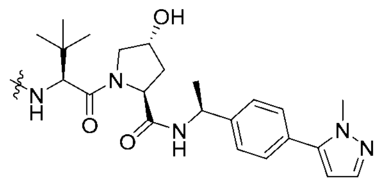,

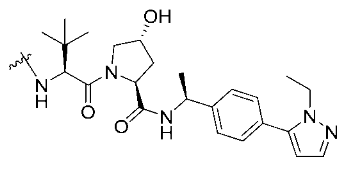,

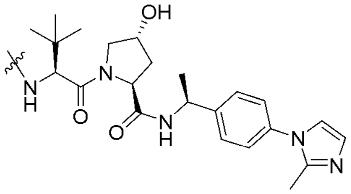,

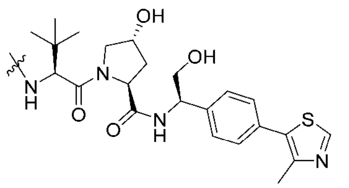,

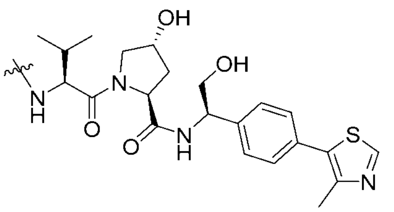,

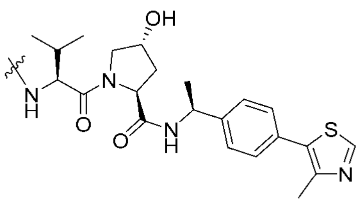,

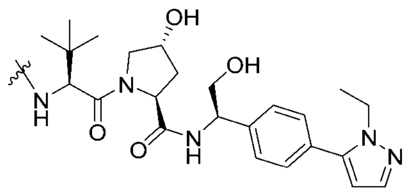,

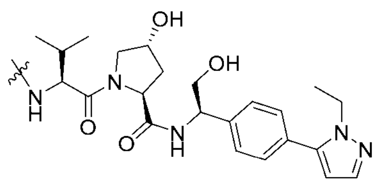,

or

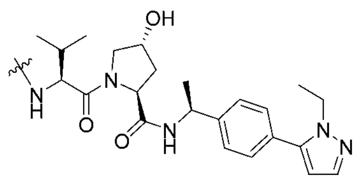.

In some embodiments, m and q are each 1.

The compound of formula I or the pharmaceutically acceptable salt thereof of the present disclosure is selected from the group consisting of compounds of formula II, formula III, formula II-A, formula III-A, formula III-B, formula IV, formula V, formula VI and formula VII, stereoisomers thereof, or pharmaceutically acceptable salts thereof:

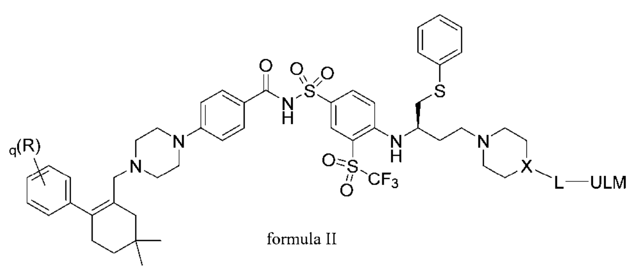

formula II

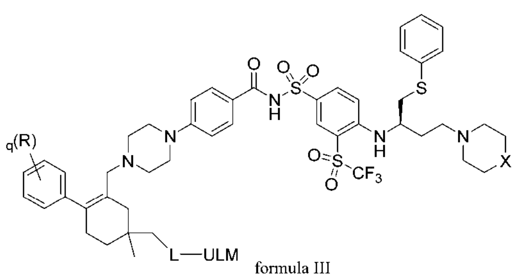

formula III

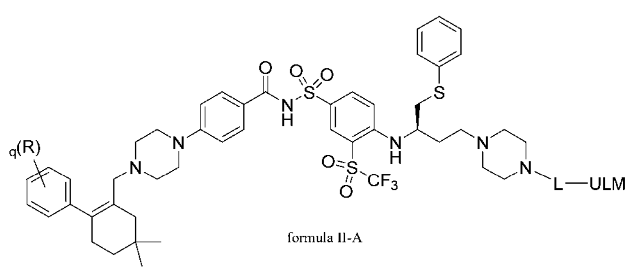

formula II-A

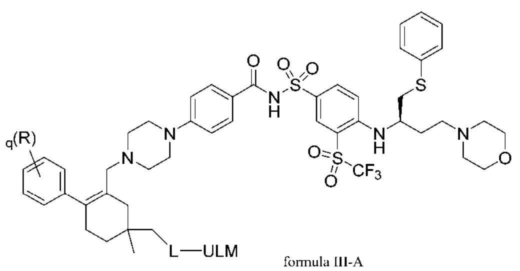

formula III-A

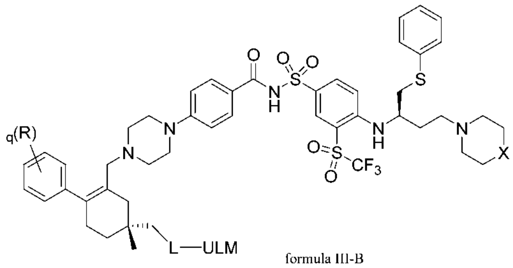

formula III-B

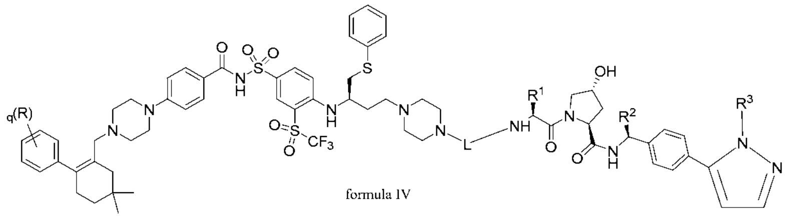

formula IV

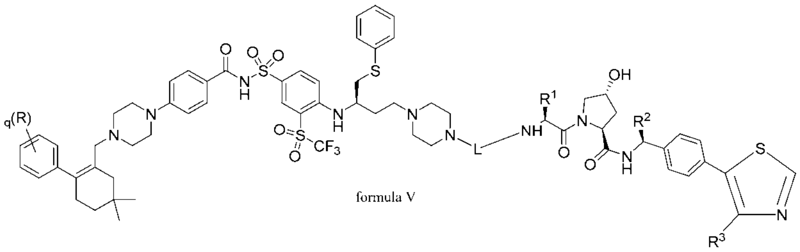

formula V

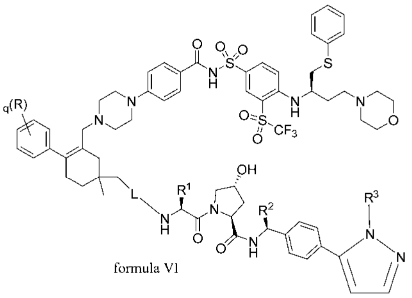

formula VI

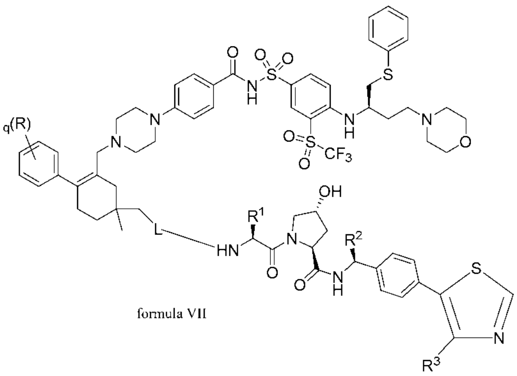

formula VII

wherein R, q, L, ULM, X, $R^1$, $R^2$, or $R^3$ is as defined in the present disclosure.

Each R, X, L, $R^1$, $R^2$, $X^1$, $X^2$, $X^3$, $X^4$, $X^5$, $R^x$, or $R^a$ of the present disclosure is independently optionally substituted with one or more substituents.

The present disclosure relates to a compound of a formula selected from the group consisting of the following formulas, a stereoisomer thereof, or a pharmaceutically acceptable salt thereof:

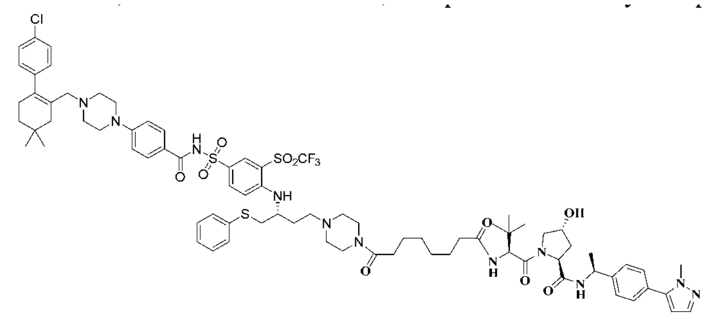

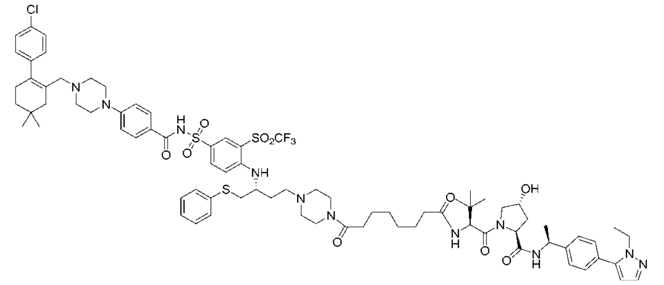

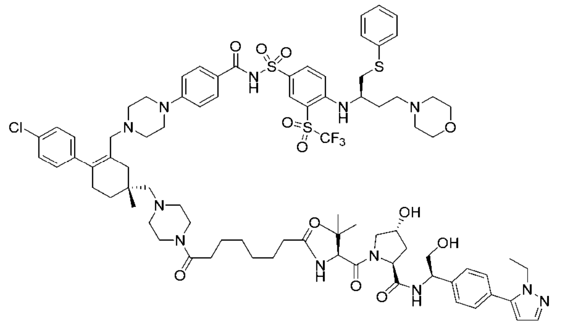

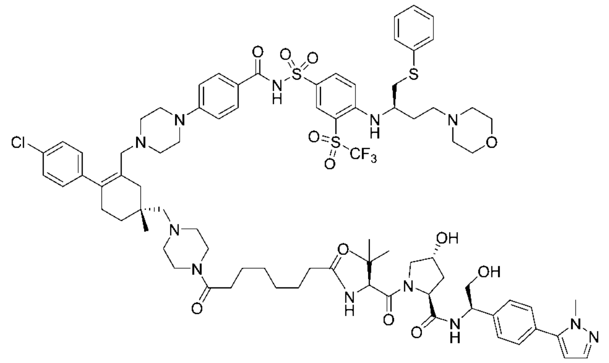

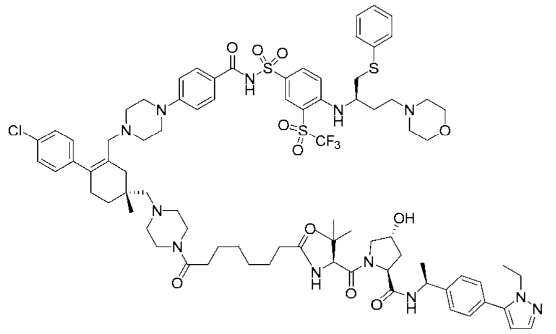

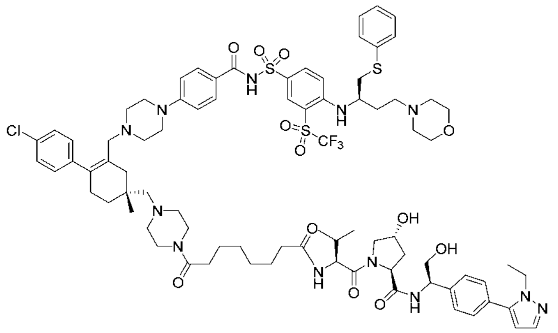

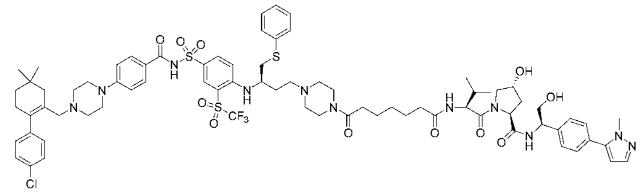

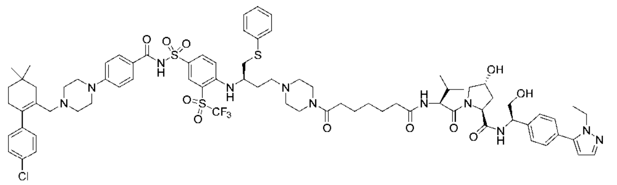

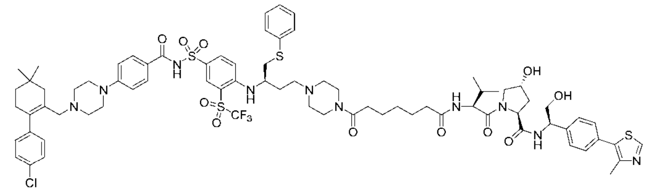

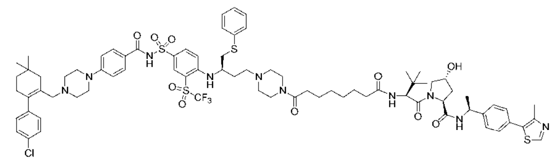

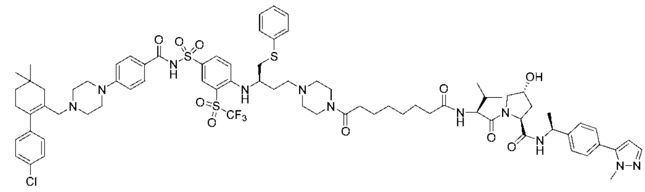

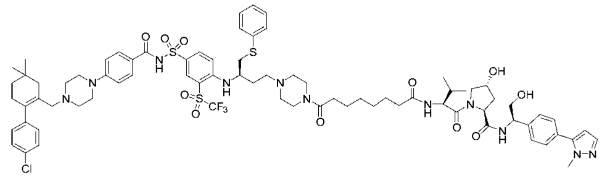

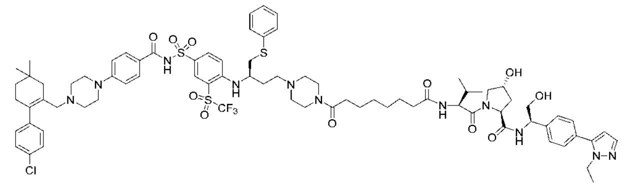

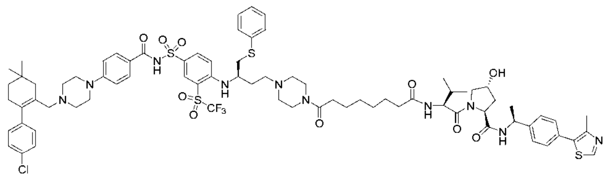

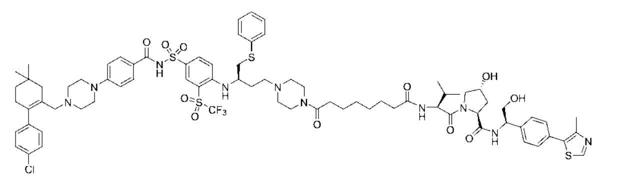

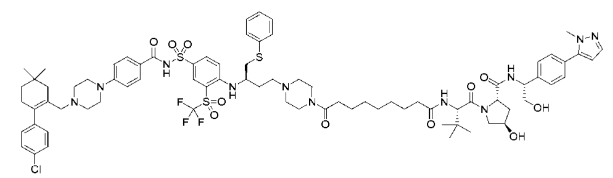

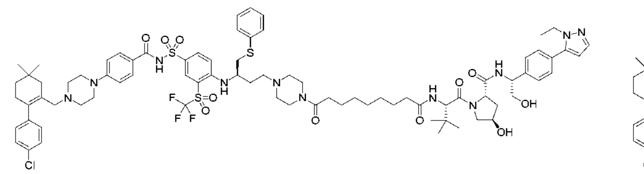

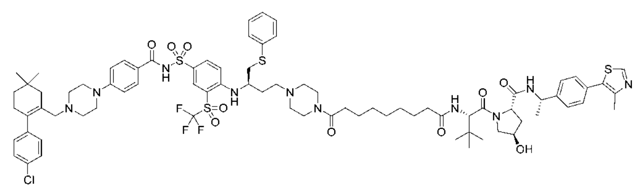

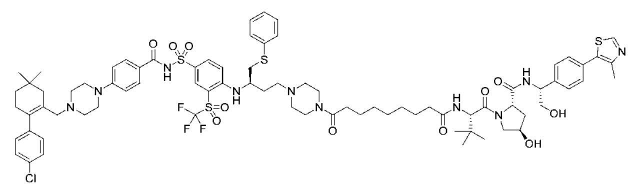

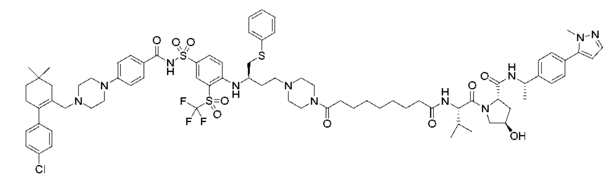

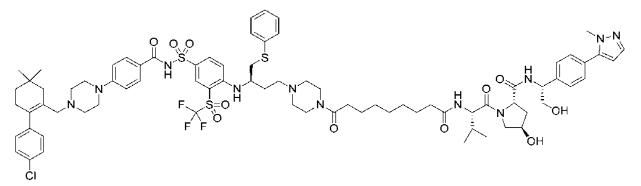

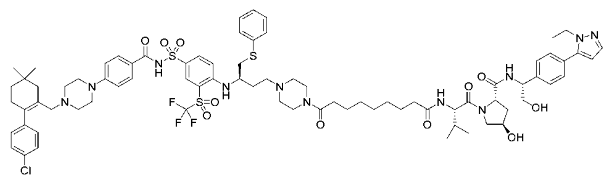

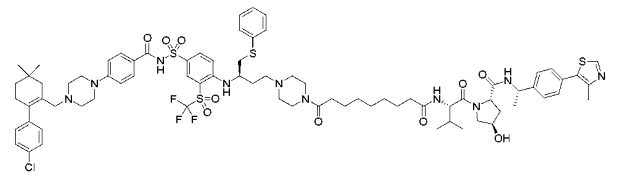

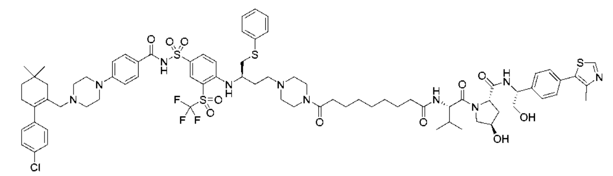

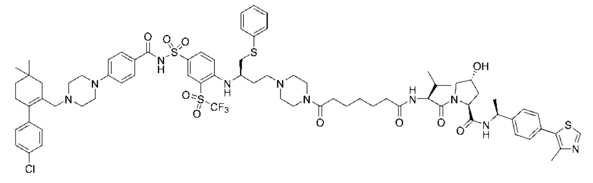

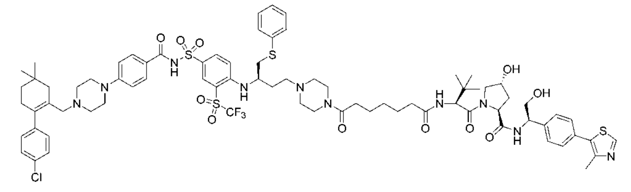

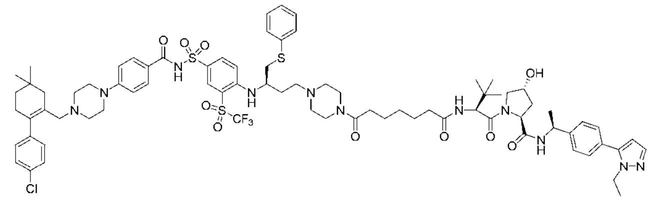

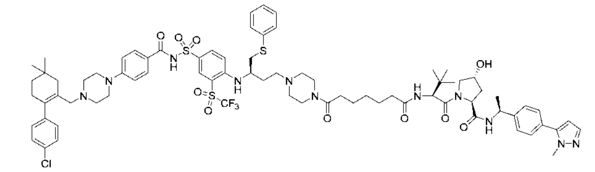

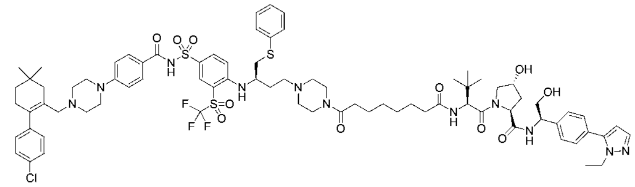

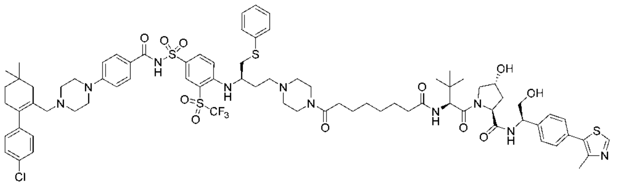

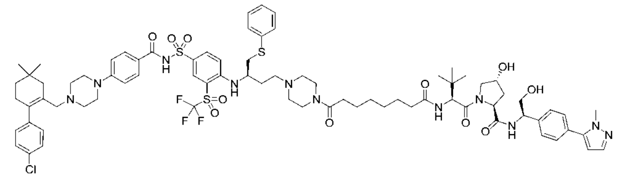

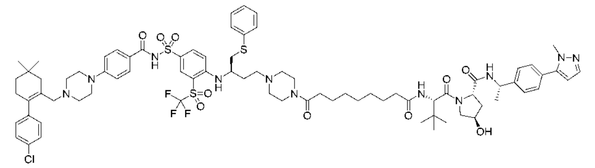

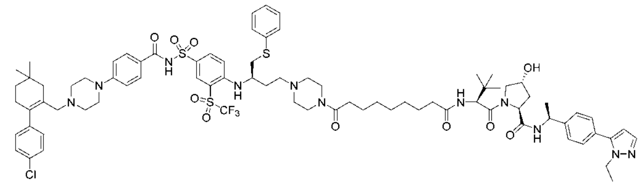

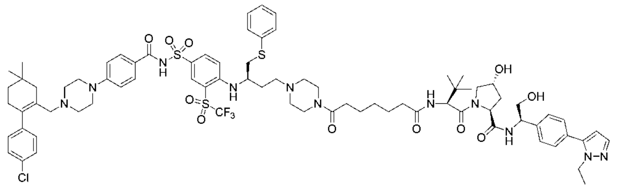

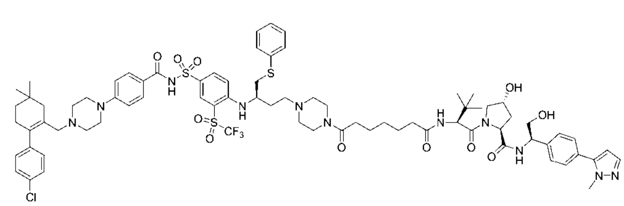

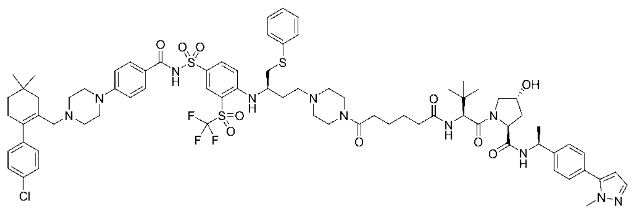

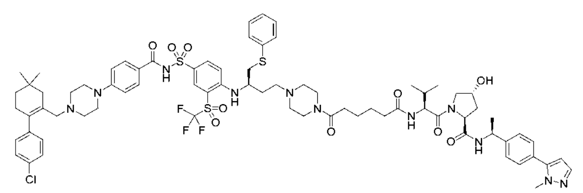

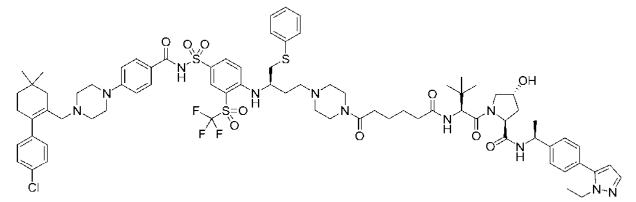

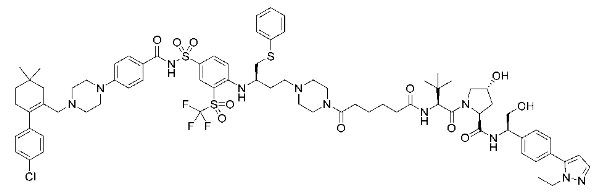

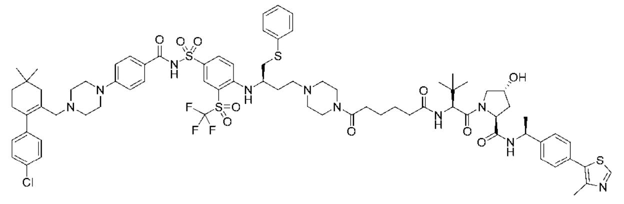

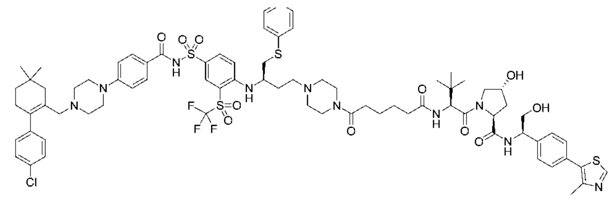

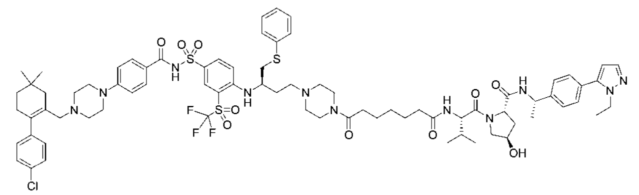

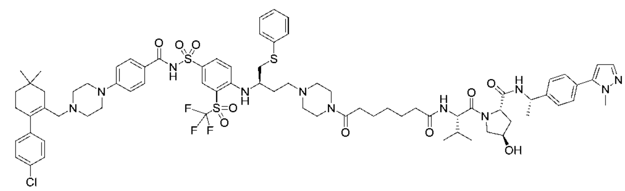

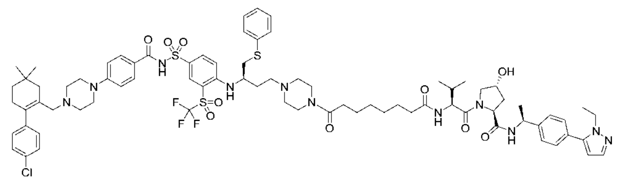

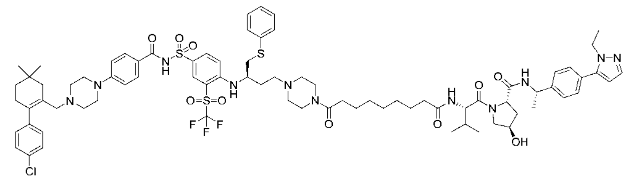

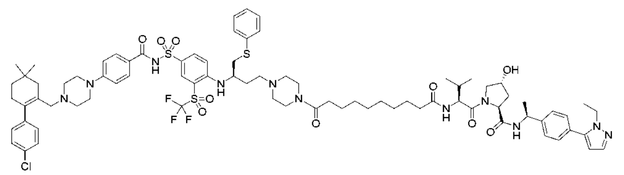

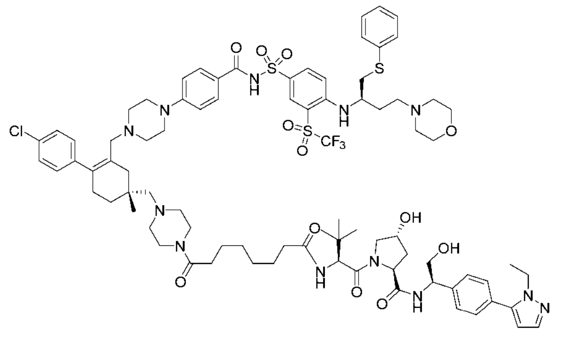

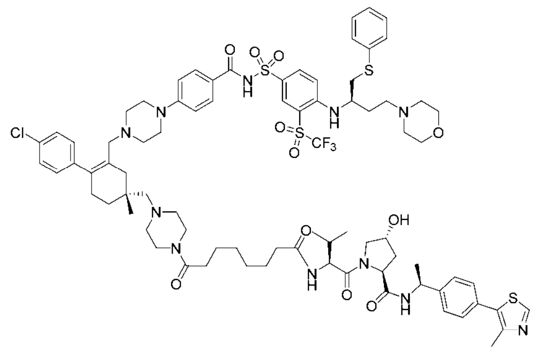

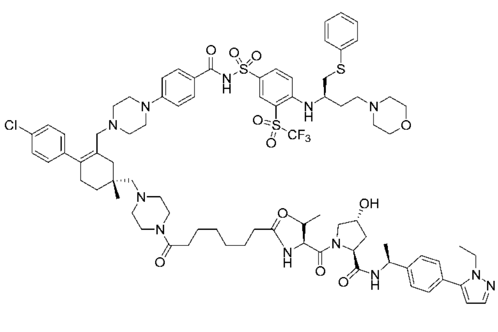

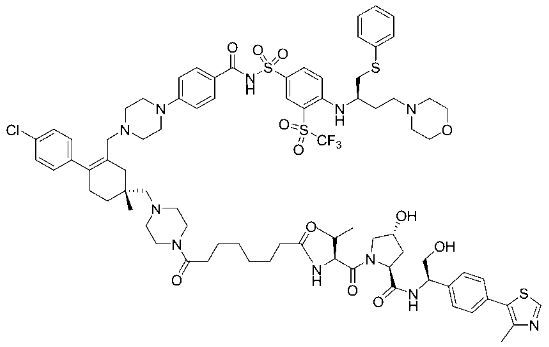

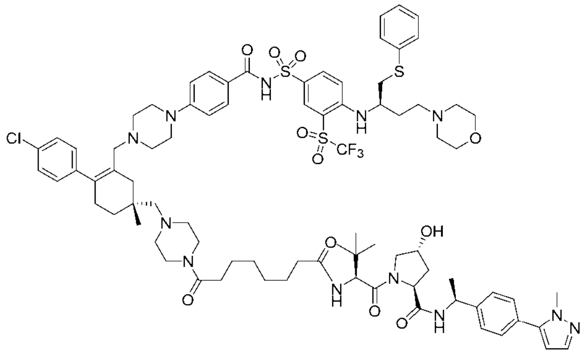

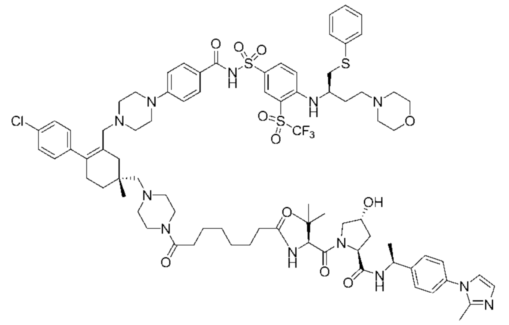

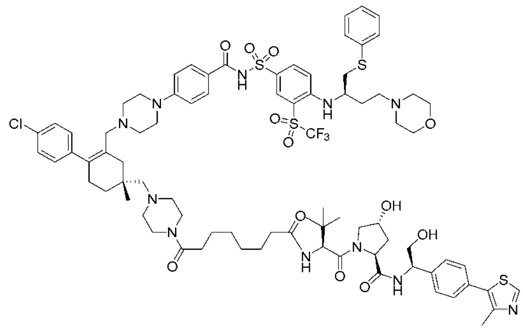

and

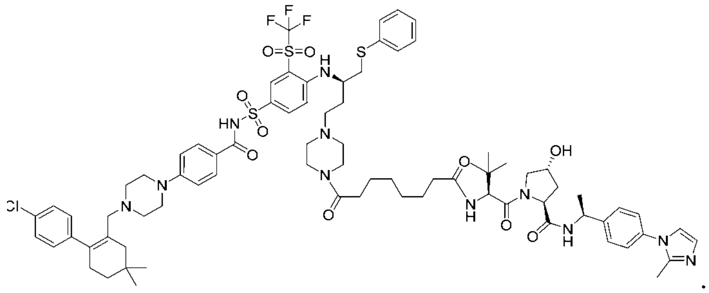

.

In another aspect, the present disclosure relates to a pharmaceutical composition, which comprises the compound, the stereoisomer thereof, or the pharmaceutically acceptable salt thereof described in the present disclosure. In some embodiments, the pharmaceutical composition of the present disclosure further comprises a pharmaceutically acceptable excipient.

In another aspect, the present disclosure relates to a pharmaceutical composition, which comprises the compound, the stereoisomer thereof, or the pharmaceutically acceptable salt thereof described in the present disclosure; the pharmaceutical composition of the present disclosure further comprises a pharmaceutically acceptable excipient. In another aspect, the present disclosure relates to use of the compound, the stereoisomer thereof, or the pharmaceutically acceptable salt thereof, or the pharmaceutical composition thereof described in the present disclosure for preparing a medicament for preventing or treating a disorder treated by degrading a target protein that binds to a targeting ligand (e.g., a small-molecule structural moiety associated with BCL-XL).

In another aspect, the present disclosure relates to use of the compound, the stereoisomer thereof, or the pharmaceutically acceptable salt thereof, or the pharmaceutical composition thereof described in the present disclosure for preparing a medicament for preventing or treating a disorder treated by binding to cereblon protein *in vivo.*

In another aspect, the present disclosure relates to use of the compound, the stereoisomer thereof, or the pharmaceutically acceptable salt thereof, or the pharmaceutical composition thereof described in the present disclosure for preparing a medicament for preventing or treating a disorder treated by binding to cereblon protein. In another aspect, the present disclosure relates to use of the compound, the stereoisomer thereof, or the pharmaceutically acceptable salt thereof, or the pharmaceutical composition thereof described in the present disclosure for preparing a medicament for preventing or treating a BCL-XL-related disease.

The present disclosure relates to a method for treating or preventing a disorder treated by degrading a target

protein that binds to a targeting ligand in a mammal, which comprises administering to a mammal, preferably a human, in need of such treatment a therapeutically effective amount of the compound, the stereoisomer thereof, or the pharmaceutically acceptable salt thereof, or the pharmaceutical composition thereof described in the present disclosure.

The present disclosure relates to a method for treating or preventing a disorder treated by binding to cereblon protein, which comprises administering to a mammal, preferably a human, in need of such treatment a therapeutically effective amount of the compound, the stereoisomer thereof, or the pharmaceutically acceptable salt thereof, or the pharmaceutical composition thereof described in the present disclosure.

The present disclosure relates to a method for treating or preventing a disorder treated by binding to cereblon protein *in vivo,* which comprises administering to a mammal, preferably a human, in need of such treatment a therapeutically effective amount of the compound, the stereoisomer thereof, or the pharmaceutically acceptable salt thereof, or the pharmaceutical composition thereof described in the present disclosure.

In another aspect, the present disclosure relates to a method for treating a BCL-XL-related disease in a mammal, which comprises administering to a mammal, preferably a human, in need of such treatment a therapeutically effective amount of the compound, the stereoisomer thereof, or the pharmaceutically acceptable salt thereof, or the pharmaceutical composition thereof described in the present disclosure.

In another aspect, the present disclosure relates to the compound, the stereoisomer thereof, or the pharmaceutically acceptable salt thereof, or the pharmaceutical composition thereof described in the present disclosure for use in preventing or treating a disorder treated by degrading a target protein that binds to a targeting ligand.

In another aspect, the present disclosure relates to the compound, the stereoisomer thereof, or the pharmaceutically acceptable salt thereof, or the pharmaceutical composition thereof described in the present disclosure for use in preventing or treating a disorder treated by binding to cereblon protein.

In another aspect, the present disclosure relates to the compound, the stereoisomer thereof, or the pharmaceutically acceptable salt thereof, or the pharmaceutical composition thereof described in the present disclosure for use in preventing or treating a disorder treated by binding to cereblon protein *in vivo.*

In another aspect, the present disclosure relates to the compound, the stereoisomer thereof, or the pharmaceutically acceptable salt thereof, or the pharmaceutical composition thereof described in the present disclosure for use in preventing or treating a BCL-XL-related disease.

In another aspect, the present disclosure relates to use of the compound, the stereoisomer thereof, or the pharmaceutically acceptable salt thereof, or the pharmaceutical composition thereof described in the present disclosure for preventing or treating a disorder treated by degrading a target protein that binds to a targeting ligand. In another aspect, the present disclosure relates to use of the compound, the stereoisomer thereof, or the pharmaceutically acceptable salt thereof, or the pharmaceutical composition thereof described in the present disclosure for preventing or treating a disorder treated by binding to cereblon protein.

In another aspect, the present disclosure relates to use of the compound, the stereoisomer thereof, or the pharmaceutically acceptable salt thereof, or the pharmaceutical composition thereof described in the present disclosure for preventing or treating a disorder treated by binding to cereblon protein *in vivo.*

In another aspect, the present disclosure relates to use of the compound, the stereoisomer thereof, or the pharmaceutically acceptable salt thereof, or the pharmaceutical composition thereof described in the present disclosure for preventing or treating a BCL-XL-related disease.

In some specific embodiments, the BCL-XL-related disease is selected from the group consisting of disorders treated by degrading and/or inhibiting a protein that binds to a BCL-XL target protein ligand; in some specific embodiments, the BCL-XL-related disease is selected from the group consisting of disorders treated by binding to cereblon protein; in some specific embodiments, the BCL-XL-related disease is selected from the group consisting of disorders treated by binding to cereblon protein *in vivo*; in some embodiments, the disease or disorder is selected from the group consisting of a tumor and a cancer.

In some specific embodiments, the disorder treated by binding to cereblon protein *in vivo* is selected from the group consisting of BCL-XL-related diseases. In some specific embodiments, the BCL-XL-related disease is selected from the group consisting of a tumor and a cancer.

In some embodiments, the present disclosure encompasses the defined variables and embodiments thereof, as well as any combination thereof.

### Technical Effects

The compound of the present disclosure exerts an inhibitory effect on the proliferation of RS4;11 cells and MOLT-4 cells, can degrade BCL-XL protein in MOLT-4 cells, has good BCL-XL protein degradation kinetic properties, exhibits metabolic stability *in vitro* (e.g., human, rat, or mouse liver microsomes), demonstrates relatively low toxicity in platelets (e.g., dog platelets), and possesses good *in vivo* efficacy (e.g., good tumor inhibition effect) and *in vivo* (e.g., mouse, rat, dog, etc.) pharmacokinetic properties. Furthermore, the compound of the present disclosure has an effective

inhibitory effect on tumor growth *in vivo.* In addition, the compound of the present disclosure has relatively low toxicity in platelets and can provide good safety for use as a medicament.

**Definitions**

Unless otherwise stated, the following terms used in the present disclosure shall have the following meanings. A certain term, unless otherwise specifically defined, should not be considered uncertain or unclear, but construed according to its common meaning in the art. When referring to a trade name, it is intended to refer to its corresponding commercial product or its active ingredient.

Unless otherwise specified,

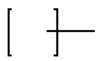

is used to indicate that a hydrogen atom at any position of a group within

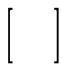

may be replaced by a group connected via "-", for example, by L.

The term "substituted" means that any one or more hydrogen atoms on a specific atom are replaced by substituents, as long as the valence of the specific atom is normal and the resulting compound is stable. When the substituent is oxo (i.e., =O), it means that two hydrogen atoms are replaced; oxo is not possible on an aromatic group.

The term "optional" or "optionally" means that the subsequently described event or circumstance may or may not occur. The description includes instances where the event or circumstance occurs and instances where it does not. For example, ethyl "optionally" substituted with halogen means that the ethyl may be unsubstituted ($CH_2CH_3$), monosubstituted (e.g., $CH_2CH_2F$), polysubstituted (e.g., $CHFCH_2F$, $CH_2CHF_2$, etc.), or fully substituted ($CF_2CF_3$). It can be understood by those skilled in the art that for any group containing one or more substituents, no substitutions or substitution patterns that are impossible to spatially exist and/or synthesize will be introduced.

The "substituent" described herein encompasses all substituents mentioned in the context of the present disclosure, including but not limited to, the terms "alkyl", "alkoxy", "heteroalkyl", "alkenyl", "alkynyl", "cycloalkenyl", "cycloalkyl", "heterocycloalkyl", "heterocycloalkenyl", "heterocyclyl", "heteroaryl", and the like, and the corresponding non-limiting or exemplary groups mentioned below, wherein some non-limiting examples of the "substituent" include deuterium, tritium, -OH, -SH, halogen, $-NH_2$, nitro, nitroso, -CN, an azido group, a sulfoxide group, a sulfone group, a sulfonamide group, carboxyl, a carboxaldehyde group, an imine group, alkyl, halogenated alkyl, cycloalkyl, halogenated cycloalkyl, alkenyl, halogenated alkenyl, cycloalkenyl, halogenated cycloalkenyl, alkynyl, halogenated alkynyl, cycloalkynyl, halogenated cycloalkynyl, heteroalkyl, halogenated heteroalkyl, alkoxy, alkylthio, aryl, aryloxy, arylthio, arylalkyl, arylalkoxy, arylalkylthio, heteroaryl, heteroaryloxy, heteroarylthio, heteroarylalkyl, heteroarylalkoxy, heteroarylalkylthio, heterocyclyl, heterocyclyloxy, heterocyclylthio, heterocyclylalkyl, heterocyclylalkoxy, heterocyclylalkylthio, acyl, acyloxy, a carbamate group, an amido group, ureido, an epoxy group, an ester group, and the like, wherein the groups are optionally substituted with one or more substituents selected from the group consisting of the following substituents: oxo, hydroxy, amino, nitro, halogen, cyano, alkyl, alkenyl, alkynyl, alkoxy, halogenated alkoxy, alkylamino, dialkylamino, halogenated alkylamino, halogenated dialkylamino, carboxy, -C(O)O-alkyl, -OC(O)-alkyl, $-C(O)NH_2$, -C(O)NH-alkyl, - $C(O)N(alkyl)_2$, -NHC(O)-alkyl, -C(O)-alkyl, -S(O)-alkyl, $-S(O)_2$-alkyl, $-S(O)_2NH_2$, $-S(O)_2$NH-alkyl, - $S(O)_2N(alkyl)_2$, cycloalkyl, cycloalkylalkyl, cycloalkyloxy, heterocyclyl, heterocyclylalkyl, heterocyclyloxy, heterocycloalkyl, heterocycloalkylalkyl, heterocycloalkyloxy, heteroaryl, heteroarylalkyl, heteroaryloxy, aryl, arylalkyl, and aryloxy.

$C_{m-n}$ herein means that the moiety has an integer number of carbon atoms in the given range. For example, "$C_{1-6}$" means that the group may have 1 carbon atom, 2 carbon atoms, 3 carbon atoms, 4 carbon atoms, 5 carbon atoms, or 6 carbon atoms. For example, $C_{1-3}$ means that the group may have 1 carbon atom, 2 carbon atoms, or 3 carbon atoms.

In some embodiments herein, the substituent is selected from the group consisting of deuterium, tritium, hydroxyl, sulfydryl, halogen, amino, nitro, nitroso, cyano, azido, a sulfoxide group, a sulfone group, a sulfonamide group, carboxyl, an aldehyde group, an imine group, $C_{1-12}$ alkyl, halogenated $C_{1-12}$ alkyl, 3- to 12-membered cycloalkyl, halogenated 3- to 12-membered cycloalkyl, $C_{2-12}$ alkenyl, halogenated $C_{2-12}$ alkenyl, 3- to 12-membered cycloalkenyl, halogenated 3- to 12-membered cycloalkenyl, $C_{2-12}$ alkynyl, halogenated $C_{2-12}$ alkynyl, 8- to 12-membered cycloalkynyl, halogenated 8- to 12-membered cycloalkynyl, $C_{1-12}$ heteroalkyl, halogenated $C_{1-12}$ heteroalkyl, $C_{1-12}$ alkoxy, $C_{1-12}$ alkylthio, 6- to 10-membered aryl, 6- to 10-membered aryloxy, 6- to 10-membered arylthio, 6- to 10-membered aryl $C_{1-12}$ alkylene, 6- to 10-membered aryl $C_{1-12}$ alkoxy, 6- to 10-membered aryl $C_{1-12}$ alkylthio, 5- to 10-membered heteroaryl, 5- to 10-membered heteroaryloxy, 5- to 10-membered heteroarylthio, 5-to 10-membered heteroarylalkylene, 5- to 10-membered heteroarylalkoxy, 5- to 10-membered heteroarylalkylthio, 3- to 12-membered heterocyclyl, 3- to 12-membered heterocyclyloxy, 3- to 12-membered heterocyclylthio, 3- to 12-membered heterocyclyl $C_{1-12}$ alkylene, 3-to 12-membered heterocyclyl $C_{1-12}$ alkoxy, 3- to 12-membered heterocyclyl $C_{1-12}$ alkylthio, $C_{1-12}$ acyl, $C_{1-12}$ acyloxy, a carbamate group, $C_{1-12}$ amido, ureido, an epoxy group, a $C_{2-12}$ ester group, and oxo, wherein these substituents are optionally substituted with one or more substituents selected from the group consisting of the following substituents: oxo, hydroxy, amino, nitro, halogen, cyano, $C_{1-12}$ alkyl, $C_{2-12}$ alkenyl, $C_{2-12}$ alkynyl, $C_{1-12}$ alkoxy, halogenated $C_{1-12}$ alkoxy, $C_{1-12}$ alkylamino, di-$C_{1-12}$ alkylamino, halogenated $C_{1-12}$ alkylamino, halogenated di-$C_{1-12}$ alkylamino, carboxyl, -C(O)O-$C_{1-12}$ alkyl, -OC(O)-$C_{1-12}$ alkyl, -C(O)$NH_2$, - C(O)NH-$C_{1-12}$ alkyl, -C(O)N($C_{1-12}$ alkyl)$_2$, -NHC(O)-$C_{1-12}$ alkyl, -C(O)-$C_{1-12}$ alkyl, -S(O)-$C_{1-12}$ alkyl, -S(O)$_2$-$C_{1-12}$ alkyl, -S(O)$_2$$NH_2$, -S(O)$_2$NH-$C_{1-12}$ alkyl, -S(O)$_2$N($C_{1-12}$ alkyl)$_2$, 3- to 12-membered cycloalkyl, 3- to 12-membered cycloalkyl $C_{1-12}$ alkylene, 3- to 12-membered cycloalkyloxy, 3- to 12-membered heterocyclyl, 3- to 12-membered heterocyclyl $C_{1-12}$ alkylene, 3- to 12-membered heterocyclyloxy, 3- to 12-membered heterocycloalkyl, 3- to 12-membered heterocycloalkyl $C_{1-12}$ alkylene, 3- to 12-membered heterocycloalkyloxy, 5- to 10-membered heteroaryl, 5- to 10-membered heteroaryl $C_{1-12}$ alkylene, 5- to 10-membered heteroaryloxy, 6- to 10-membered aryl, 6- to 10-membered aryl $C_{1-12}$ alkylene, and 6- to 10-membered aryloxy.

"One or more" herein refers to an integer ranging from one to ten. For example, "one or more" refers to one, two, three, four, five, six, seven, eight, nine, or ten; or "one or more" refers to one, two, three, four, five, or six; or "one or more" refers to one, two, or three.

In some embodiments, the "one or more" is selected from the group consisting of one, two, three, four, five, six, and more. In some embodiments, the "one or more" is selected from the group consisting of one, two, three, four, five, and six. In some embodiments, the "one or more" is selected from the group consisting of one, two, and three. In some embodiments, the "one or more" is selected from the group consisting of one and two.

When any variable (e.g., R) occurs more than once in the constitution or structure of a compound, the definition of the variable in each case is independent. For example, if a group contains 2 R, the definition of each R is independent.

When a bond is crosslinked to two atoms of a ring (including monocyclic, fused, or spiro ring), the bond may be bonded to any atom on the ring (including monocyclic, fused, or spiro ring).

The term "halo" or "halogen" refers to fluorine, chlorine, bromine, and iodine.

The term "hydroxyl" refers to an -OH group.

The term "amino" refers to an -$NH_2$ group.

The term "cyano" refers to a -CN group.

The term "sulfydryl" refers to an -SH group.

The term "nitro" refers to an -$NO_2$ group.

The term "heteroatom" includes atoms of any element other than carbon or hydrogen. Preferred heteroatoms are boron, nitrogen, oxygen, sulfur, silicon, and phosphorus. In one embodiment, the heteroatom is selected from the group consisting of N, O, and S, wherein the nitrogen atom is optionally quaternized, and the nitrogen, sulfur, and phosphorus heteroatoms may be optionally oxidized (i.e., NO, S(O)p, and P(O)p, wherein p is 1 or 2).

The term "alkyl" refers to hydrocarbyl with a general formula of $C_nH_{2n+1}$. The alkyl may be linear or branched. For example, the term "$C_{1-6}$ alkyl" refers to alkyl containing 1 to 6 carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc.). Similarly, the alkyl moieties (i.e., alkyl) of alkoxy, alkylamino, dialkylamino, alkylsulfonyl, and alkylthio have the same definition as described herein. As another example, the term "$C_{1-3}$ alkyl" refers to alkyl containing 1 to 3 carbon atoms (e.g., methyl, ethyl, propyl, and isopropyl).

The term "alkoxy" refers to -O-alkyl.

The term "heteroalkyl" refers to an alkyl structure containing a heteroatom. Unless otherwise indicated, the heteroalkyl is usually alkyl containing 1 to 3 heteroatoms (preferably 1 or 2 heteroatoms) independently selected from the group consisting of sulfur, oxygen, and/or nitrogen. Usually, where more than one heteroatom is present, the heteroatoms are not adjacent to each other. Exemplary heteroalkyl groups include alkoxy, alkoxyalkyl, alkylamino, alkylaminoalkyl, dialkylamino, dialkylaminoalkyl, and the like.

The term "alkenyl" refers to linear or branched unsaturated aliphatic hydrocarbyl consisting of carbon atoms and hydrogen atoms and having at least one double bond. Non-limiting examples of the alkenyl include, but are not limited to, ethenyl, 1-propenyl, 2-propenyl, 1-butenyl, isobutenyl, 1,3-butadienyl, and the like.

The term "alkynyl" refers to linear or branched unsaturated aliphatic hydrocarbyl consisting of carbon atoms and hydrogen atoms and having at least one triple bond. Non-limiting examples of the alkynyl include, but are not limited to, ethynyl (-C≡CH), 1-propynyl (-C≡C-$CH_3$), 2-propynyl (-$CH_2$-C≡CH), 1,3-butadiynyl (-C≡C-C≡CH), and the like.

The term "cycloalkenyl" refers to a non-aromatic carbocyclic ring that is not fully saturated and may exist as a monocyclic ring, a bicyclic bridged ring, or a spiro ring. Unless otherwise indicated, the carbocyclic ring is usually a 4- to 20-membered ring, a 4- to 15-membered ring, a 4- to 10-membered ring, or a 4- to 8-membered ring. Non-limiting examples of the cycloalkenyl include, but are not limited to, cyclopentenyl, cyclopentadienyl, cyclohexenyl, cyclohexadienyl, cycloheptenyl, cycloheptadienyl, and the like.

The term "cycloalkyl" refers to a carbocyclic ring that is fully saturated and may exist as a monocyclic ring, a bridged ring, or a spiro ring. Unless otherwise indicated, the carbon ring is usually a 3- to 20-membered ring, a 3-to 15-membered ring, a 3- to 12-membered ring, or a 3- to 10-membered ring (e.g., a 5- to 8-membered ring). Non-limiting examples of the cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

The term "heterocycloalkyl" refers to a cyclic group that is fully saturated and may exist as a monocyclic ring, a bridged ring, or a spiro ring. Unless otherwise indicated, the heterocyclic ring is usually a 3- to 20-membered, 3- to 15-membered, 3- to 10-membered, 3- to 7-membered, 3- to 6-membered, or 3- to 5-membered ring containing 1 to 3 heteroatoms (preferably 1 or 2 heteroatoms) independently selected from the group consisting of boron, nitrogen, oxygen, sulfur, silicon, and/or phosphorus (preferably sulfur, oxygen, and/or nitrogen), wherein the nitrogen atom is optionally quaternized, and the nitrogen, sulfur and phosphorus heteroatoms may be optionally oxidized (i.e., NO, S(O)p, and P(O)p, wherein p is 1 or 2). Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-thioxanyl, 1,4-dioxanyl, thiomorpholinyl, 1,3-dithianyl, and 1,4-dithianyl; examples of 7-membered heterocycloalkyl include, but are not limited to, azepanyl, oxepanyl, and thiepanyl. Monocyclic heterocycloalkyl having 5 or 6 ring atoms is preferred. The term "heterocycloalkenyl" includes cycloalkenyl in which up to 3 carbon atoms, in one embodiment up to 2 carbon atoms, and in another embodiment 1 carbon atom, are each independently replaced by boron, nitrogen, oxygen, sulfur, silicon, or phosphorus (preferably O, S, or N), provided that at least one cycloalkenyl carbon-carbon double bond is retained, wherein the nitrogen atom is optionally quaternized, and the nitrogen, sulfur, and phosphorus heteroatoms may be optionally oxidized (i.e., NO, S(O)p, and P(O) p, wherein p is 1 or 2). A cyclic group that may exist as a monocyclic ring, a bridged ring, or a spiro ring may be a 3- to 20-membered ring, a 3- to 15-membered ring, a 3- to 12-membered ring, or a 3- to 10-membered ring (e.g., a 5- to 8-membered ring). Examples of the heterocycloalkenyl include, but are not limited to, dihydropyrrolyl, tetrahydropyridinyl, tetrahydroazepinyl, or azaspirooctenyl.

Unless otherwise indicated, the carbocyclic ring is usually a 4- to 20-membered ring, a 4- to 15-membered ring, a 4- to 12-membered ring, or a 4- to 8-membered ring (or a 5- to 6-membered ring). Non-limiting examples of the cycloalkenyl include, but are not limited to, cyclopentenyl, cyclopentadienyl, cyclohexenyl, cyclohexadienyl, cycloheptenyl, cycloheptadienyl, and the like.

The term "heterocyclyl" refers to a non-aromatic ring that is fully saturated or partially unsaturated (but not a fully unsaturated heteroaromatic group) and may exist as a monocyclic ring, a bridged ring, or a spiro ring. Unless otherwise indicated, the heterocyclic ring is usually a 3- to 20-membered, 3- to 15-membered, 3- to 10-membered, or 3- to 7-membered (or 5- to 6-membered) ring containing 1 to 3 heteroatoms (preferably 1 or 2 heteroatoms) independently selected from the group consisting of boron, nitrogen, oxygen, sulfur, silicon, and/or phosphorus (preferably sulfur, oxygen, and/or nitrogen), wherein the nitrogen atom is optionally quaternized, and the nitrogen, sulfur and phosphorus heteroatoms may be optionally oxidized (i.e., NO, S(O)p, and P(O)p, wherein p is 1 or 2). Non-limiting examples of the heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, thiomorpholinyl, tetrahydrothienyl, and the like.

The term "aryl" refers to an all-carbon aromatic monocyclic or fused polycyclic group having a conjugated $\pi$-electron system. For example, the aryl may have 6-20 carbon atoms, 6-14 carbon atoms, or 6-12 carbon atoms (6-10 carbon atoms, or 6 carbon atoms, i.e., phenyl). Non-limiting examples of the aryl include, but are not limited to, phenyl, naphthyl, anthryl, and the like.

The term "heteroaryl" refers to a monocyclic or fused polycyclic system that contains at least one ring atom selected from the group consisting of N, O and S, with the remaining ring atoms being C, and that has at least one aromatic ring. Preferably, the heteroaryl has a single 4- to 8-membered ring, particularly a 5- to 8-membered ring (or a 5- to 6-membered ring), or has multiple fused rings containing 6 to 20, 6 to 15, or 6 to 14, particularly 6 to 10 ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

The group or structural fragment such as -L- and specific options thereof in the present disclosure may optionally adopt a left-to-right reading order, correspondingly connected to the left side group and the right side group of said group or fragment in the general formula, respectively; for example, when L is selected from

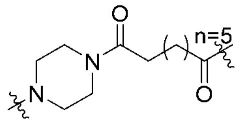,

according to the left-to-right reading order, the left side of L is connected to the fragment

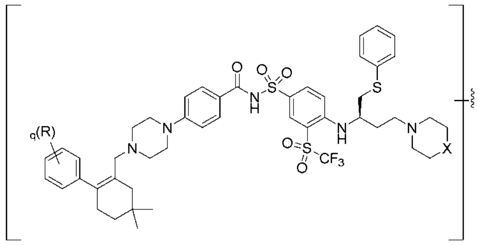

corresponding to the left side in the general formula, and the right side is connected to the right side fragment ULM, thus forming

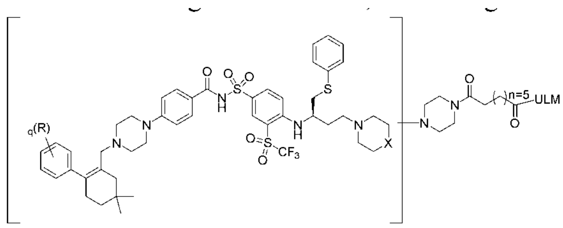.

Optionally, the group or structural fragment such as -L- and specific options thereof in the present disclosure may adopt a right-to-left reading order, correspondingly connected to the left side group and the right side group of said group or fragment in the general formula, respectively; for example, when L is selected from

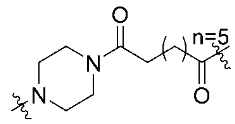,

according to the right-to-left reading order, the right side of L is connected to the fragment

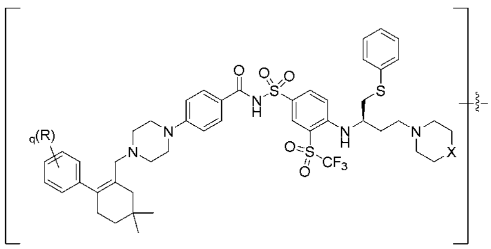

corresponding to the left side in the general formula, and the left side is connected to the fragment ULM corresponding to the right side in the general formula, thus forming a fragment such as

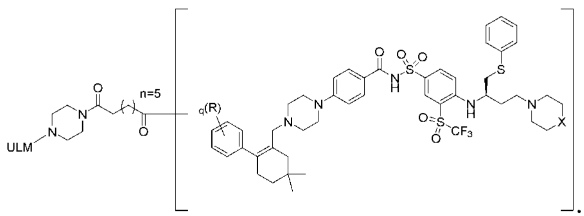.

The other groups are as described above.

The term "treat", "treating", or "treatment" means administering the compound or formulation described in the present disclosure to ameliorate or eliminate a disease or one or more symptoms related to the disease, and includes:

(i) inhibiting the disease or disease state, i.e., arresting its progression; and
(ii) alleviating the disease or disease state, i.e., causing its regression.

The term "prevent", "preventing", or "prevention" means administering the compound or formulation described in the present disclosure to prevent a disease or one or more symptoms related to the disease, and includes preventing the occurrence of the disease or disease state in a mammal, particularly when such a mammal is predisposed to the disease state but has not yet been diagnosed with it.

The term "therapeutically effective amount" refers to an amount of the compound of the present disclosure for (i) treating or preventing a specific disease, condition, or disorder; (ii) relieving, ameliorating, or eliminating one or more symptoms of a specific disease, condition, or disorder, or (iii) preventing or delaying the onset of one or more symptoms of the specific disease, condition, or disorder described herein. The amount of the compound of the present disclosure composing the "therapeutically effective amount" varies depending on the compound, the disease state and its severity, the mode of administration, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may refer to a metal salt, an ammonium salt, a salt formed with an organic base, a salt formed with an inorganic acid, a salt formed with an organic acid, a salt formed with a basic or acidic amino acid, etc.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those excipients that do not have a significant irritating effect on an organism and do not impair the biological activity and properties of the active compound. Suitable excipients are well known to those skilled in the art, for example, carbohydrates, waxes, water-soluble and/or water-swellable polymers, hydrophilic or hydrophobic materials, gelatin, oils, solvents, water, etc.

The word "comprise" and variations thereof such as "comprises" or "comprising" will be understood in an open, non-exclusive sense, i.e., "including but not limited to".

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogen atoms. A valence tautomer includes the interconversion via recombination of some bonding electrons.

The present disclosure also includes isotopically labeled compounds of the present disclosure, which are identical to those recited herein but have one or more atoms replaced by an atom having an atomic mass or mass number different from the atomic mass or mass number generally found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, $^{36}Cl$, and the like. For example, it should be understood that compounds formed by replacing one or more hydrogen atoms in the compound of formula I of the present disclosure with a deuterium atom still fall within the scope of the compound of formula I of the present disclosure.

Certain isotopically labeled compounds of the present disclosure (e.g., those labeled with $^{3}H$ and $^{14}C$) can be used to analyze compounds and/or substrate tissue distribution. Tritiated (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$, and $^{18}F$, can be used in positron emission tomography (PET) studies to determine substrate occupancy. The isotopically labeled compounds of the present disclosure can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., $^{2}H$) may provide certain therapeutic advantages (e.g., increased *in vivo* half-life or reduced dose) resulting from greater metabolic stability and hence may be

preferred in some circumstances in which deuterium substitution may be partial or complete, wherein partial deuterium substitution refers to substitution of at least one hydrogen with at least one deuterium.

The compound of the present disclosure may be asymmetrical, for example, having one or more stereoisomers. Unless otherwise stated, all stereoisomers are included, for example, enantiomers and diastereoisomers. The compound containing asymmetric carbon atoms of the present disclosure may be isolated in an optically active pure form or in a racemic form. The optically active pure form may be separated from a racemic mixture or may be synthesized using a chiral raw material or a chiral reagent.

The pharmaceutical composition of the present disclosure may be prepared by combining the compound of the present disclosure with a suitable pharmaceutically acceptable excipient.

The pharmaceutical composition of the present disclosure can be manufactured using methods well known in the art, such as conventional mixing, dissolving, granulating, dragee-making, levigating, emulsifying, lyophilizing, etc. In all of the administration methods of the compound of formula I described herein, the daily dose administered is from 0.001 mg/kg body weight to 2000 mg/kg body weight, given in individual or separated doses.

All patents, patent applications, and other identified publications are explicitly incorporated herein by reference for the purpose of description and disclosure. Any reference to these publications herein shall not be construed as an admission that the publications form part of the commonly recognized knowledge in the art.

The compounds of the present disclosure can be prepared using a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions in the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthetic procedure or a reaction process based on the existing embodiments.

An important consideration in synthetic route planning in the art is the selection of suitable protecting groups for reactive functional groups (e.g., amino in the present disclosure). For example, reference may be made to Greene's Protective Groups in Organic Synthesis (4th Ed.) Hoboken, New Jersey: John Wiley & Sons, Inc.

The compounds or the pharmaceutically acceptable salts thereof of the present disclosure can be prepared using the following preparation routes:

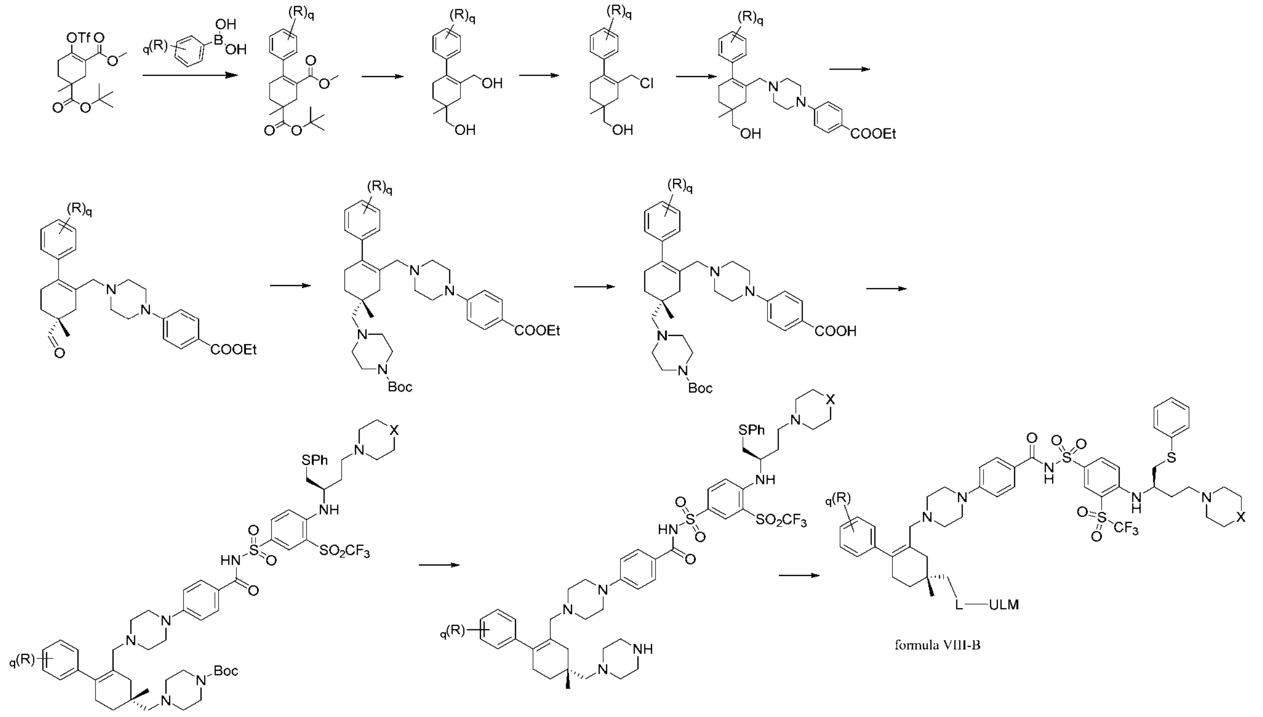

formula VIII-B

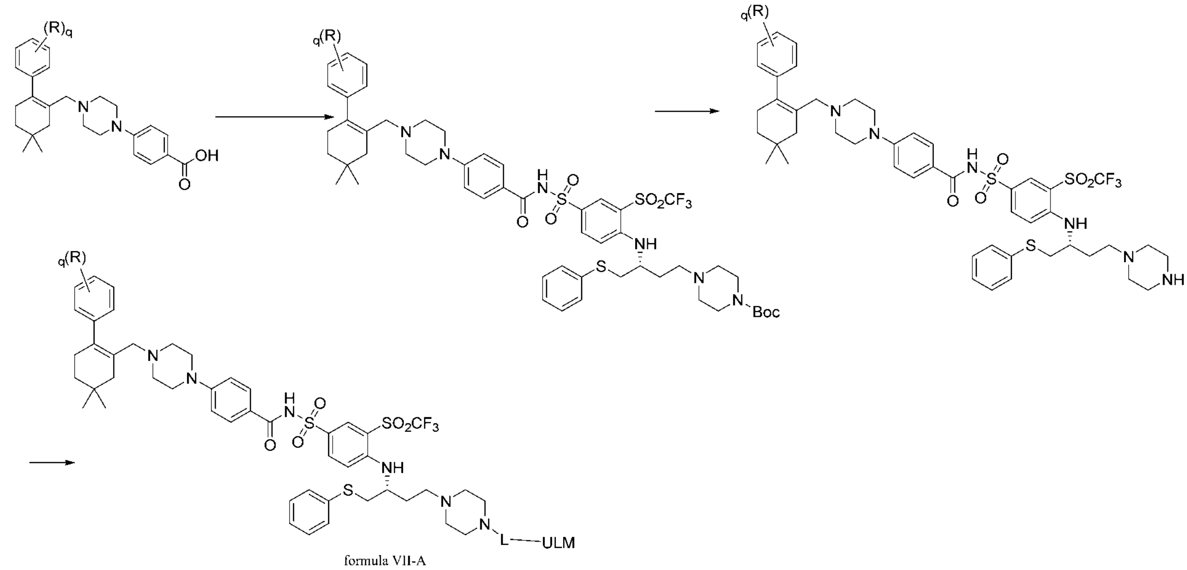

formula VII-A

wherein R, q, L, ULM, or X is as defined in the present disclosure.

The following abbreviations are used in the present disclosure:

HATU represents *O*-(7-azabenzotriazol-1-yl)-*N,N,N',N'*-tetramethyluronium hexafluorophosphate; DCM represents dichloromethane; MeOH represents methanol; DIPEA represents *N*-ethyldiisopropylamine; Pd(dppf)$Cl_2$ represents [1,1'-bis(diphenylphosphino)ferrocene]palladium(II) dichloride; PE represents petroleum ether; EA represents ethyl acetate; $Tf_2O$ represents trifluoromethanesulfonic anhydride; $Pd(PPh_3)_4$ represents tetrakis(triphenylphosphine)palladium; LAH represents lithium aluminum hydride; NaOH represents sodium hydroxide; DMSO represents dimethyl sulfoxide; DMAP represents 4-dimethylaminopyridine; EDCI represents carbodiimide hydrochloride; Boc represents tert-butoxycarbonyl; and Et represents ethyl.

For clarity, the present disclosure is further described with the following examples, which are, however, not intended to limit the scope of the present disclosure. All the reagents used in the present disclosure are commercially available and can be used without further purification.

## DETAILED DESCRIPTION

The following specific examples are intended to enable those skilled in the art to more clearly understand and

implement the present disclosure. These specific examples should not be considered as limitations on the scope of the present disclosure, but merely as exemplary descriptions and typical representatives of the present disclosure.

**Example 1: Preparation of Compound 1**

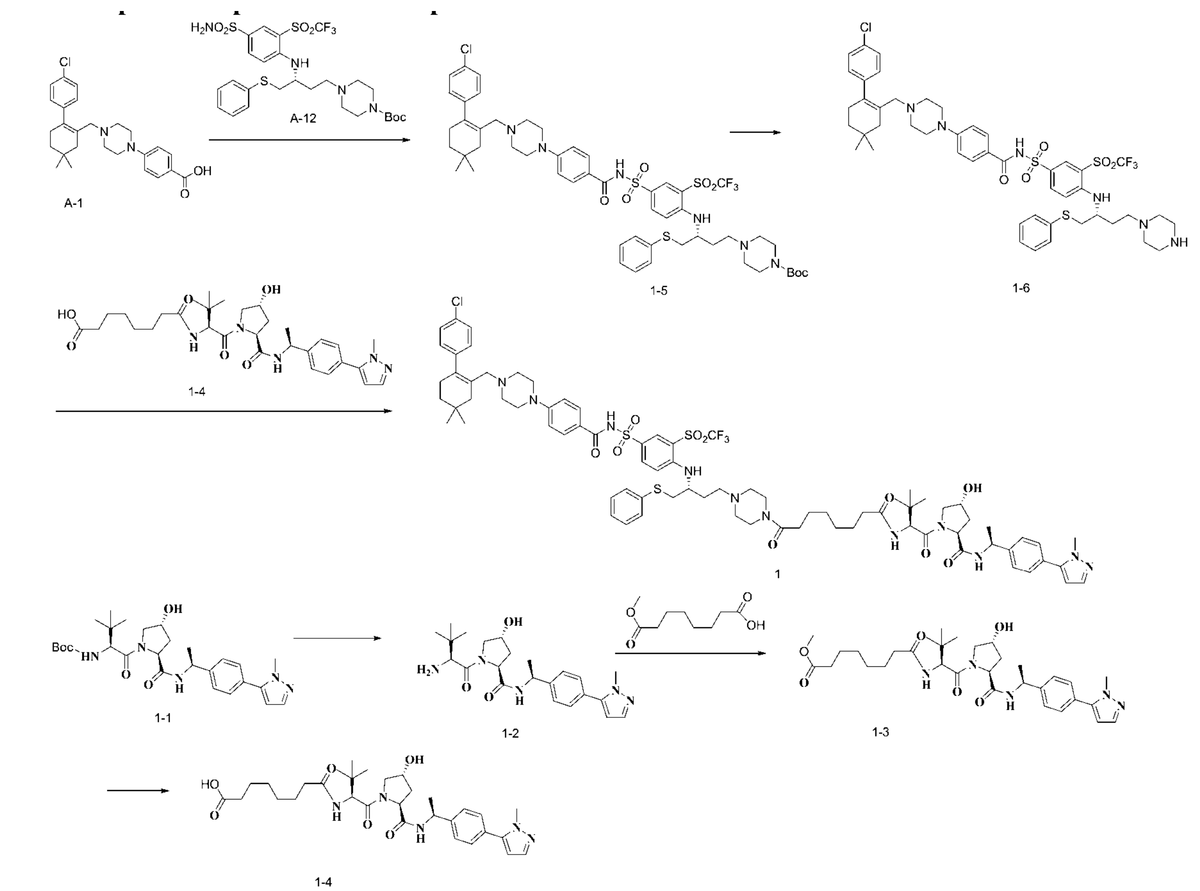

A-1 A-12 1-5 1-6

1-4 1

1-1 1-2 1-3

1-4

**Preparation of intermediate 1-2**

Intermediate 1-1 (400 mg) was dissolved in dichloromethane, and trifluoroacetic acid (1088 μL, 15.2 mmol) was added in two batches. The mixture was reacted at room temperature overnight. After the reaction was completed as monitored by TLC, the mixture was concentrated at 45 °C to give a crude product of intermediate 1-2, which was directly used in the next step without purification.

LCMS (ESI) m/z: 428.3 $[M+H]^+$.

**Preparation of intermediate 1-3**

Intermediate 1-2 (100 mg) was dissolved in dichloromethane, and monomethyl suberate (53 mg), triethylamine (142 mg), and HATU (133 mg) were added. The mixture was reacted at room temperature for 3 h. After the reaction was completed as monitored by TLC, the reaction solution was directly separated and purified by silica gel column chromatography (DCM:MeOH) to give 105 mg of intermediate 1-3.

LCMS (ESI) m/z: 598.3 $[M+H]^+$.

**Preparation of intermediate 1-4**

Intermediate 1-3 (0.43 g) was dissolved in methanol, and an aqueous lithium hydroxide solution (2 N, 2.16 mL) was added. The mixture was stirred at room temperature overnight. The reaction solution was adjusted to pH 5-6 with 3 N hydrochloric acid, extracted with DCM, subjected to liquid separation, dried, and concentrated to give 0.27 g of

intermediate 1-4.

LCMS (ESI) m/z: 584.3 $[M+H]^+$.

**Preparation of intermediate 1-5**

Intermediates A-12 (0.25 g) and A-1 (0.21 g) were dissolved in 3 mL of DCM, and EDCI·HCl (0.092 g), DMAP (0.059 g), and triethylamine (199 μL) were added. The mixture was stirred at room temperature overnight. The reaction solution was diluted with a DCM-methanol mixed solution, adjusted to pH 5-6 with 10% acetic acid, and extracted, followed by liquid separation. The organic phase was washed with saturated sodium bicarbonate to pH 7-8, subjected to liquid separation, dried, and concentrated, and the residue was purified by column chromatography (DCM:MeOH) to give 0.25 g of intermediate 1-5.

LCMS (ESI) m/z: 1073.4 $[M+H]^+$.

**Preparation of intermediate 1-6**

Intermediate 1-5 (48 mg) was dissolved in dichloromethane, and trifluoroacetic acid (135 μL) was added in two batches. The mixture was reacted at room temperature overnight. After the reaction was completed as monitored by TLC, the mixture was concentrated at 45 °C to give a crude product of intermediate 1-6, which was directly used in the next step without purification.

LCMS (ESI) m/z: 487.7 $[M + 2H]^{2+}$.

**Preparation of compound 1**

Intermediate 1-6 (91 mg) was dissolved in dichloromethane, and intermediate 1-4 (65 mg), triethylamine (130 μL), and HATU (53 mg) were added, the mixture was reacted at room temperature for 1.5 h. The mixture was then diluted with DCM and extracted with saturated ammonium chloride, followed by liquid separation. The organic phase was washed with water, followed by liquid separation. The aqueous phase was back-extracted with DCM. The organic phases were combined and washed with saturated sodium chloride, followed by liquid separation. The resulting organic phase was dried over anhydrous sodium sulfate, filtered under vacuum, and concentrated, and the crude product was subjected to preparative liquid chromatography to give 71 mg of compound 1.

LCMS (ESI) m/z: 770.5 $[M + 2H]^{2+}$.

$^1H$ NMR (500 MHz, $CDCl_3$) δ 8.34 (d, J = 1.8 Hz, 1H), 8.17 (s, 1H), 8.09 (dd, J = 9.2, 1.7 Hz, 1H), 7.70 (d, J = 8.8 Hz, 2H), 7.50 (d, J = 1.8 Hz, 1H), 7.39 (dt, J = 11.5, 7.7 Hz, 8H), 7.31 - 7.27 (m, 4H), 7.07 (t, J = 8.2 Hz, 1H), 6.98 (d, J = 8.3 Hz, 2H), 6.76 (d, J = 8.9 Hz, 2H), 6.61 (d, J = 9.5 Hz, 1H), 6.44 (t, J = 11.9 Hz, 1H), 6.27 (d, J = 1.8 Hz, 1H), 5.09 (dt, J = 14.2, 7.0 Hz, 2H), 4.72 (t, J = 8.0 Hz, 1H), 4.61 (d, J = 8.9 Hz, 1H), 4.51 (s, 1H), 4.11 (d, J = 11.5 Hz, 1H), 3.87 (s, 3H), 3.67 (s, 1H), 3.60 (dd, J = 11.2, 3.1 Hz, 1H), 3.45 (s, 2H), 3.34 (s, 4H), 3.10 (dd, J = 13.8, 4.9 Hz, 1H), 3.06 - 2.96 (m, 3H), 2.62 (d, J = 13.9 Hz, 2H), 2.55 (s, 4H), 2.43 (ddd, J = 24.1, 12.8, 5.5 Hz, 6H), 2.32 - 2.15 (m, 8H), 2.01 (d, J = 9.3 Hz, 2H), 1.71 (dd, J = 23.3, 17.4 Hz, 1H), 1.46 (t, J = 7.0 Hz, 6H), 1.38 - 1.23 (m, 6H), 1.04 (s, 10H), 0.97 (s, 6H).

**Example 2: Preparation of Compound 2**

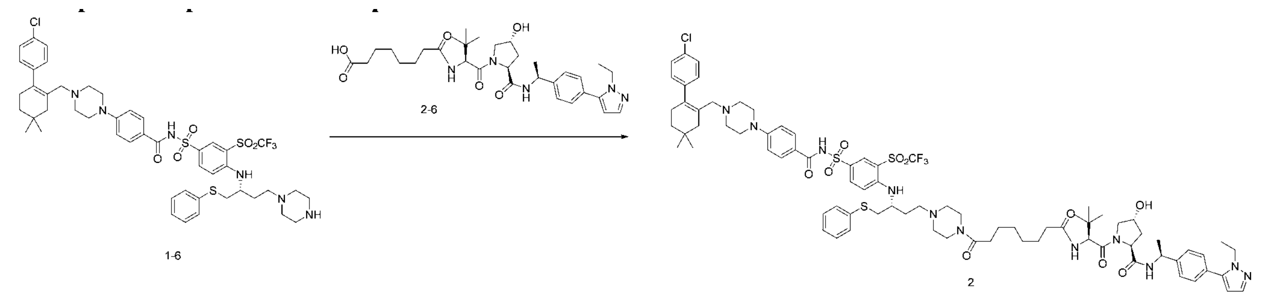

1-6 2-6 2

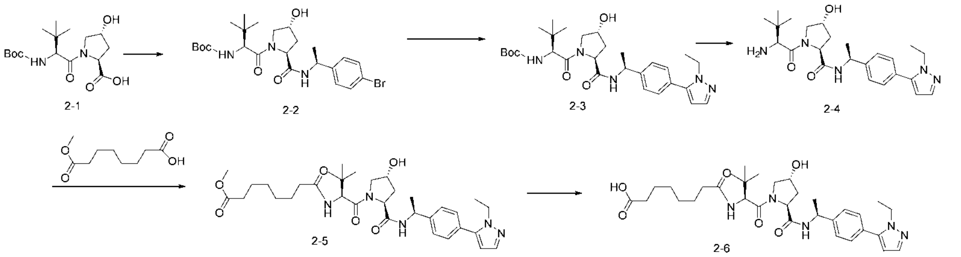

2-1 2-2 2-3 2-4 2-5 2-6

## Preparation of intermediate 2-2

Intermediate 2-1 (5.0 g) and (S)-(-)-1-(4-bromophenyl)ethylamine (3.5 g) were dissolved in dichloromethane, and HATU (8.3 g) and DIPEA (5.6 g) were added. The mixture was reacted at room temperature overnight. The mixture was then extracted with water, followed by liquid separation. The organic phase was washed with saturated sodium chloride, subjected to liquid separation, dried, and concentrated. The crude product was purified by column chromatography (DCM:MeOH) to give 7.5 g of intermediate 2-2.
LCMS (ESI) m/z: 526.2 $[M+H]^+$.

## Preparation of intermediate 2-3

Intermediate 2-2 (1.0 g) and 1-ethyl-5-(4,4,5,5-tetramethyl-1,3,2-dioxaborolan-2-yl)-1*H*-pyrazole (0.51 g) were dissolved in 20 mL of dioxane and 10 mL of water, and potassium carbonate (1.3 g) and Pd(dppf)$Cl_2$ (0.14 g) were added. The mixture was purged with nitrogen and then reacted at 105 °C for 5 h. The mixture was then cooled, filtered through diatomite under vacuum, and concentrated to dryness. Water and DCM were added for extraction, followed by liquid separation. The organic phase was washed with saturated sodium chloride, subjected to liquid separation, dried, and concentrated. The crude product was subjected to column chromatography (DCM:MeOH) to give 1.0 g of intermediate 2-3.
LCMS (ESI) m/z: 542.3 $[M+H]^+$.

## Preparation of intermediate 2-4

Intermediate 2-3 (890 mg) was dissolved in dichloromethane, and trifluoroacetic acid (2448 μL) was added in two batches. The mixture was reacted at room temperature overnight. After the reaction was completed as monitored by TLC, the mixture was concentrated at 45 °C to give a crude product of intermediate 2-4, which was directly used in the next step without purification.
LCMS (ESI) m/z: 442.3 $[M+H]^+$.

## Preparation of intermediate 2-5

Intermediate 2-4 (720 mg) was dissolved in dichloromethane, and monomethyl suberate (740 mg), triethylamine (2.0 mL), and HATU (930 mg) were added. The mixture was reacted at room temperature overnight. After the reaction was completed as monitored by TLC, the reaction solution was extracted with water and dichloromethane, followed by liquid separation. The organic phase was washed with saturated sodium chloride, subjected to liquid separation, dried, and concentrated, and the crude product was separated by silica gel column chromatography (DCM:MeOH) to give 520 mg of intermediate 2-5.
LCMS (ESI) m/z: 612.3 $[M+H]^+$.

**Preparation of intermediate 2-6**

Intermediate 2-5 (0.52 g) was dissolved in methanol, and an aqueous lithium hydroxide solution (2 N, 2.54 mL) was added. The mixture was stirred at room temperature overnight. The reaction solution was adjusted to pH 5-6 with 3 N hydrochloric acid, extracted with DCM, subjected to liquid separation, dried, and concentrated to give 0.45 g of intermediate 2-6.

LCMS (ESI) m/z: 598.3 $[M+H]^+$.

**Preparation of compound 2**

Intermediate 1-6 (82 mg) was dissolved in dichloromethane, and intermediate 2-6 (60 mg), triethylamine (117 μL), and HATU (48 mg) were added. The mixture was reacted at room temperature for 1.5 h. The mixture was then diluted with DCM and extracted with saturated ammonium chloride, followed by liquid separation. The organic phase was washed with water, followed by liquid separation. The aqueous phase was back-extracted with DCM. The organic phases were combined and washed with saturated sodium chloride, followed by liquid separation. The resulting organic phase was dried over anhydrous sodium sulfate, filtered under vacuum, and concentrated, and the crude product was subjected to preparative liquid chromatography to give 83 mg of compound 2.

LCMS (ESI) m/z: 777.0 $[M + 2H]^{2+}$.

$^1H$ NMR (500 MHz, $CDCl_3$) δ 8.34 (d, J = 1.8 Hz, 1H), 8.17 (s, 2H), 8.12 - 8.07 (m, 1H), 7.69 (t, J = 9.1 Hz, 2H), 7.54 (d, J = 1.8 Hz, 1H), 7.37 (dt, J = 12.9, 7.9 Hz, 7H), 7.30 (t, J = 8.0 Hz, 4H), 7.07 (t, J = 7.8 Hz, 1H), 6.99 (d, J = 8.3 Hz, 2H), 6.76 (d, J = 8.9 Hz, 2H), 6.61 (d, J = 9.5 Hz, 1H), 6.48 (d, J = 8.7 Hz, 1H), 6.24 (d, J = 1.7 Hz, 1H),5.09 (dd, J = 14.2, 6.9 Hz, 2H), 4.70 (dd, J = 26.3, 18.2 Hz, 2H), 4.62 (d, J = 8.9 Hz, 1H), 4.51 (s, 1H), 4.14 (dt, J = 20.7, 10.2 Hz, 3H), 3.89 (s, 1H), 3.75 - 3.53 (m, 3H), 3.52 - 3.43 (m, 3H), 3.36 (s, 4H), 3.14 - 3.08 (m, 3H), 3.06 - 2.97 (m, 1H), 2.61 (s, 4H), 2.51 - 2.39 (m, 6H), 2.35 - 2.16 (m, 8H), 2.04 (s, 2H), 1.76 - 1.66 (m, 1H), 1.47 (t, J = 6.5 Hz, 5H), 1.40 (t, J = 7.2 Hz, 3H), 1.28 (s, 6H), 1.04 (s, 9H), 0.98 (s, 6H).

**Example 3: Preparation of Compound 3**

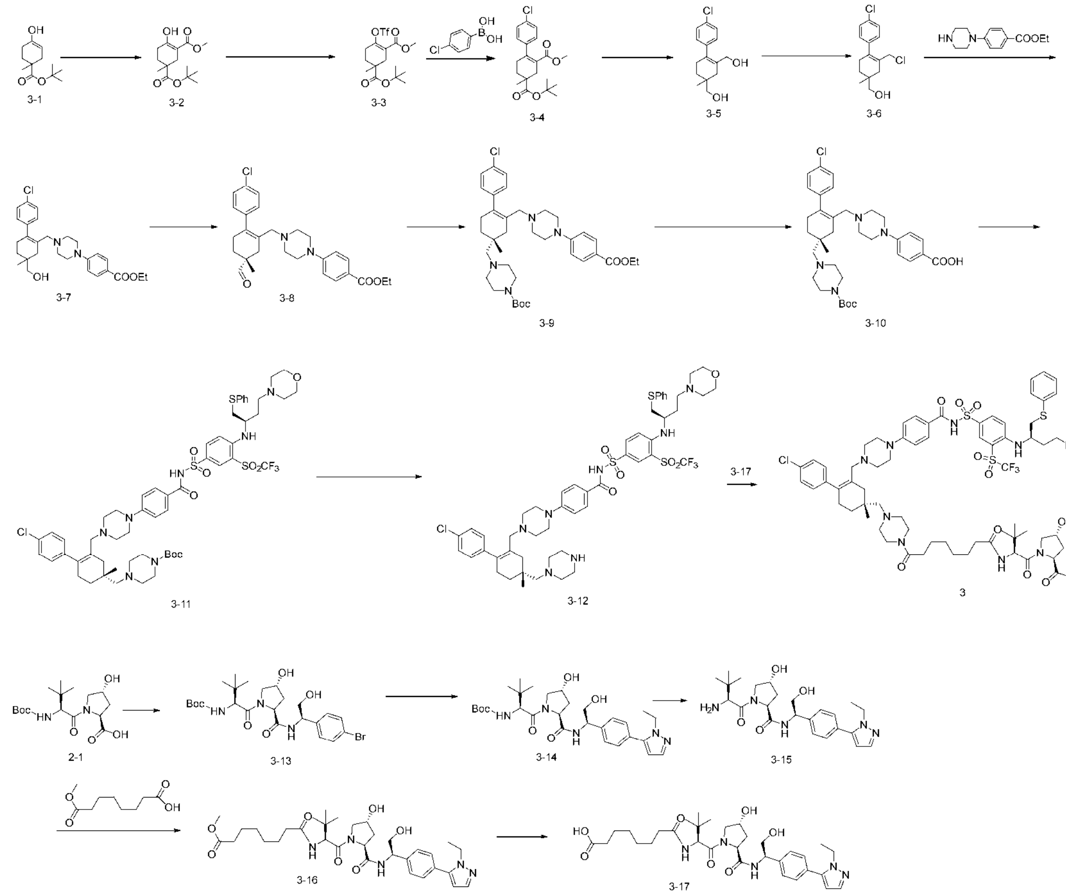

3-1 3-2 3-3 3-4 3-5 3-6 3-7 3-8 3-9 3-10 3-11 3-12 3 2-1 3-13 3-14 3-15 3-16 3-17

### Preparation of intermediate 3-2

Dimethyltetrahydrofuran (300 mL) was added to a reaction flask and then stirred at 0 °C under a nitrogen atmosphere. After the desired temperature was reached, NaH (25.6 g) was added. Intermediate 3-1 (20 g) was dissolved in 50 mL of dimethyltetrahydrofuran, and the mixture was slowly added dropwise to the above suspension. Dimethyl carbonate (25.6 g) was added, and the resulting mixture was refluxed at 75 °C for 5 h. After the reaction was completed as monitored by LC-MS, the reaction solution was cooled to room temperature and slowly added to 300 mL of ice water to quench the reaction. After the addition was completed, the mixture was naturally separated into layers. The organic layer was dried over anhydrous sodium sulfate and filtered under vacuum, and the filtrate was concentrated to give a crude product (25 g). The crude product was subjected to column chromatography (PE:EA) to give 10 g of intermediate 3-2.

### Preparation of intermediate 3-3

Intermediate 3-2 (9.0 g) was dissolved in 108 mL of DCM, and the mixture was stirred at -78 °C. After the desired temperature was reached, DIPEA (21.5 g) was added. $Tf_2O$ (28.2 g) was slowly added dropwise to the reaction solution. After the addition was completed, the mixture was stirred for 30 min and then allowed to react at room temperature overnight. Under an ice bath, the mixture was extracted with 100 mL of saturated sodium bicarbonate, followed by liquid separation. The organic layer was washed with saturated sodium chloride, followed by liquid separation. The resulting organic layer was dried and concentrated to give 50 g of a crude product, which was directly used in the next step without purification.

### Preparation of intermediate 3-4

Intermediate 3-3 (2.6 g) was dissolved in 40 mL of a toluene-ethanol (2:1) mixed solvent. *p*-Chlorophenylboronic acid (1.2 g) was added, and then $Pd(PPh_3)_4$ (0.15 g) was added, followed by the addition of an aqueous sodium carbonate solution (2 N, 6.45 mL). The mixture was purged with nitrogen and then reacted at 90 °C for 8 h. The mixture was then filtered under vacuum, and the filter cake was washed with EA. The filtrates were combined and concentrated. EA and water were added for extraction, followed by liquid separation. The EA layer was dried and concentrated. The crude

product was subjected to column chromatography to give 1.2 g of intermediate 3-4.

ESI-MS: m/z = 365.1 $[M+H]^+$.

### Preparation of intermediate 3-5

Intermediate 3-4 (6.5 g) was dissolved in 90 mL of anhydrous tetrahydrofuran, and the mixture was stirred at 0 °C. LAH (1.7 g) was added in three batches, and the resulting mixture was reacted at 0 °C for 3 h. Water (1.7 mL) was added, and the mixture was stirred. Then, 1.7 mL of a 15% aqueous NaOH solution was added, followed by the addition of 5.1 mL of water. The mixture was allowed to be stirred at room temperature for 0.5 h. Anhydrous sodium sulfate was added, and the resulting mixture was stirred for 3 h and then filtered through diatomite under vacuum. The solid on the flask wall was washed with EA. The filtrates were combined, dried over anhydrous sodium sulfate, and concentrated to give 4.5 g of a crude product, which was then slurried with 45 mL of a DCM:n-hexane (1:9) mixed solution. The resulting slurry was filtered under vacuum to give 3.6 g of intermediate 3-5.

ESI-MS: m/z = 289.1 $[M+Na]^+$.

### Preparation of intermediate 3-6

Intermediate 3-5 (1.0 g) was dissolved in 15 mL of DCM, and the mixture was stirred at -30 °C. NCS (0.55 g) was dissolved in 3 mL of DCM, and the mixture was added to the above mixture, followed by the addition of dimethyl sulfide (300 μL). The resulting mixture was reacted at -30 °C for 2 h. After the reaction was completed, the mixture was extracted with DCM and water, followed by liquid separation. The DCM phase was dried and concentrated to give 1.2 g of a crude product, which was then subjected to column chromatography (PE:EA) to give 0.56 g of intermediate 3-6.

### Preparation of intermediate 3-7

Intermediate 3-6 (3.9 g) was dissolved in 78 mL of acetonitrile, and ethyl (4-piperazin-1-yl)benzoate (4.8 g) and potassium carbonate (5.7 g) were added in sequence. The mixture was reacted at room temperature overnight. After the reaction was completed, the mixture was extracted with ethyl acetate and water, followed by liquid separation. The organic phase was washed with saturated sodium chloride, followed by liquid separation. The organic phase was dried and concentrated to give 7 g of a crude product, which was then mixed with 5 g of silica gel and subjected to column chromatography (PE:EA) to give 4.77 g of intermediate 3-7.

ESI-MS: m/z = 483.2 $[M+H]^+$.

### Preparation of intermediate 3-8

Oxalyl chloride (4.37 mL) was dissolved in dichloromethane (43.2 mL), and the mixture was purged with nitrogen and cooled to -78 °C. A solution of DMSO (5.47 mL) in dichloromethane was added. The mixture was stirred for 15 min, and then a solution of intermediate 3-7 (2.4 g) in dichloromethane was added. The mixture was stirred for 30 min, and then triethylamine (21.4 mL) was added. After 10 min, the resulting mixture was allowed to be stirred at room temperature. After 2.5 h, the pH was adjusted to neutral with saturated sodium bicarbonate, and the mixture was extracted three times with dichloromethane. The organic phase obtained from the extraction was concentrated, and the residue was subjected to column chromatography (PE/EA) to give intermediate 3-8 (200 mg).

### Preparation of intermediate 3-9

Intermediate 3-8 (250 mg) and tert-butyl piperazine-1-carboxylate (145.36 mg) were dissolved in anhydrous dichloromethane (10 mL), and triethylamine (722.9 μL) was added. The mixture was purged with nitrogen and stirred at room temperature. After half an hour, sodium triacetoxyborohydride (772 mg) was added, and the mixture was stirred at room temperature for 16 h. Then, 50 mL of water was added to the system to quench the reaction. The mixture was extracted with 50 mL of dichloromethane. The organic phase obtained from the extraction was concentrated, and the residue was subjected to column chromatography (PE/EA) to give intermediate 3-9 (200 mg).

### Preparation of intermediate 3-10

Intermediate 3-9 (200 mg) was dissolved in a system of methanol/tetrahydrofuran/water (5/1/1, 28 mL), and lithium hydroxide monohydrate (387 mg) was added. The mixture was stirred at room temperature for 16 h. The pH of the system was adjusted to neutral with 1 N hydrochloric acid, and the mixture was extracted with water and ethyl acetate. The organic phase was dried and concentrated to give intermediate 3-10 (192 mg).

**Preparation of intermediate 3-11**

Intermediate 3-10 (192 mg) and (*R*)-4-((4-morpholin-1-(phenylthio)butan-2-yl)amino)-3-((trifluoromethyl)sulfonyl)benzenesulfonamide (187 mg) were dissolved in dichloromethane (4 mL), and EDCI (148 mg) and DMAP (94 mg) were added. The mixture was stirred at room temperature for 16 h. The pH of the system was adjusted to neutral with a saturated sodium bicarbonate solution, and the mixture was extracted with dichloromethane. The organic phase was concentrated, and the residue was subjected to column chromatography (DCM/MeOH) to give intermediate 3-11 (332 mg).

**Preparation of intermediate 3-12**

Intermediate 3-11 (332 mg) was dissolved in dichloromethane (3 mL), and a solution of hydrochloric acid in dioxane (0.287 mL) was added. The mixture was stirred at room temperature for 1 h. The system was directly concentrated to give a crude product of intermediate 3-12 (300 mg).

**Preparation of intermediate 3-13**

Intermediate 2-1 (3.0 g) and (*R*)-2-amino-2-(4-bromophenyl)ethanol (1.88 g) were dissolved in dichloromethane, and EDCI (2.34 g) and DIPEA (2.25 g) were added. The mixture was reacted at room temperature overnight. A 5% aqueous hydrochloric acid solution (100 mL) was added to the reaction solution, and the mixture was extracted with dichloromethane. The aqueous phase was then back-extracted once. The organic phases were combined, washed with a saturated aqueous sodium bicarbonate solution and saturated brine, dried over anhydrous sodium sulfate, and filtered. The filtrate was concentrated under reduced pressure to give 3.8 g of intermediate 3-13.
ESI-MS: m/z = 543.1 $[M+H]^+$.

**Preparation of intermediate 3-14**

Intermediate 3-13 (1.0 g) and 1-ethyl-5-(4,4,5,5-tetramethyl-1,3,2-dioxaborolan-2-yl)-1*H*-pyrazole (0.51 g) were dissolved in 20 mL of dioxane and 10 mL of water, and potassium carbonate (1.3 g) and $Pd(dppf)Cl_2$ (0.14 g) were added. The mixture was purged with nitrogen and then reacted at 105 °C for 5 h. The mixture was then cooled, filtered through diatomite under vacuum, and concentrated to dryness. Water and DCM were added for extraction, followed by liquid separation. The organic phase was washed with saturated sodium chloride, subjected to liquid separation, dried, and concentrated. The crude product was subjected to column chromatography (DCM:MeOH) to give 1.0 g of intermediate 3-14.
ESI-MS: m/z = 558.2 $[M+H]^+$.

**Preparation of intermediate 3-15**

Intermediate 3-14 (890 mg) was dissolved in dichloromethane, and trifluoroacetic acid (2448 μL) was added in two batches. The mixture was reacted at room temperature overnight. After the reaction was completed as monitored by TLC, the mixture was concentrated at 45 °C to give a crude product of intermediate 3-15, which was directly used in the next step.
ESI-MS: m/z = 458.3 $[M+H]^+$.

**Preparation of intermediate 3-16**

Intermediate 3-15 (720 mg) was dissolved in dichloromethane, and monomethyl suberate (740 mg), triethylamine (2.0 mL), and HATU (930 mg) were added. The mixture was reacted at room temperature overnight. After the reaction was completed as monitored by TLC, the reaction solution was extracted with water and dichloromethane, followed by liquid separation. The organic phase was washed with saturated sodium chloride, subjected to liquid separation, dried, and concentrated, and the crude product was separated by silica gel column chromatography (DCM:MeOH) to give 520 mg of intermediate 3-16.
ESI-MS: m/z = 628.3 $[M+H]^+$.

**Preparation of intermediate 3-17**

Intermediate 3-16 (0.52 g) was dissolved in methanol, and an aqueous lithium hydroxide solution (2 N, 2.54 mL) was added. The mixture was stirred at room temperature overnight. The reaction solution was adjusted to pH 5-6 with 3 N

hydrochloric acid, extracted three times with DCM, subjected to liquid separation, dried, and concentrated to give 0.45 g of intermediate 3-17.

ESI-MS: m/z = 614.3 $[M+H]^+$.

### Preparation of compound 3

Intermediate 3-12 (121 mg) was dissolved in DMF, and intermediate 3-17 (70 mg), DIPEA (148 mg), and HATU (65 mg) were added. The mixture was reacted at room temperature for 3 h. The crude product from the reaction solution was purified by preparative liquid chromatography to give the target compound 3 (46 mg).

ESI-MS: m/z = 828.1 $[M+2H]^{2+}$.

### Example 4: Preparation of Compound 4

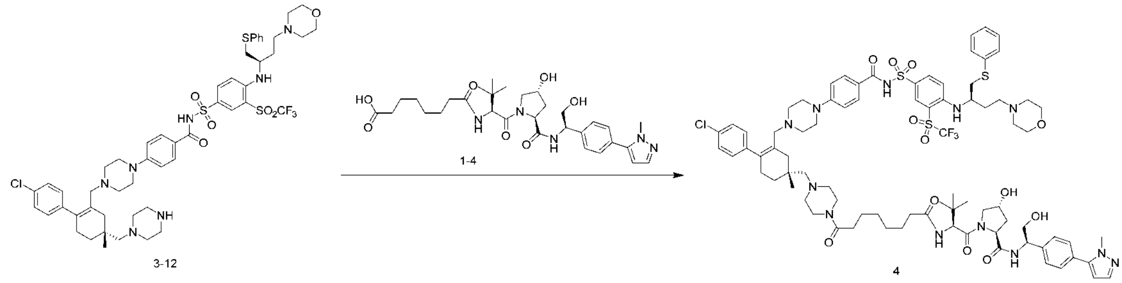


Intermediate 3-12 (106 mg) was dissolved in dichloromethane, and intermediate 1-4 (70 mg), triethylamine (140 μL), and HATU (57 mg) were added. The mixture was reacted at room temperature for 1.5 h. The mixture was then diluted with DCM and extracted with saturated ammonium chloride, followed by liquid separation. The organic phase was washed with water, followed by liquid separation. The aqueous phase was back-extracted with DCM. The organic phases were combined and washed with saturated sodium chloride, followed by liquid separation. The resulting organic phase was dried over anhydrous sodium sulfate, filtered under vacuum, and concentrated, and the crude product was subjected to preparative liquid chromatography to give 64 mg of the target compound 4. LCMS (ESI) m/z: 812.5 $[M+2H]^{2+}$.

### Example 5: Preparation of Compound 5

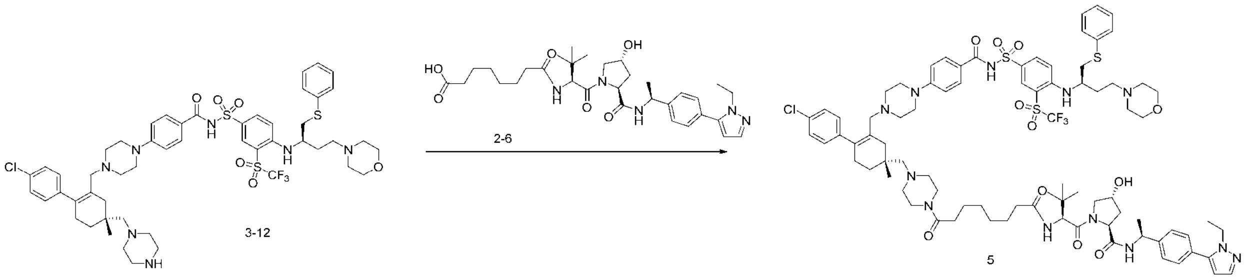


Intermediate 3-12 (100 mg) was dissolved in dichloromethane, and intermediate 2-6 (68 mg), triethylamine (125 μL), and HATU (53 mg) were added. The mixture was reacted at room temperature for 1.5 h. The mixture was then diluted with DCM and extracted with saturated ammonium chloride, followed by liquid separation. The organic phase was washed with water, followed by liquid separation. The aqueous phase was back-extracted with DCM. The organic phases were combined and washed with saturated sodium chloride, followed by liquid separation. The resulting organic phase was dried over anhydrous sodium sulfate, filtered under vacuum, and concentrated, and the crude product was subjected to preparative liquid chromatography to give 21 mg of the target compound 5.

ESI-MS: m/z = 820.2$[M+2H]^{2+}$.

**Example 6: Preparation of Compound 6**

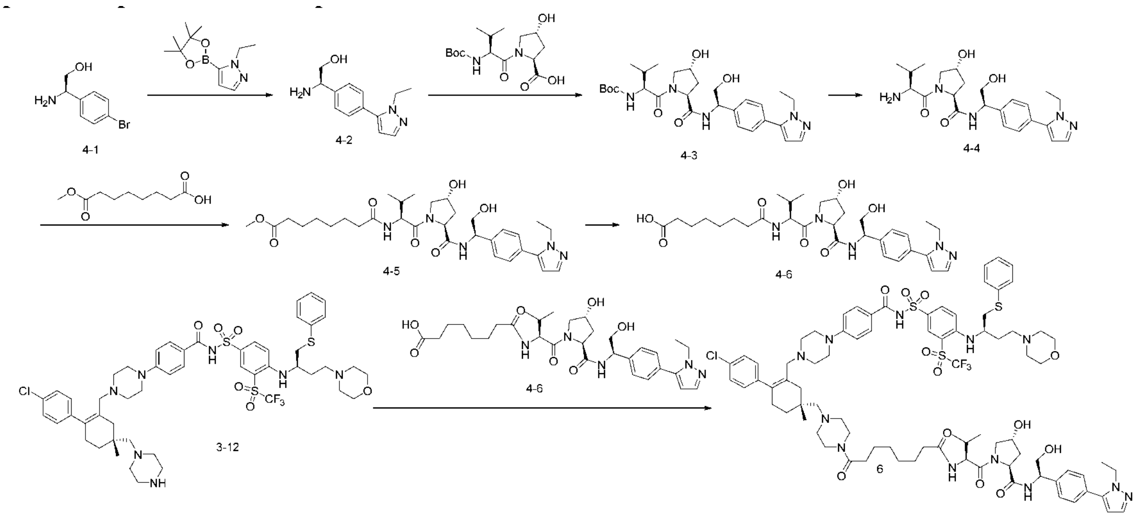

**Preparation of intermediate 4-2**

Intermediate 4-1 (1.4 g) and 1-ethyl-5-(4,4,5,5-tetramethyl-1,3,2-dioxaborolan-2-yl)-1*H*-pyrazole (2.16 g) were dissolved in a system of 1,4-dioxane/water (4/1, 50 mL), and potassium carbonate (2.76 g) and [1,1'-bis(diphenylphosphino)ferrocene]palladium(II) dichloride (730 mg) were added. The mixture was purged with nitrogen, heated to 90 °C, and stirred for three hours. After the three hours, the heating was stopped, and the reaction solution was directly concentrated to dryness. The residue was subjected to column chromatography (dichloromethane/methanol) to give the product intermediate 4-2 (1.52 g).
LC-MS: m/z $[M+H]^+$: 232.1.

**Preparation of intermediate 4-3**

Intermediate 4-2 (2.031 g) and 1-(Boc-*L*-pentyl)-(4R)-4-hydroxy-*L*-proline (1.421 g) were dissolved in *N,N*-dimethylformamide (40 mL), and *N,N*-diisopropylethylamine (2.142 mL) was added. The mixture was stirred at room temperature for 20 min. After the twenty minutes, *O*-(7-azabenzotriazol-1-yl)-*N,N,N',N'*-tetramethyluronium hexafluorophosphate (4.675 g) was added to the reaction system. The mixture was stirred for another 16 h. The reaction solution was then diluted with 60 mL of ethyl acetate and washed with 60 mL of saturated brine. After the washing, the organic phase was concentrated, and the residue was subjected to column chromatography (dichloromethane/methanol) to give the product intermediate 4-3 (2 g).
LC-MS: m/z $[M+H]^+$: 544.3.

**Preparation of intermediate 4-4**

Intermediate 4-3 (2 g) was dissolved in a system of trifluoroacetic acid/dichloromethane (1/3, 20 mL), and the system was stirred at room temperature for 30 min, then adjusted to pH 7 with a 7 M solution of ammonia in methanol, and concentrated to dryness. The residue was subjected to column chromatography (dichloromethane/methanol) to give the product intermediate 4-4 (2 g).
LC-MS: m/z $[M+H]^+$: 444.47.

**Preparation of intermediate 4-5**

Intermediate 4-4 (100 mg) and monomethyl suberate (44.5 μL) were dissolved in dichloromethane (5 mL), and triethylamine (62.5 μL) was added. The mixture was stirred at room temperature for half an hour, and HATU (94 mg) was added. The resulting mixture was stirred at room temperature for 16 h. Then, the reaction solution was directly concentrated to dryness. The residue was subjected to column chromatography (dichloromethane/methanol) to give the product intermediate 4-5 (85 mg).

LC-MS: m/z $[M+H]^+$: 614.3.

**Preparation of intermediate 4-6**

Intermediate 4-5 (85 mg) was dissolved in methanol (1.5 mL), and a 2 N lithium hydroxide solution (208 μL) was added. The mixture was stirred at room temperature for 16 h. The pH was then adjusted to neutral with 2 N hydrochloric acid, and the mixture was extracted with ethyl acetate. The organic phase was directly concentrated to dryness to give the product intermediate 4-6 (80 mg).
LC-MS: m/z $[M+H]^+$: 600.3.

**Preparation of compound 6**

Intermediate 4-6 (28 mg) and intermediate 3-12 (50 mg) were dissolved in dichloromethane (1 mL), and triethylamine (66 μL) was added. The mixture was stirred for half an hour. HATU (27 mg) was added, and the mixture was stirred at room temperature overnight. The system was concentrated, and the residue was subjected to preparative chromatography to give the target compound 6 (51 mg, purity: 98.85%).
ESI-MS: m/z = 821.16 $[M+2H]^{2+}$.

**Example 7: Preparation of Compound 7**

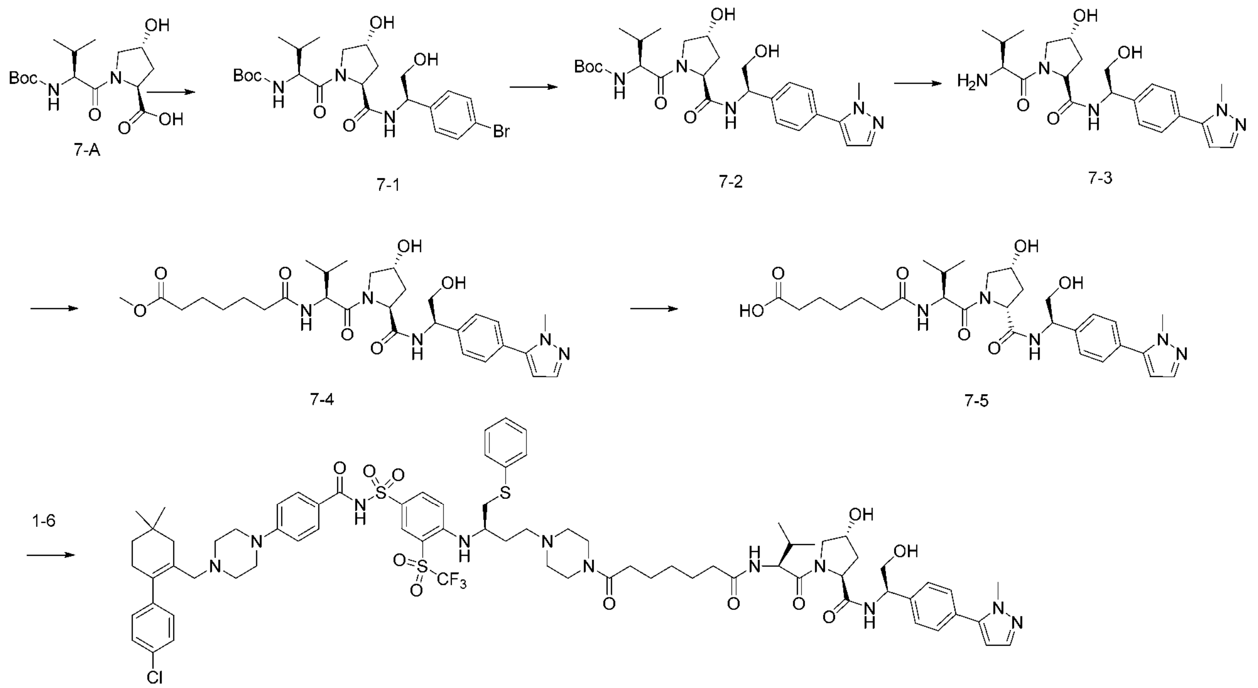


7-A 7-1 7-2 7-3 7-4 7-5 7

1) Preparation of intermediate 7-1

**7-A** (2 g) and (R)-2-amino-2-(4-bromophenyl)ethanol (1.43 g, 6.663 mmol) were dissolved in dichloromethane, and EDCI (2.32 g) and HOBT (1.64 g) were added. The mixture was reacted at room temperature overnight. Water was added, followed by liquid separation. The organic phase was purified by silica gel column chromatography (DCM:MeOH) to give 2.8 g of intermediate **7-1.** ESI-MS: m/z = 550.2 $[M+Na]^+$.

2) Preparation of intermediate 7-2

Intermediate **7-1** (500 mg) and 1-methyl-1*H*-pyrazole-5-boronic acid pinacol ester (197 mg) were dissolved in 10 mL of dioxane and 2 mL of water, and potassium carbonate (262 mg) and $Pd(dppf)Cl_2$ (35 mg) were added. The mixture was purged with nitrogen, then reacted at 90 °C for 3 h, cooled, dried, and concentrated. The residue was subjected to

column chromatography (DCM:MeOH) to give 512 mg of intermediate **7-2.** ESI-MS: m/z = 552.3 $[M+Na]^+$.

3) Preparation of intermediate 7-3

Intermediate **7-2** (512 mg) was dissolved in hydrochloric acid/dioxane (4 M), and the mixture was stirred at room temperature for 3 h and then concentrated at 38 °C to give intermediate **7-3.** ESI-MS: m/z = 430.4 $[M+H]^+$.

4) Preparation of intermediate 7-4

Intermediate **7-3** (100 mg) was dissolved in dichloromethane, and monomethyl pimelate (49 mg), DIPEA (104 mg), and EDCI (67 mg) were added. The mixture was reacted at room temperature overnight. After the reaction was completed, the mixture was concentrated, and the residue was separated by silica gel column chromatography (DCM:MeOH) to give 135 mg of intermediate **7-4.** ESI-MS: m/z = 586.3 $[M+H]^+$.

5) Preparation of intermediate 7-5

Intermediate **7-4** (135 mg) was dissolved in methanol, and an aqueous lithium hydroxide solution (2 N, 0.5 mL) was added. The mixture was stirred at room temperature overnight. The reaction solution was adjusted to pH 5-6 with 2 N hydrochloric acid, extracted with DCM, subjected to liquid separation, dried, and concentrated to give 64 mg of intermediate **7-5.** ESI-MS: m/z = 572.3 $[M+H]^+$.

6) Preparation of compound 7

Intermediate **1-6** (50 mg), intermediate **7-5** (29 mg), triethylamine (26 mg), and HATU (29 mg) were dissolved in dichloromethane (3 mL), and the mixture was stirred at room temperature for 16 h and then concentrated under reduced pressure. The residue was purified to give compound 7 (3 mg).

ESI-MS: m/z = 763.7 $[M+2H]^{2+}$.

$^1H$ NMR (500 MHz, $CDCl_3$) δ 8.36 (d, 1H), 8.08 (dd, 1H), 7.71 (d, 2H), 7.49 (d, 1H), 7.39 - 7.25 (m, 10H), 7.11 (t, 1H), 6.97 (d, 2H), 6.75 (d, 2H), 6.62 (d, 1H), 6.36 (d, 1H), 6.27 (d, 1H), 5.07 (dt, 1H), 4.72 (t, 1H), 4.61 (d, 1H), 4.49 (s, 1H), 4.08 (d, 1H), 3.89 (s, 4H), 3.65 - 3.36 (m, 13H), 3.08 (qd, 3H), 2.89 (s, 1H), 2.50 - 2.01 (m, 19H), 1.58 (s, 5H), 1.45 (t, 6H), 1.38 - 1.23 (m, 4H), 0.98 (s, 12H).

**Example 8: Preparation of Compound 8**

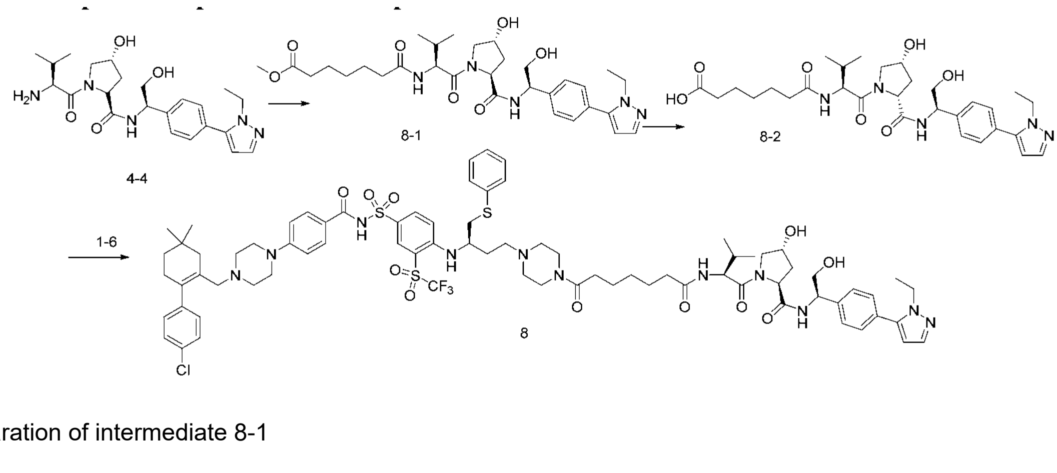


1) Preparation of intermediate 8-1

Intermediate **8-1** (138 mg) was obtained by following step 4 of Example 7, while replacing intermediate **7-3** with intermediate **4-4.** ESI-MS: m/z = 600.3 $[M+H]^+$.

2) Preparation of intermediate 8-2

Intermediate **8-2** (70 mg) was obtained by following step 5 of Example 7, while replacing intermediate **7-4** with intermediate **8-1.** ESI-MS: m/z = 586.3 $[M+H]^+$.

3) Preparation of compound 8

Compound **8** (2 mg) was obtained by following step 6 of Example 7, while replacing intermediate **7-5** with intermediate **8-2.** ESI-MS: m/z = 770.7 $[M+2H]^{2+}$.

$^1H$ NMR (500 MHz, $CDCl_3$) $\delta$ 8.35 (d, 1H), 8.08 (dd, 1H), 7.71 (d, 2H), 7.50 (d, 1H), 7.39 - 7.25 (m, 10H), 7.11 (t, 1H), 6.97 (d, 2H), 6.73 (d, 2H), 6.60 (d, 1H), 6.36 (d, 1H), 6.27 (d, 1H), 5.07 (dt, 1H), 4.71 (t, 1H), 4.61 (d, 1H), 4.48 (s, 1H), 4.08 (d, 1H), 3.89 (s, 4H), 3.65 - 3.33 (m, 13H), 3.07 (qd, 3H), 2.88 (s, 1H), 2.50 - 2.01 (m, 19H), 1.58 (s, 5H), 1.45 (t, 6H), 1.38 - 1.23 (m, 6H), 0.98 (s, 12H).

**Example 9: Preparation of Compound 9**

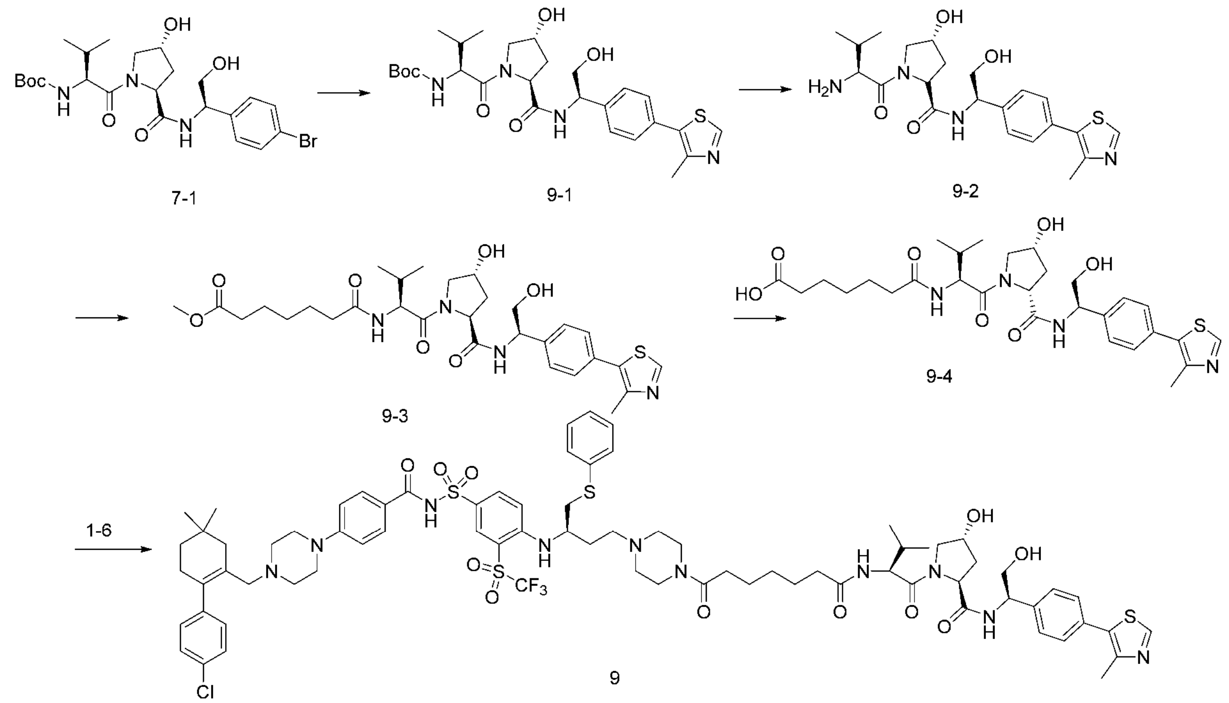


7-1 9-1 9-2 9-3 9-4 9

1) Preparation of intermediate 9-1

Intermediate **7-1** (500 mg) and 4-methylthiazole-5-boronic acid pinacol ester (214 mg) were dissolved in 10 mL of dioxane and 2 mL of water, and potassium carbonate (262 mg) and $Pd(dppf)Cl_2$ (35 mg) were added. The mixture was purged with nitrogen and then reacted at 90 °C for 3 h, cooled, dried, and concentrated. The residue was purified by silica gel column chromatography (DCM:MeOH) to give 500 mg of intermediate **9-1.** ESI-MS: m/z = 569.2 $[M+Na]^+$.

2) Preparation of intermediate 9-2

Intermediate **9-1** (500 mg) was dissolved in hydrochloric acid/dioxane (4 M), and the mixture was stirred at room temperature for 3 h and then concentrated at 38 °C to give intermediate **9-2.** ESI-MS: m/z = 447.3 $[M+H]^+$.

3) Preparation of intermediate 9-3

Intermediate **9-3** (139 mg) was obtained by following step 4 of Example 7, while replacing intermediate **7-3** with intermediate **9-2.** ESI-MS: m/z = 603.3 $[M+H]^+$.

4) Preparation of intermediate 9-4

Intermediate **9-4** (50 mg) was obtained by following step 5 of Example 7, while replacing intermediate **7-4** with intermediate **9-3.** ESI-MS: m/z = 589.3 $[M+H]^+$.

5) Preparation of compound 9

Compound **9** (3 mg) was obtained by following step 6 of Example 7, while replacing intermediate **7-5** with intermediate **9-4.**

ESI-MS: m/z = 772.3 $[M+2H]^{2+}$.

$^1$H NMR (500 MHz, $CDCl_3$) δ 8.35 (d, 1H), 8.07 (dd, 1H), 7.70 (d, 2H), 7.50 (d, 1H), 7.39 - 7.25 (m, 10H), 7.11 (t, 1H), 6.98 (d, 2H), 6.73 (d, 2H), 6.61 (d, 1H), 6.36 (d, 1H), 6.27 (d, 1H), 5.07 (dt, 1H), 4.71 (t, 1H), 4.61 (d, 1H), 4.48 (s, 1H), 4.08 (d, 1H), 3.89 (s, 4H), 3.65 - 3.35 (m, 13H), 3.07 (qd, 3H), 2.89 (s, 1H), 2.51 - 2.03 (m, 18H), 1.58 (s, 5H), 1.45 (t, 6H), 1.38 - 1.24 (m, 4H), 0.98 (s, 12H).

**Example 10: Preparation of Compound 10**

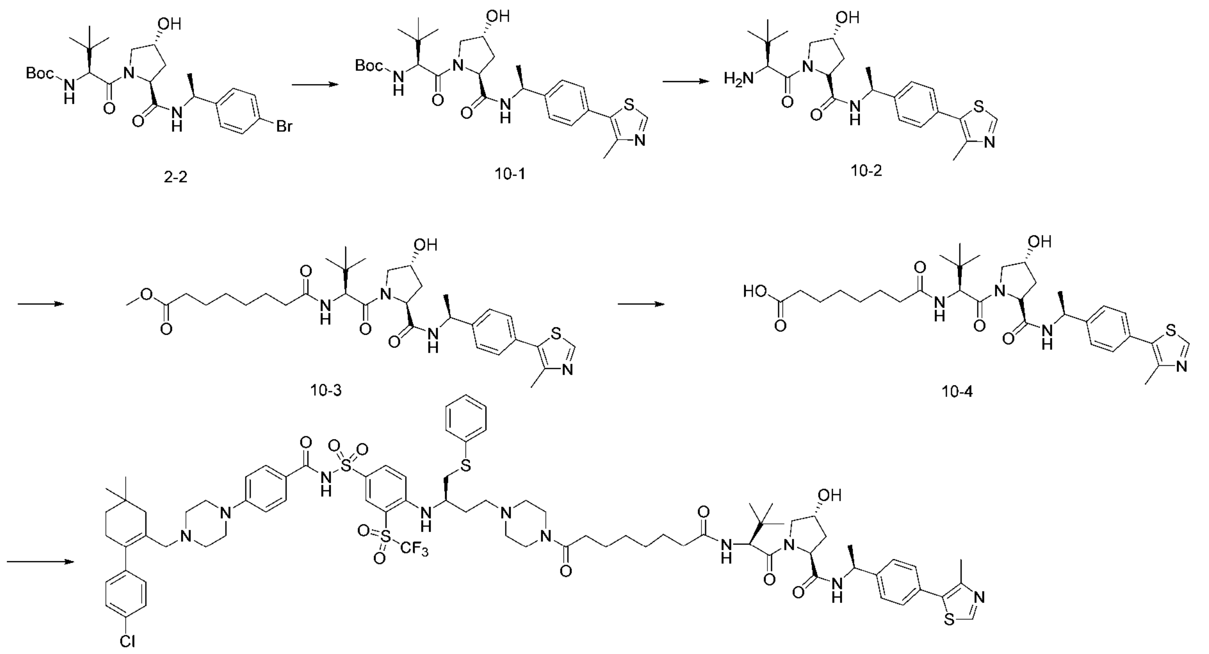

2-2 10-1 10-2 10-3 10-4 10

1) Preparation of intermediate 10-1

Intermediate **10-1** (1 g) was obtained by following step 2 of Example 7, while replacing 1-methyl-1*H*-pyrazole-5-boronic acid pinacol ester with 4-methylthiazole-5-boronic acid pinacol ester. ESI-MS: m/z = 545.3 $[M+H]^+$.

2) Preparation of intermediate 10-2

Intermediate **10-2** (0.8 g) was obtained by following step 3 of Example 7, while replacing intermediate **7-2** with intermediate **10-1.** ESI-MS: m/z = 445.2 $[M+H]^+$.

3) Preparation of intermediate 10-3

Intermediate **10-3** (141 mg) was obtained by following step 4 of Example 7, while replacing intermediate **7-3** with intermediate **10-2** and replacing monomethyl pimelate with monomethyl suberate. ESI-MS: m/z = 615.3 $[M+H]^+$.

4) Preparation of intermediate 10-4

Intermediate **10-4** (80 mg) was obtained by following step 5 of Example 7, while replacing intermediate **7-4** with intermediate **10-3.** ESI-MS: m/z = 601.3 $[M+H]^+$.

5) Preparation of compound 4

Compound **10** (20 mg) was obtained by following step 6 of Example 7, while replacing intermediate **7-5** with intermediate **10-4.**

ESI-MS: m/z = 778.3 $[M+2H]^{2+}$.

$^1H$ NMR (500 MHz, $CDCl_3$) δ 8.67 (s, 1H), 8.34 (d, 1H), 8.09 (dd, 1H), 7.71 (d, 2H), 7.43 - 7.23 (m, 12H), 7.07 (t, 1H), 6.98 (d, 2H), 6.75 (d, 2H), 6.61 (d, 1H), 6.38 (d, 1H), 5.07 (dt, 1H), 4.72 (t, 1H), 4.61 (d, 1H), 4.49 (s, 1H), 4.08 (d, 1H), 3.89 (s, 3H), 3.65 - 3.36 (m, 11H), 3.08 (qd, 3H), 2.89 (s, 1H), 2.50 - 2.01 (m, 19H), 1.58 (s, 5H), 1.46 (t, 6H), 1.38 - 1.23 (m, 6H), 1.04 (s, 6H), 0.97 (s, 9H).

**Example 11: Preparation of Compound 11**

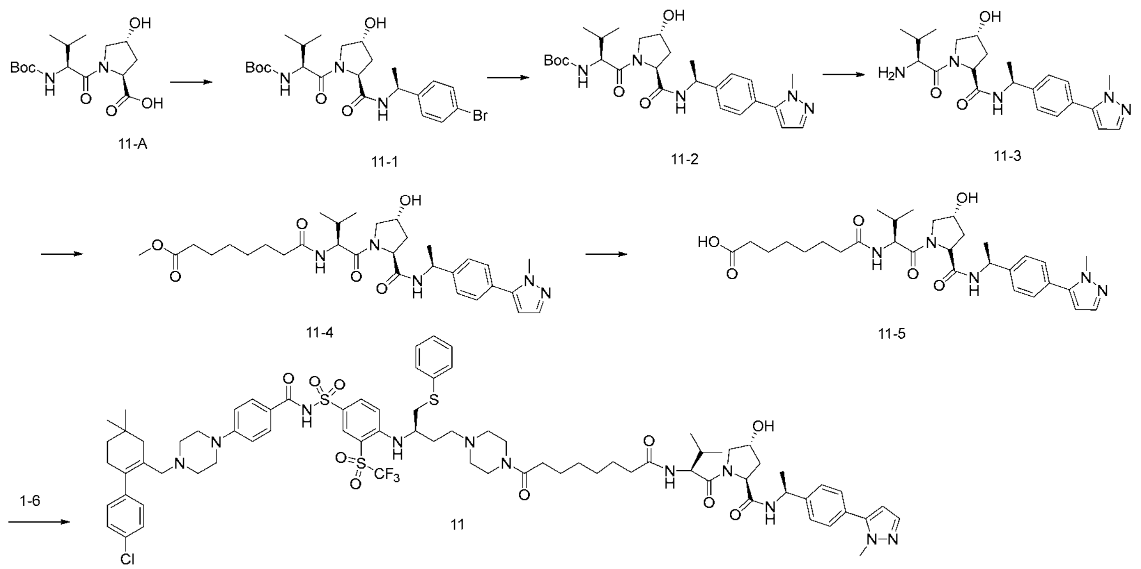


11-A

11-1

11-2

11-3

11-4

11-5

11

1) Preparation of intermediate 11-1

11-A (600 mg) and (*S*)-(-)-1-(4-bromophenyl)ethylamine (400 mg) were dissolved in dichloromethane, and EDCI (697 mg) and HOBT (491 mg) were added. The mixture was reacted at room temperature overnight. Water was added, and the organic phase was separated and purified by column chromatography (DCM:MeOH) to give 750 mg of intermediate **11-1.** ESI-MS: m/z = 534.2 $[M+Na]^+$.

2) Preparation of intermediate 11-2

Intermediate **11-2** (0.5 g) was obtained by following step 2 of Example 7, while replacing intermediate **7-1** with intermediate **11-1.** ESI-MS: m/z = 514.3 $[M+H]^+$.

3) Preparation of intermediate 11-3

Intermediate **11-3** (0.4 g) was obtained by following step 3 of Example 7, while replacing intermediate **7-2** with intermediate **11-2.** ESI-MS: m/z = 414.2 $[M+H]^+$.

4) Preparation of intermediate 11-4

Intermediate **11-4** (120 mg) was obtained by following step 4 of Example 7, while replacing intermediate **7-3** with intermediate **11-3** and replacing monomethyl pimelate with monomethyl suberate. ESI-MS: m/z = 584.3 $[M+H]^+$.

5) Preparation of intermediate 11-5

Intermediate **11-5** (80 mg) was obtained by following step 5 of Example 7, while replacing intermediate **7-4** with

intermediate **11-4.** ESI-MS: m/z = 570.3 [M+H]+.

6) Preparation of compound 11

Compound **11** (5 mg) was obtained by following step 6 of Example 7, while replacing intermediate **7-5** with intermediate **11-5.**

ESI-MS: m/z = 762.7 [M+2H]2+.

$^1$H NMR (500 MHz, $CDCl_3$) δ 8.35 (d, 1H), 8.07 (dd, 1H), 7.70 (d, 2H), 7.49 (d, 1H), 7.39 - 7.25 (m, 10H), 7.10 (t, 1H), 6.98 (d, 2H), 6.75 (d, 2H), 6.62 (d, 1H), 6.36 (d, 1H), 6.27 (d, 1H), 5.07 (dt, 1H), 4.72 (t, 1H), 4.61 (d, 1H), 4.49 (s, 1H), 4.08 (d, 1H), 3.89 (s, 4H), 3.65 - 3.36 (m, 12H), 3.08 (qd, 3H), 2.89 (s, 3H), 2.50 - 2.01 (m, 18H), 1.58 (s, 5H), 1.45 (t, 6H), 1.38 - 1.23 (m, 6H), 0.98 (s, 12H).

**Example 12: Preparation of Compound 12**

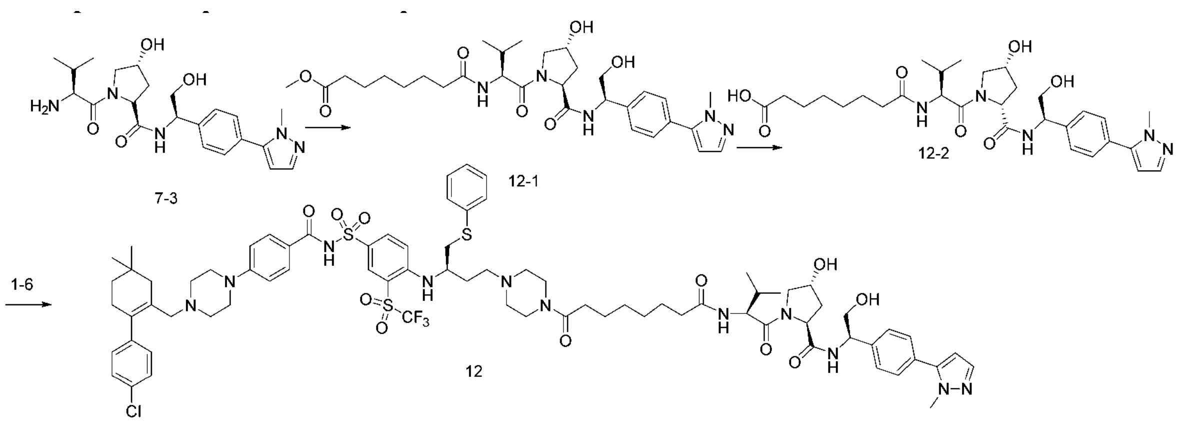


7-3

12-1

12-2

12

1) Preparation of intermediate 12-1

Intermediate **12-1** (114 mg) was obtained by following step 4 of Example 7, while replacing monomethyl pimelate with monomethyl suberate. ESI-MS: m/z = 600.3 [M+H]+.

2) Preparation of intermediate 12-2

Intermediate **12-2** (70 mg) was obtained by following step 5 of Example 7, while replacing intermediate **7-4** with intermediate **12-1.** ESI-MS: m/z = 586.3 [M+H]+.

3) Preparation of compound 12

Compound **12** (6 mg) was obtained by following step 6 of Example 7, while replacing intermediate **7-5** with intermediate **12-2.** ESI-MS: m/z = 770.7 [M+2H]2+.

$^1$H NMR (500 MHz, $CDCl_3$) δ 8.36 (d, 1H), 8.09 (dd, 1H), 7.71 (d, 2H), 7.49 (d, 1H), 7.39 - 7.25 (m, 10H), 7.11 (t, 1H), 6.98 (d, 2H), 6.77 (d, 2H), 6.62 (d, 1H), 6.36 (d, 1H), 6.27 (d, 1H), 5.07 (dt, 1H), 4.71 (t, 1H), 4.63 (d, 1H), 4.49 (s, 1H), 4.08 (d, 1H), 3.89 (s, 4H), 3.66 - 3.36 (m, 13H), 3.07 (qd, 3H), 2.89 (s, 1H), 2.50 - 2.01 (m, 19H), 1.58 (s, 5H), 1.46 (t, 6H), 1.38 - 1.23 (m, 6H), 0.98 (s, 12H).

**Example 13: Preparation of Compound 13**

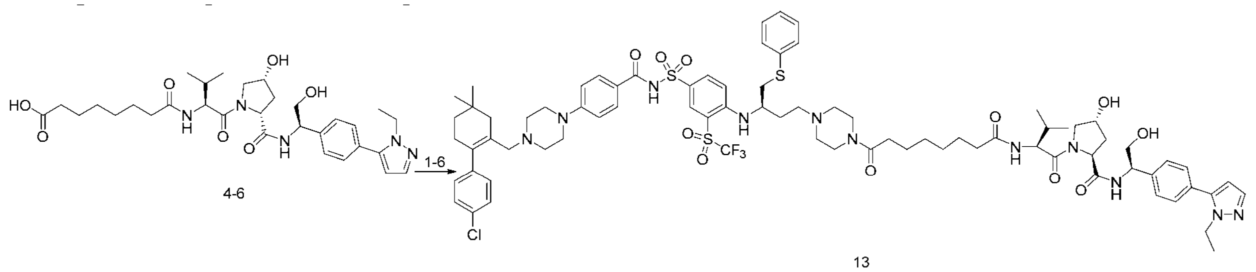


4-6

13

1) Preparation of compound 13

Compound **13** (27 mg) was obtained by following step 5 of Example 7, while replacing intermediate **7-5** with intermediate **4-6.**

ESI-MS: m/z = 777.7 $[M+2H]^{2+}$.

$^1H$ NMR (500 MHz, $CDCl_3$) $\delta$ 8.35 (d, 1H), 8.09 (dd, 1H), 7.71 (d, 2H), 7.54 (d, 1H), 7.43 - 7.24 (m, 10H), 7.07 (t, 1H), 6.98 (d, 2H), 6.75 (d, 2H), 6.64 (d, 1H), 6.38 (d, 1H), 6.21 (d, 1H), 5.29 (dt, 1H), 4.65 (t, 1H), 4.53 (d, 1H), 4.45 (s, 1H), 4.08 (d, 1H), 3.90 (s, 3H), 3.65 - 3.36 (m, 14H), 3.089 (qd, 3H), 2.88 (s, 1H), 2.40 - 2.01 (m, 21H), 1.58 (s, 5H), 1.46 (t, 6H), 1.39 - 1.25 (m, 6H), 0.98 (s, 6H), 0.94 (s, 6H).

**Example 14: Preparation of Compound 14**

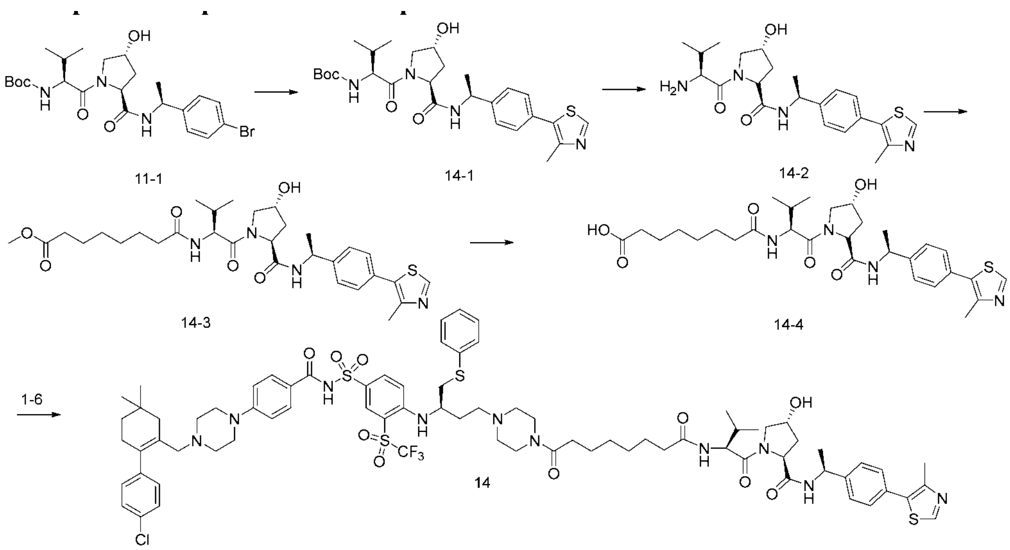


11-1

14-1

14-2

14-3

14-4

14

1) Preparation of intermediate 14-1

Intermediate **14-1** (200 mg) was obtained by following step 2 of Example 7, while replacing 1-methyl-1*H*-pyrazole-5-boronic acid pinacol ester with 4-methylthiazole-5-boronic acid pinacol ester. ESI-MS: m/z = 553.3 $[M+Na]^+$.

2) Preparation of intermediate 14-2

Intermediate **14-2** (162 mg) was obtained by following step 3 of Example 7, while replacing intermediate **7-2** with intermediate **14-1.** ESI-MS: m/z = 431.3 $[M+H]^+$.

3) Preparation of intermediate 14-3

Intermediate **14-3** (141 mg) was obtained by following step 4 of Example 7, while replacing intermediate **7-3** with intermediate **14-2** and replacing monomethyl pimelate with monomethyl suberate. ESI-MS: m/z = 601.3 $[M+H]^+$.

4) Preparation of intermediate 14-4

Intermediate **14-4** (75 mg) was obtained by following step 5 of Example 7, while replacing intermediate **7-4** with intermediate **14-3.** ESI-MS: m/z = 587.3 $[M+H]^+$.

5) Preparation of compound 14

Compound **14** (4 mg) was obtained by following step 6 of Example 7, while replacing intermediate **7-5** with intermediate **14-4.**

ESI-MS: m/z = 771.3 $[M+2H]^{2+}$.

$^1H$ NMR (500 MHz, $CDCl_3$) δ 8.36 (d, 1H), 8.08 (dd, 1H), 7.71 (d, 2H), 7.50 (d, 1H), 7.41 - 7.24 (m, 10H), 7.10 (t, 1H), 6.97 (d, 2H), 6.73 (d, 2H), 6.60 (d, 1H), 6.34 (d, 1H), 6.28 (d, 1H), 5.03 (dt, 1H), 4.72 (t, 1H), 4.61 (d, 1H), 4.48 (s, 1H), 4.09 (d, 1H), 3.90 (s, 4H), 3.65 - 3.34 (m, 12H), 3.07 (qd, 3H), 2.88 (s, 1H), 2.51 - 2.01 (m, 19H), 1.58 (s, 5H), 1.45 (t, 6H), 1.38 - 1.23 (m, 6H), 0.98 (s, 12H).

**Example 15: Preparation of Compound 15**

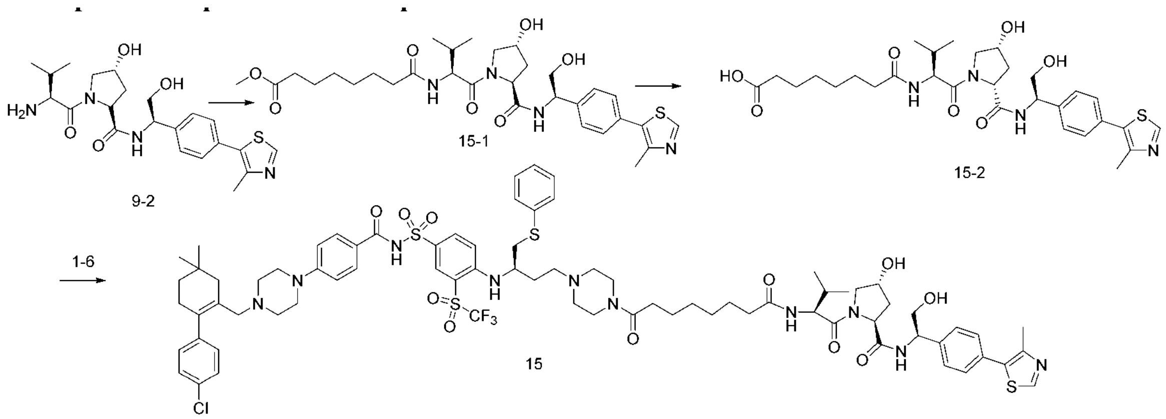


1) Preparation of intermediate 15-1

Intermediate **15-1** (73 mg) was obtained by following step 4 of Example 7, while replacing intermediate **7-3** with intermediate **9-2** and replacing monomethyl pimelate with monomethyl suberate. ESI-MS: m/z = 617.15 $[M+H]^+$.

2) Preparation of intermediate 15-2

Intermediate **15-2** (60 mg) was obtained by following step 5 of Example 7, while replacing intermediate **7-4** with intermediate **15-1.** ESI-MS: m/z = 603.17 $[M+H]^+$.

3) Preparation of compound 15

Compound 15 (6 mg) was obtained by following step 6 of Example 7, while replacing intermediate **7-5** with intermediate **15-2.** ESI-MS: m/z = 779.3 $[M+2H]^{2+}$.

$^1H$ NMR (500 MHz, DMSO-$d_6$+$D_2O$) δ 8.95 (s, 1H), 8.26 (s, 5H), 8.07 (s, 1H), 7.90 (s, 1H), 7.70 (m, 2H), 7.35 (m, 10H), 7.18 (m, 1H), 7.10 (m, 2H), 6.90 (s, 1H), 6.75 (m, 2H), 4.88 (m, 1H), 4.56 - 4.43 (m, 3H), 4.30 (s, 1H), 3.69 - 3.53 (m, 9H), 3.33 (m, 4H), 3.15 (s, 4H), 2.74 (s, 3H), 2.44 (s, 3H), 2.30 (s, 7H), 2.24 (s, 5H), 1.84 (s, 1H), 1.70 (s, 1H), 1.55 - 1.35 (m, 7H), 1.25 (s, 9H), 0.94 (s, 12H).

**Example 16: Preparation of Compound 16**

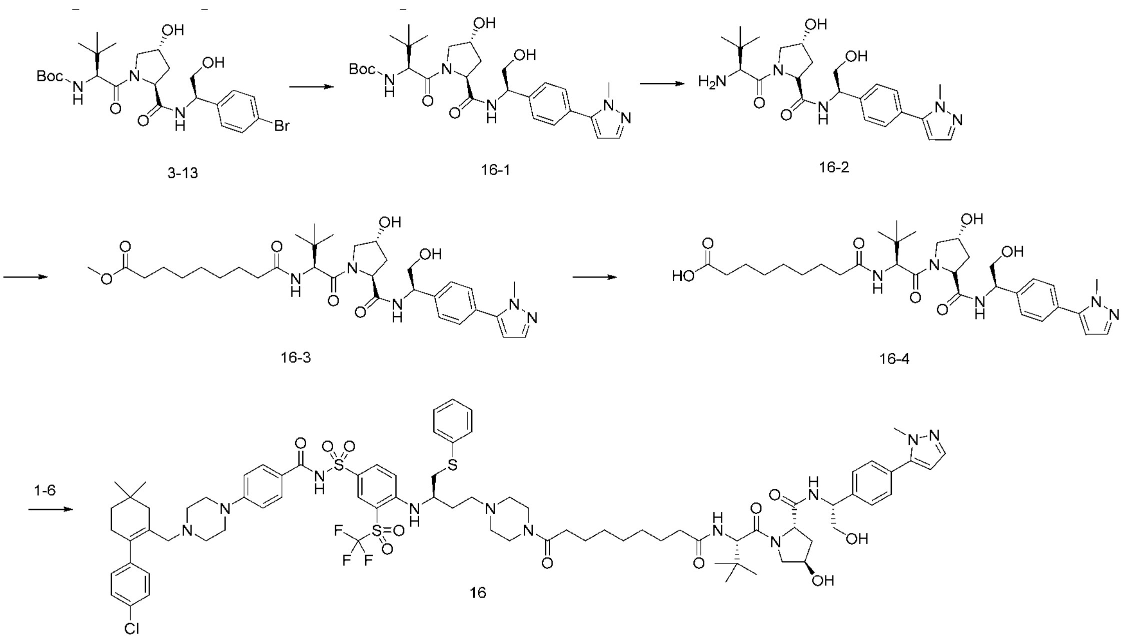


3-13 16-1 16-2

16-3 16-4

16

1) Preparation of intermediate 16-1

Intermediate **16-1** (1 g) was obtained by following step 2 of Example 7, while replacing intermediate **7-1** with intermediate **3-13.** ESI-MS: m/z = 544.3 $[M+H]^+$.

2) Preparation of intermediate 16-2

Intermediate **16-2** (200 mg) was obtained by following step 3 of Example 7, while replacing intermediate **7-2** with intermediate **16-1.** ESI-MS: m/z = 444.2 $[M+H]^+$.

3) Preparation of intermediate 16-3

Intermediate **16-3** (283 mg) was obtained by following step 4 of Example 7, while replacing intermediate **7-3** with intermediate **16-2** and replacing monomethyl pimelate with monomethyl azelate. ESI-MS: m/z = 628.3 $[M+H]^+$.

4) Preparation of intermediate 16-4

Intermediate **16-4** (270 mg) was obtained by following step 5 of Example 7, while replacing intermediate **7-4** with intermediate **16-3.**

ESI-MS: m/z = 614.3 $[M+H]^+$.

5) Preparation of compound 16

Compound **16** (30 mg) was obtained by following step 6 of Example 7, while replacing intermediate **7-5** with intermediate **16-4.**

ESI-MS: m/z = 784.7 $[M+2H]^{2+}$.

$^1$H NMR (500 MHz, DMSO) δ 8.37 (s, 1H), 8.13 (s, 2H), 7.96 (s, 1H), 7.76 (m, 3H), 7.51 - 6.82 (m, 16H), 6.37 (s, 1H), 5.11 (s, 1H), 4.90 (s, 1H), 4.75 (s, 1H), 4.55 - 4.46 (m, 2H), 4.29 (s, 2H), 4.09 (s, 2H), 3.84 (s, 3H), 3.63 (s, 5H), 3.27 - 3.10 (s, 7H), 2.88 (s, 2H), 2.61 - 1.73 (m, 25H), 1.34 (m, 12H), 0.95 (s, 14H).

**Example 17: Preparation of Compound 17**

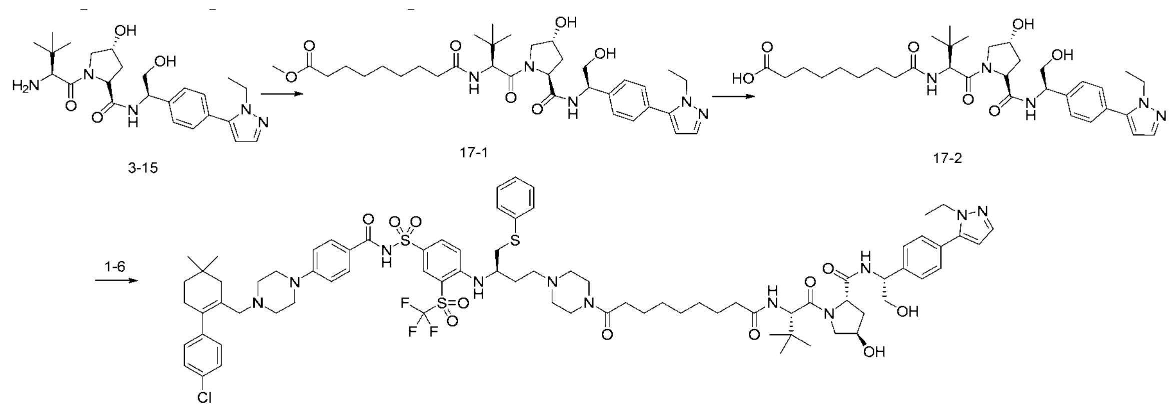


3-15 17-1 17-2

17

1) Preparation of intermediate 17-1

Intermediate **17-1** (280 mg) was obtained by following step 4 of Example 7, while replacing intermediate **7-3** with intermediate **3-15** and replacing monomethyl pimelate with monomethyl azelate. ESI-MS: m/z = 642.3 [M+H]$^+$.

2) Preparation of intermediate 17-2

Intermediate **17-2** (270 mg) was obtained by following step 5 of Example 7, while replacing intermediate **7-4** with intermediate **17-1.** ESI-MS: m/z = 628.3 [M+H]$^+$.

3) Preparation of compound 17

Compound **17** (13 mg) was obtained by following step 6 of Example 7, while replacing intermediate **7-5** with intermediate **17-2.**

ESI-MS: m/z = 792.46 [M+2H]$^{2+}$.

$^1$H NMR (500 MHz, DMSO) δ 8.40 (s, 1H), 8.14 (s, 2H), 7.93 (s, 1H), 7.75 (m, 3H), 7.50 - 6.80 (m, 16H), 6.35 (s, 1H), 5.12 (s, 1H), 4.94 (s, 1H), 4.73 (s, 1H), 4.55 - 4.45 (m, 2H), 4.29 (s, 2H), 4.08 (s, 2H), 3.85 (s, 3H), 3.63 (s, 5H), 3.26 - 3.10 (s, 7H), 2.89 (s, 2H), 2.62 - 1.75 (m, 25H), 1.35 (m, 14H), 0.94 (s, 14H)

**Example 18: Preparation of Compound 18**

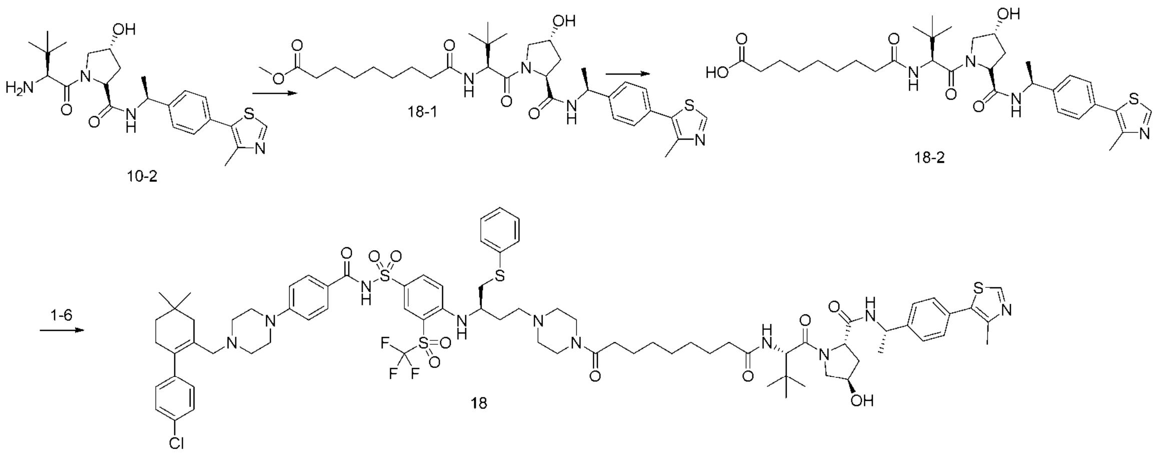


10-2 18-1 18-2

18

1) Preparation of intermediate 18-1

Intermediate **18-1** (141 mg) was obtained by following step 4 of Example 7, while replacing intermediate **7-3** with intermediate **10-2** and replacing monomethyl pimelate with monomethyl azelate. ESI-MS: m/z = 629.3 [M+H]$^+$.

2) Preparation of intermediate 18-2

Intermediate **18-2** (80 mg) was obtained by following step 5 of Example 7, while replacing intermediate **7-4** with intermediate **18-1.** ESI-MS: m/z = 615.3 $[M+H]^+$.

3) Preparation of compound 18

Compound **18** (5 mg) was obtained by following step 6 of Example 7, while replacing intermediate **7-5** with intermediate **18-2.**

ESI-MS: m/z = 785.6 $[M+2H]^{2+}$.

$^1$H NMR (500 MHz, $CDCl_3$) δ 8.66 (s, 1H), 8.34 (d, 1H), 8.09 (dd, 1H), 7.71 (d, 2H), 7.46 - 7.24 (m, 12H), 7.09 (t, 1H), 6.98 (d, 2H), 6.77 (d, 2H), 6.61 (d, 1H), 6.38 (d, 1H), 5.07 (dt, 1H), 4.72 (t, 1H), 4.60 (d, 1H), 4.49 (s, 1H), 4.07 (d, 1H), 3.89 (s, 3H), 3.65 - 3.36 (m, 11H), 3.08 (qd, 3H), 2.89 (s, 1H), 2.50 - 2.01 (m, 19H), 1.58 (s, 5H), 1.46 (t, 6H), 1.38 - 1.23 (m, 8H), 1.04 (s, 6H), 0.97 (s, 9H).

**Example 19: Preparation of Compound 19**

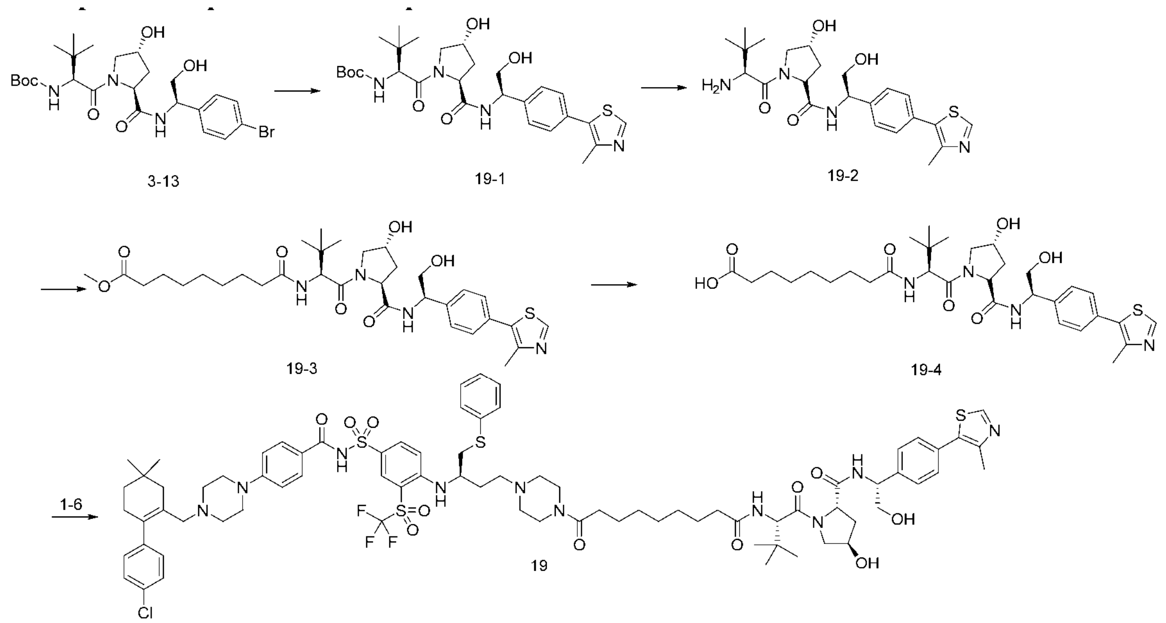


3-13 19-1 19-2 19-3 19-4 19

1) Preparation of intermediate 19-1

Intermediate **19-1** (1.2 g) was obtained by following step 2 of Example 7, while replacing intermediate **7-1** with intermediate **3-13** and replacing 1-methyl-1*H*-pyrazole-5-boronic acid pinacol ester with (4-methylthiazol-5-yl)boronic acid. ESI-MS: m/z = 561.2 $[M+H]^+$.

2) Preparation of intermediate 19-2

Intermediate **19-2** (0.9 g) was obtained by following step 3 of Example 7, while replacing intermediate **7-2** with intermediate **19-1.** ESI-MS: m/z = 461.3 $[M+H]^+$.

3) Preparation of intermediate 19-3

Intermediate **19-3** (370 mg) was obtained by following step 4 of Example 7, while replacing intermediate **7-3** with intermediate **19-2** and replacing monomethyl pimelate with monomethyl azelate. ESI-MS: m/z = 645.3 $[M+H]^+$.

4) Preparation of intermediate 19-4

Intermediate **19-4** (210 mg) was obtained by following step 5 of Example 7, while replacing intermediate **7-4** with intermediate **19-3.** ESI-MS: m/z = 631.3 $[M+H]^+$.

5) Preparation of compound 19

Compound **19** (38 mg) was obtained by following step 6 of Example 7, while replacing intermediate **7-5** with intermediate **19-4.**

ESI-MS: m/z = 793.2 $[M+2H]^{2+}$.

$^1H$ NMR (500 MHz, $DMSO-d_6+D_2O$) δ 8.96 (s, 1H), 8.27 (s, 5H), 8.05 (s, 1H), 7.92 (s, 1H), 7.72 (m, 2H), 7.36 (m, 10H), 7.20 (m, 1H), 7.12 (m, 2H), 6.86 (s, 1H), 6.79 (m, 2H), 4.87 (m, 1H), 4.54 - 4.44 (m, 3H), 4.30 (s, 1H), 3.67 - 3.57 (m, 9H), 3.32 (m, 4H), 3.13 (s, 4H), 2.75 (s, 2H), 2.46 (s, 3H), 2.28 (s, 7H), 2.21 (s, 5H), 1.83 (s, 1H), 1.71 (s, 1H), 1.53 - 1.39 (m, 7H), 1.24 (s, 11H), 0.95 (s, 15H)

**Example 20: Preparation of Compound 20**

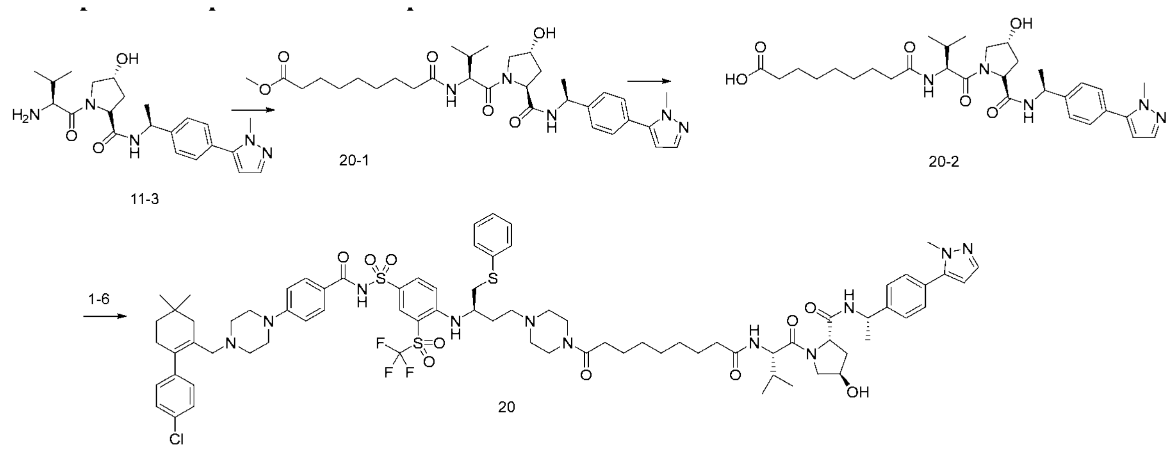


11-3 20-1 20-2 20

1) Preparation of intermediate 20-1

Intermediate **20-1** (0.3 g) was obtained by following step 4 of Example 7, while replacing intermediate **7-3** with intermediate **11-3** and replacing monomethyl pimelate with monomethyl azelate. ESI-MS: m/z = 598.3 $[M+H]^+$.

2) Preparation of intermediate 20-2

Intermediate **20-2** (140 mg) was obtained by following step 5 of Example 7, while replacing intermediate **7-4** with intermediate **20-1.** ESI-MS: m/z = 584.2 $[M+H]^+$.

3) Preparation of compound 20

Compound **20** (15 mg) was obtained by following step 6 of Example 7, while replacing intermediate **7-5** with intermediate **20-2.**

ESI-MS: m/z = 771.0 $[M+2H]^{2+}$.

$^1H$ NMR (500 MHz, DMSO) δ 8.37 (s, 1H), 8.12 (s, 2H), 7.95 (s, 1H), 7.77 (m, 3H), 7.52 - 6.81 (m, 16H), 6.35 (s, 1H), 5.10 (s, 1H), 4.89 (s, 1H), 4.72 (s, 1H), 4.53 - 4.45 (m, 2H), 4.28 (s, 2H), 4.10 (s, 2H), 3.86 (s, 3H), 3.65 (s, 5H), 3.25 - 3.08 (s, 7H), 2.90 (s, 2H), 2.60 - 1.70 (m, 23H), 1.36 (m, 15H), 0.95 (s, 11H).

**Example 21: Preparation of Compound 21**

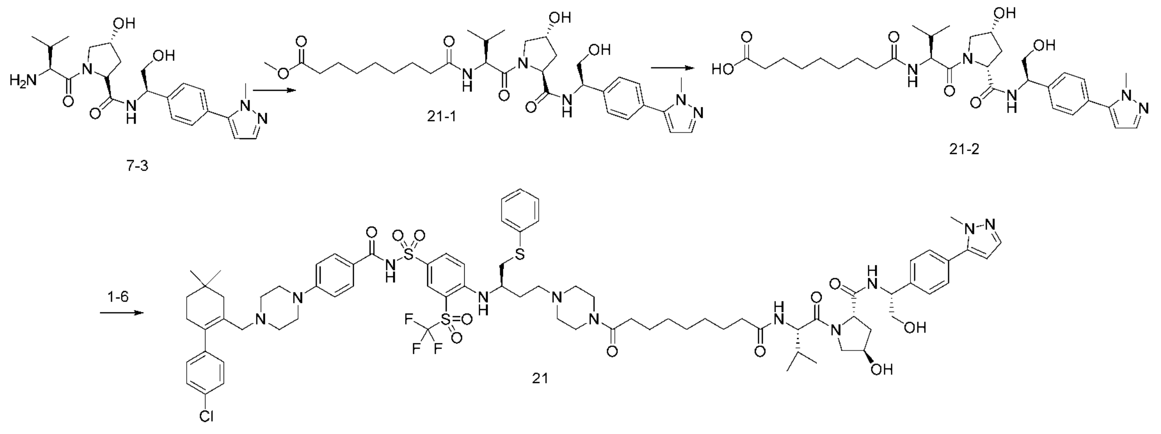


7-3 21-1 21-2 21

1) Preparation of intermediate 21-1

Intermediate **21-1** (380 mg) was obtained by following step 4 of Example 7, while replacing monomethyl pimelate with monomethyl azelate. ESI-MS: m/z = 614.3 $[M+H]^+$.

2) Preparation of intermediate 21-2

Intermediate **21-2** (200 mg) was obtained by following step 5 of Example 7, while replacing intermediate **7-4** with intermediate **21-1.** ESI-MS: m/z = 600.2 $[M+H]^+$.

3) Preparation of compound 21

Compound **21** (5 mg) was obtained by following step 6 of Example 7, while replacing intermediate **7-5** with intermediate **21-2.**

ESI-MS: m/z = 777.7 $[M+2H]^{2+}$.

$^1H$ NMR (500 MHz, DMSO) δ 8.39 (s, 1H), 8.13 (s, 2H), 7.95 (s, 1H), 7.77 (m, 3H), 7.50 - 6.81 (m, 16H), 6.36 (s, 1H), 5.09 (s, 1H), 4.92 (s, 1H), 4.74 (s, 1H), 4.57 - 4.43 (m, 2H), 4.30 (s, 2H), 4.11 (s, 2H), 3.83 (s, 3H), 3.66 (s, 5H), 3.25 - 3.06 (s, 7H), 2.85 (s, 2H), 2.61 - 1.70 (m, 26H), 1.38 (m, 12H), 0.95 (s, 11H).

**Example 22: Preparation of Compound 22**

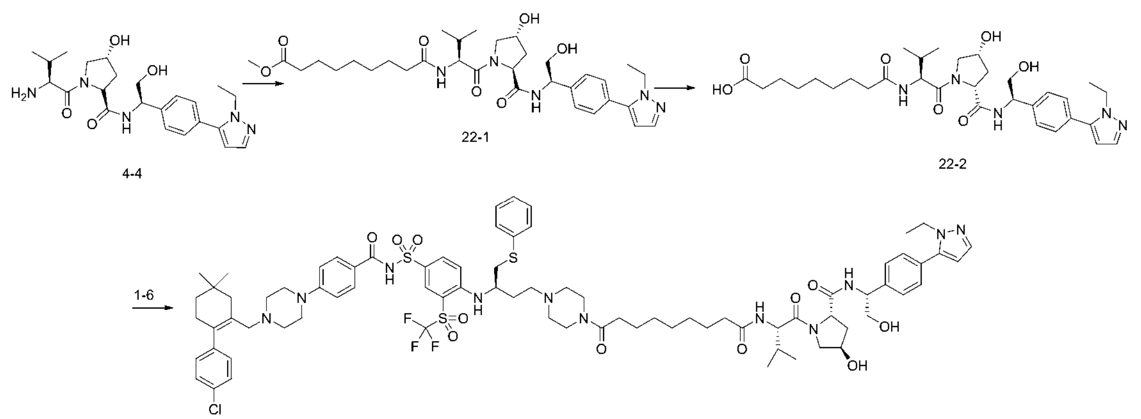


4-4 22-1 22-2

22

1) Preparation of intermediate 22-1

Intermediate **22-1** (280 mg) was obtained by following step 4 of Example 7, while replacing intermediate **7-3** with intermediate **4-4** and replacing monomethyl pimelate with monomethyl azelate. ESI-MS: m/z = 628.29 $[M+H]^+$.

2) Preparation of intermediate 22-2

Intermediate **22-2** (153 mg) was obtained by following step 5 of Example 7, while replacing intermediate **7-4** with intermediate **22-1.** ESI-MS: m/z = 614.3 $[M+H]^+$.

3) Preparation of compound 22

Compound **22** (6 mg) was obtained by following step 6 of Example 7, while replacing intermediate **7-5** with intermediate **22-2.**

ESI-MS: m/z = 784.7 $[M+2H]^{2+}$.

$^1H$ NMR (500 MHz, DMSO) δ 8.40 (s, 1H), 8.15 (s, 2H), 7.94 (s, 1H), 7.76 (m, 3H), 7.60 - 6.75 (m, 16H), 6.38 (s, 1H), 5.10 (s, 1H), 4.90 (s, 1H), 4.75 (s, 1H), 4.58 - 4.40 (m, 2H), 4.28 (s, 2H), 4.10 (s, 2H), 3.85 (s, 3H), 3.64 (s, 5H), 3.25 - 3.09 (s, 7H), 2.87 (s, 2H), 2.60 - 1.70 (m, 26H), 1.36 (m, 14H), 0.94 (s, 11H).

**Example 23: Preparation of Compound 23**

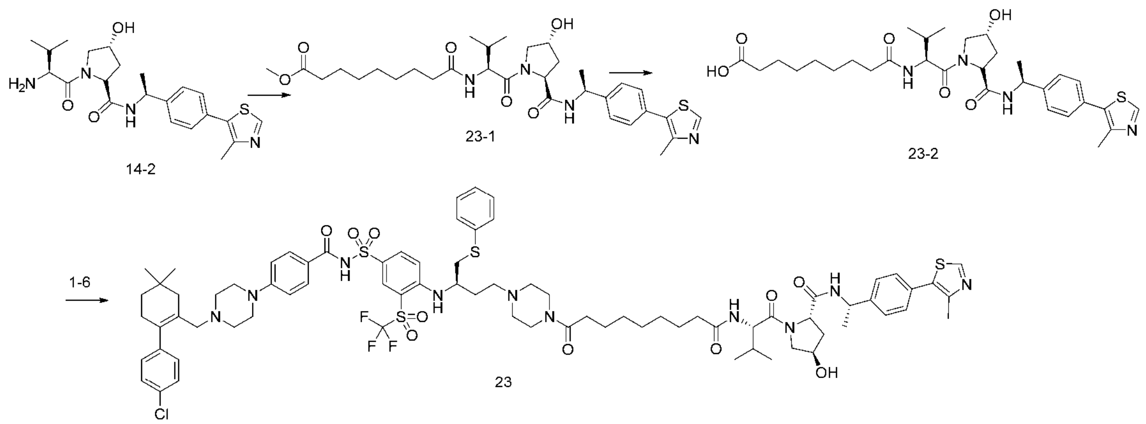


14-2

23-1

23-2

23

1) Preparation of intermediate 23-1

Intermediate **23-1** (231 mg) was obtained by following step 4 of Example 7, while replacing intermediate **7-3** with intermediate **14-2** and replacing monomethyl pimelate with monomethyl azelate. ESI-MS: m/z = 615.2 $[M+H]^+$.

2) Preparation of intermediate 23-2

Intermediate **23-2** (180 mg) was obtained by following step 5 of Example 7, while replacing intermediate **7-4** with intermediate **23-1.** ESI-MS: m/z = 601.2 $[M+H]^+$.

3) Preparation of compound 23

Compound **23** (6 mg) was obtained by following step 6 of Example 7, while replacing intermediate **7-5** with intermediate **23-2.**

ESI-MS: m/z = 778.3 $[M+2H]^{2+}$.

$^1H$ NMR (500 MHz, DMSO-$d_6$+$D_2O$) δ 8.90 (s, 1H), 8.25 (s, 5H), 8.09 (s, 1H), 7.86 (s, 1H), 7.72 (m, 2H), 7.38 (m, 10H), 7.20 (m, 1H), 7.07 (m, 2H), 6.89 (s, 1H), 6.73 (m, 2H), 4.90 (m, 1H), 4.55 - 4.33 (m, 3H), 4.29 (s, 1H), 3.66 - 3.51 (m, 8H), 3.32 (m, 4H), 3.12 (s, 4H), 2.72 (s, 3H), 2.47 (s, 3H), 2.35 (s, 5H), 2.26 (s, 5H), 1.85 (s, 1H), 1.70 (s, 1H), 1.56 - 1.32 (m, 10H), 1.26 (s, 11H), 0.94 (s, 12H).

**Example 24: Preparation of Compound 24**

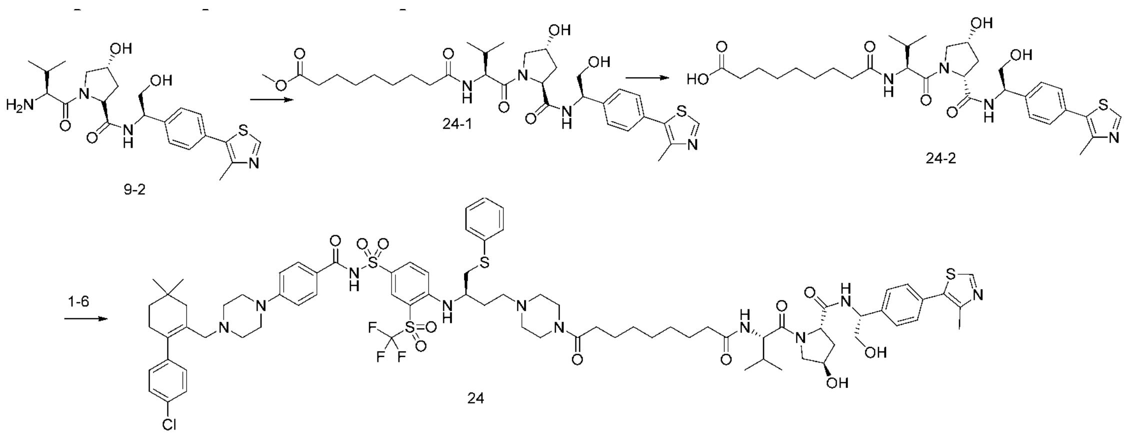


9-2 24-1 24-2 24

1) Preparation of intermediate 24-1

Intermediate **24-1** (65 mg) was obtained by following step 4 of Example 7, while replacing intermediate **7-3** with intermediate **9-2** and replacing monomethyl pimelate with monomethyl azelate. ESI-MS: m/z = 631.3 $[M+H]^+$.

2) Preparation of intermediate 24-2

Intermediate **24-2** (55 mg) was obtained by following step 5 of Example 7, while replacing intermediate **7-4** with intermediate **24-1.** ESI-MS: m/z = 617.2 $[M+H]^+$.

3) Preparation of compound 24

Compound **24** (4 mg) was obtained by following step 6 of Example 7, while replacing intermediate **7-5** with intermediate **24-2.**

ESI-MS: m/z = 786.3 $[M+2H]^{2+}$.

$^1$H NMR (500 MHz, DMSO-$d_6$+$D_2O$) δ 8.96 (s, 1H), 8.26 (s, 5H), 8.08 (s, 1H), 7.89 (s, 1H), 7.68 (m, 2H), 7.36 (m, 10H), 7.19 (m, 1H), 7.11 (m, 2H), 6.93 (s, 1H), 6.78 (m, 2H), 4.87 (m, 1H), 4.53 - 4.43 (m, 3H), 4.28 (s, 1H), 3.66 - 3.50 (m, 9H), 3.31 (m, 4H), 3.13 (s, 4H), 2.75 (s, 3H), 2.46 (s, 3H), 2.31 (s, 7H), 2.22 (s, 5H), 1.86 (s, 1H), 1.72 (s, 1H), 1.54 - 1.32 (m, 9H), 1.23 (s, 9H), 0.95 (s, 12H).

**Example 25: Preparation of Compound 25**

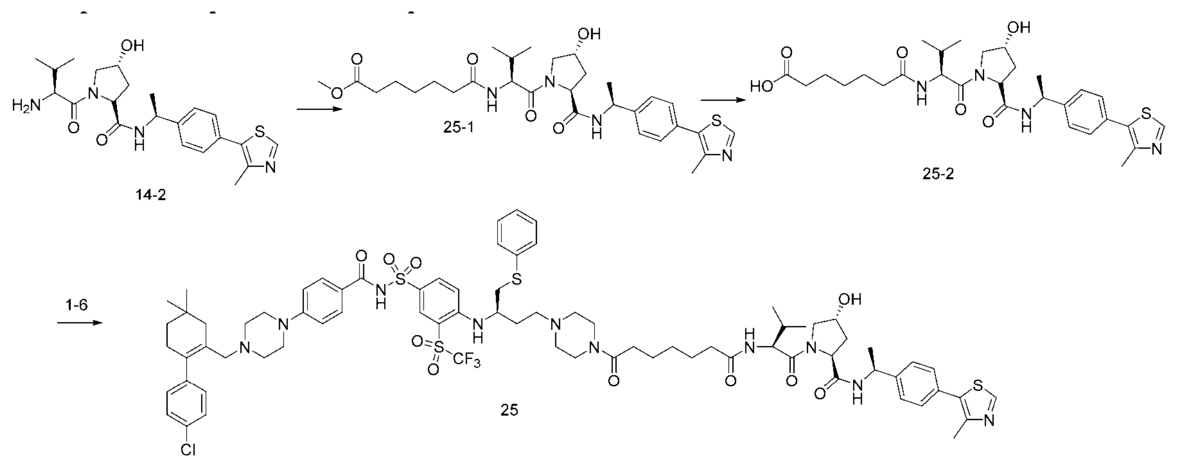


14-2 25-1 25-2 25

1) Preparation of intermediate 25-1

Intermediate **25-1** (120 mg) was obtained by following step 4 of Example 7, while replacing intermediate **7-3** with

intermediate **14-2.** ESI-MS: m/z = 587.3 [M+H]+.

2) Preparation of intermediate 25-2

Intermediate **25-2** (100 mg) was obtained by following step 5 of Example 7, while replacing intermediate **7-4** with intermediate 25-1. ESI-MS: m/z = 573.3 [M+H]+.

3) Preparation of compound 25

Compound **25** (15 mg) was obtained by following step 6 of Example 7, while replacing intermediate **7-5** with intermediate **25-2.**

ESI-MS: m/z = 764.3 $[M+2H]^{2+}$.

$^1$H NMR (500 MHz, DMSO) δ 12.18 (s, 1H), 8.99 (s, 1H), 8.35 (m, 1H), 8.19 (m, 1H), 8.00 (m, 1H), 7.90 (m, 1H), 7.78 (m, 2H), 7.47 - 7.35 (m, 6H), 7.29 (m, 2H), 7.23 (m, 2H), 7.19 - 7.12 (m, 4H), 6.97 (m, 2H), 4.91 (m, 1H), 4.44 - 4.27 (m, 5H), 4.13 (s, 2H), 3.60 (m, 6H), 3.37 (m, 7H), 3.19 (s, 2H), 2.83 - 2.74 (m, 2H), 2.46 (s, 3H), 2.31 (m, 4H), 2.22 - 1.88 (m, 9H), 1.79 (m, 1H), 1.48 (m, 7H), 1.37 (m, 3H), 1.26 (m, 6H), 1.00 (s, 6H), 0.91 - 0.81 (s, 6H).

**Example 26: Preparation of Compound 26**

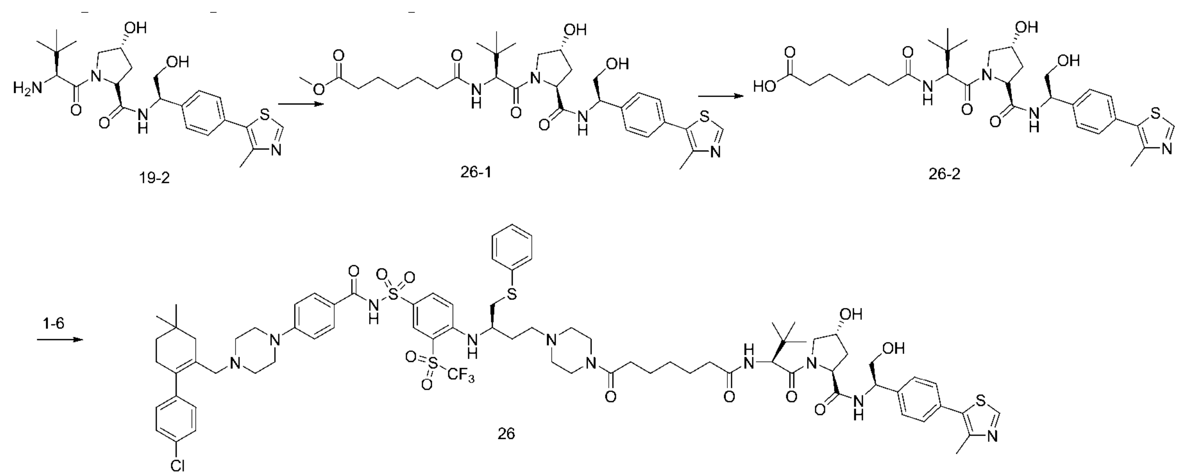


19-2 26-1 26-2

26

1) Preparation of intermediate 26-1

Intermediate **26-1** (150 mg) was obtained by following step 4 of Example 7, while replacing intermediate **7-3** with intermediate **19-2.** ESI-MS: m/z = 617.3 [M+H]+.

2) Preparation of intermediate 26-2

Intermediate **26-2** (86 mg) was obtained by following step 5 of Example 7, while replacing intermediate **7-4** with intermediate **26-1.** ESI-MS: m/z = 603.3 [M+H]+.

3) Preparation of compound 26

Compound **26** (12 mg) was obtained by following step 6 of Example 7, while replacing intermediate **7-5** with intermediate **26-2.** ESI-MS: m/z = 779.2 $[M+2H]^{2+}$.

**Example 27: Preparation of Compound 27**

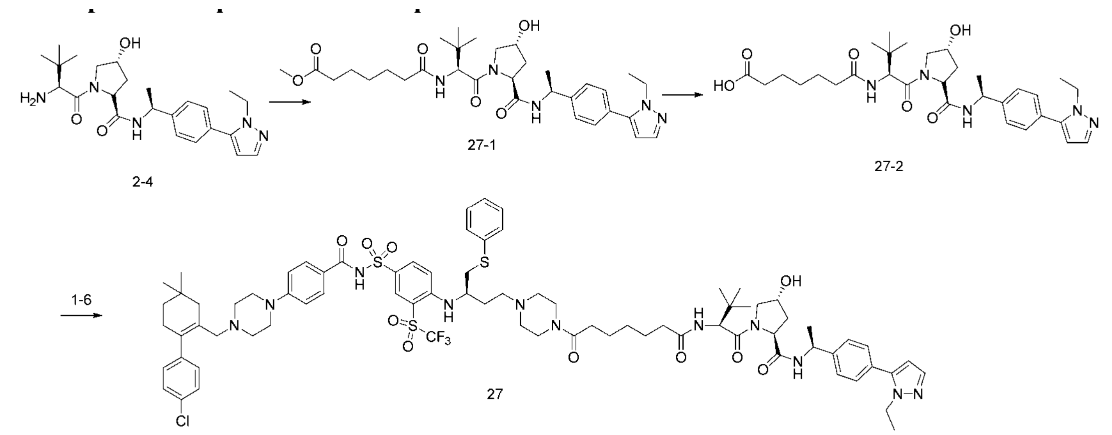


2-4 27-1 27-2 27

1) Preparation of intermediate 27-1

Intermediate **27-1** (500 mg) was obtained by following step 4 of Example 7, while replacing intermediate **7-3** with intermediate **2-4.** ESI-MS: m/z = 598.3 $[M+H]^+$.

2) Preparation of intermediate 27-2

Intermediate **27-2** (334 mg) was obtained by following step 5 of Example 7, while replacing intermediate **7-4** with intermediate **27-1.** ESI-MS: m/z = 584.3 $[M+H]^+$.

3) Preparation of compound 27

Compound **27** (50 mg) was obtained by following step 6 of Example 7, while replacing intermediate **7-5** with intermediate **27-2.** ESI-MS: m/z = 769.7 $[M+2H]^{2+}$.

$^1H$ NMR (500 MHz, $CDCl_3$) δ 8.34 (d, 1H), 8.17 (s, 2H), 8.12 - 8.07 (m, 1H), 7.69 (t, 2H), 7.54 (d, 1H), 7.37 (dt, 7H), 7.29 (dd, 6H), 7.07 (t, 1H), 6.99 (d, 2H), 6.76 (d, 2H), 6.61 (d, 1H), 6.48 (d, 1H), 6.24 (d, 1H), 5.09 (dd, 2H), 4.70 (dd, 2H), 4.62 (d, 1H), 4.51 (s, 1H), 4.14 (dt, 3H), 3.89 (s, 1H), 3.75 - 3.53 (m, 3H), 3.52 - 3.43 (m, 3H), 3.14 - 3.08 (m, 3H), 3.06 - 2.97 (m, 1H), 2.61 (s, 4H), 2.51 - 2.39 (m, 6H), 2.36 - 2.07 (m, 10H), 1.76 - 1.66 (m, 1H), 1.57 - 1.40 (m, 9H), 1.28 (s, 6H), 1.04 (s, 9H), 0.98 (s, 6H).

**Example 28: Preparation** of **Compound 28**

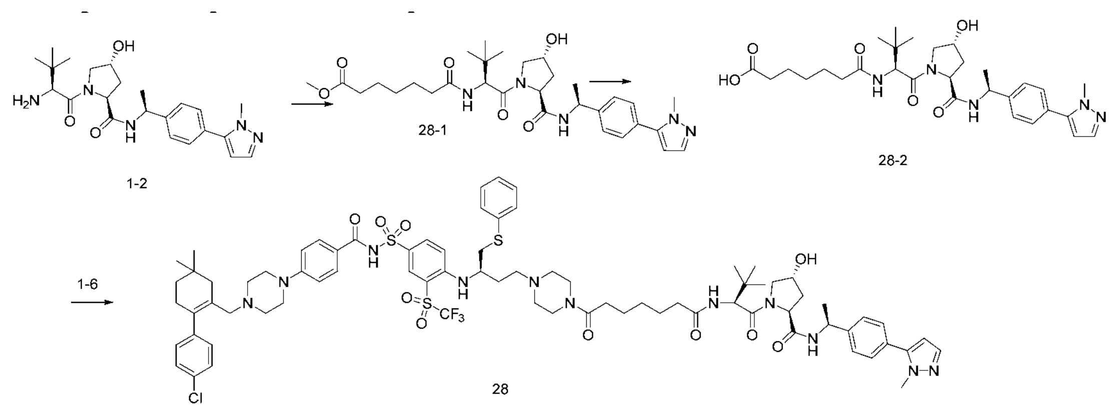


1-2 28-1 28-2 28

1) Preparation of intermediate 28-1

Intermediate **28-1** (423 mg) was obtained by following step 4 of Example 7, while replacing intermediate **7-3** with intermediate **1-2.** ESI-MS: m/z = 584.3 $[M+H]^+$.

2) Preparation of intermediate 28-2

Intermediate **28-2** (260 mg) was obtained by following step 5 of Example 7, while replacing intermediate **7-4** with intermediate **28-1.** ESI-MS: m/z = 570.3 [M+H]$^+$.

3) Preparation of compound 28

Compound **28** (20 mg) was obtained by following step 6 of Example 7, while replacing intermediate **7-5** with intermediate **28-2.**

ESI-MS: m/z = 762.8 [M+2H]$^{2+}$.

$^1$H NMR (500 MHz, $CDCl_3$) δ 8.34 (d, 1H), 8.16 (s, 2H), 8.12 - 8.06 (m, 1H), 7.70 (t, 2H), 7.55 (d, 1H), 7.37 (dt, 7H), 7.30 (dd, 6H), 7.06 (t, 1H), 6.99 (d, 2H), 6.76 (d, 2H), 6.61 (d, 1H), 6.48 (d, 1H), 6.25 (d, 1H), 5.09 (dd, 2H), 4.70 (dd, 2H), 4.62 (d, 1H), 4.52 (s, 1H), 4.14 (dt, 3H), 3.89 (s, 1H), 3.75 - 3.54 (m, 3H), 3.52 - 3.42 (m, 3H), 3.14 - 3.08 (m, 3H), 3.08 - 2.97 (m, 1H), 2.61 (s, 4H), 2.51 - 2.39 (m, 6H), 2.36 - 2.07 (m, 10H), 1.76 - 1.66 (m, 1H), 1.57 - 1.28 (m, 13H), 1.04 (s, 9H), 0.98 (s, 6H).

**Example 29: Preparation of Compound 29**

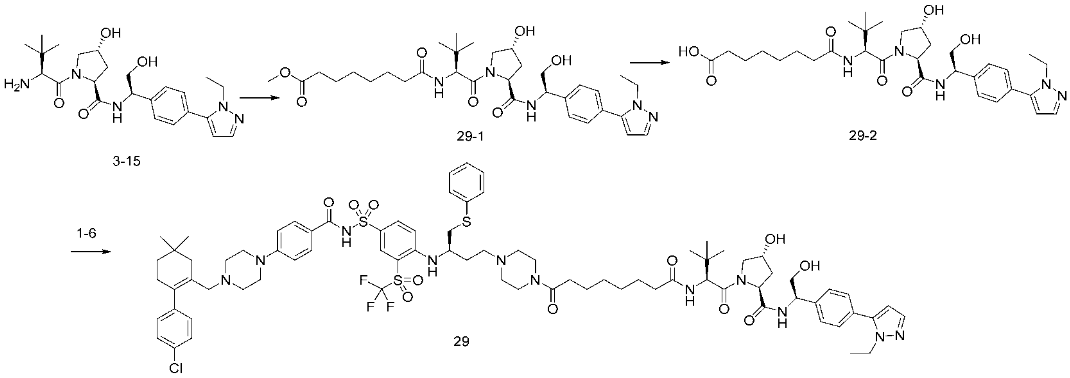


3-15

29-1

29-2

29

1) Preparation of intermediate 29-1

Intermediate **29-1** (113 mg) was obtained by following step 4 of Example 7, while replacing intermediate **7-3** with intermediate **3-15** and replacing monomethyl pimelate with monomethyl suberate. ESI-MS: m/z = 628.3 [M+H]$^+$.

2) Preparation of intermediate 29-2

Intermediate **29-2** (69 mg) was obtained by following step 5 of Example 7, while replacing intermediate **7-4** with intermediate **29-1.** ESI-MS: m/z = 614.3 [M+H]$^+$.

3) Preparation of compound 29

Compound **29** (13 mg) was obtained by following step 6 of Example 7, while replacing intermediate **7-5** with intermediate **29-2.** ESI-MS: m/z = 784.8 [M+2H]$^{2+}$.

$^1$H NMR (500 MHz, DMSO) δ 8.40 (s, 1H), 8.14 (s, 2H), 7.93 (s, 1H), 7.75 (m, 3H), 7.50 - 6.80 (m, 16H), 6.35 (s, 1H), 5.12 (s, 1H), 4.94 (s, 1H), 4.73 (s, 1H), 4.55 - 4.45 (m, 2H), 4.29 (s, 2H), 4.08 (s, 2H), 3.85 (s, 3H), 3.63 (s, 5H), 3.26 - 3.10 (s, 7H), 2.89 (s, 2H), 2.62 - 1.75 (m, 24H), 1.35 (m, 12H), 0.94 (s, 15H).

**Example 30: Preparation of Compound 30**

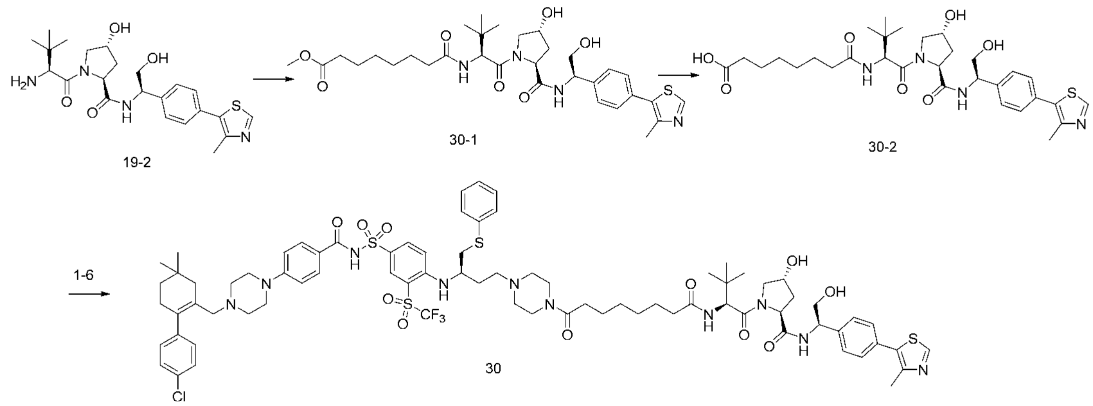


19-2 30-1 30-2 30

1) Preparation of intermediate 30-1

Intermediate **30-1** (100 mg) was obtained by following step 4 of Example 7, while replacing intermediate 7-3 with intermediate **19-2** and replacing monomethyl pimelate with monomethyl suberate. ESI-MS: m/z = 631.3 $[M+H]^+$.

2) Preparation of intermediate 30-2

Intermediate **30-2** (50 mg) was obtained by following step 5 of Example 7, while replacing intermediate **7-4** with intermediate **30-1.** ESI-MS: m/z = 617.3 $[M+H]^+$.

3) Preparation of compound 30

Compound **30** (4 mg) was obtained by following step 6 of Example 7, while replacing intermediate **7-5** with intermediate **30-2.**

ESI-MS: m/z = 786.3 $[M+2H]^{2+}$.

$^1$H NMR (500 MHz, DMSO) δ 8.98 (s, 1H), 8.35 (m, 1H), 8.12 (s, 1H), 7.95 (m, 1H), 7.79 (m, 1H), 7.72 (m, 2H), 7.38 (m, 8H), 7.27 (m, 2H), 7.18 (m, 1H), 7.12 (m, 2H), 6.98 (m, 1H), 6.85 (m, 2H), 5.10 (m, 1H), 4.88 (m, 1H), 4.75 (m, 1H), 4.54 - 4.44 (m, 2H), 4.29 (s, 1H), 4.08 (s, 1H), 3.61 (m, 5H), 3.23 (s, 5H), 2.82 (s, 2H), 2.46 (s, 3H), 2.36 (s, 6H), 2.22 (m, 8H), 2.15 - 2.06 (m, 2H), 1.99 (s, 5H), 1.91 (s, 1H), 1.87 - 1.73 (m, 2H), 1.53 - 1.38 (m, 7H), 1.24 (s, 7H), 0.95 (s, 15H).

**Example 31: Preparation of Compound 31**

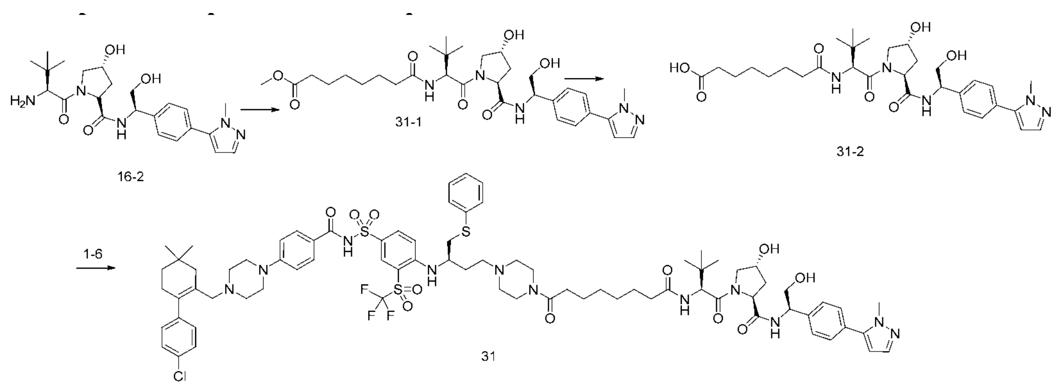


16-2 31-1 31-2 31

1) Preparation of intermediate 31-1

Intermediate **31-1** (117 mg) was obtained by following step 4 of Example 7, while replacing intermediate **7-3** with intermediate **16-2** and replacing monomethyl pimelate with monomethyl suberate. ESI-MS: m/z = 614.3 $[M+H]^+$.

2) Preparation of intermediate 31-2

Intermediate **31-2** (50 mg) was obtained by following step 5 of Example 7, while replacing intermediate **7-4** with intermediate **31-1.** ESI-MS: m/z = 600.3 $[M+H]^+$.

3) Preparation of compound 31

Compound **31** (6 mg) was obtained by following step 6 of Example 7, while replacing intermediate **7-5** with intermediate **31-2.** ESI-MS: m/z = 777.7 $[M+2H]^{2+}$.

$^1H$ NMR (500 MHz, $CDCl_3$) δ 8.35 (d, 1H), 8.16 (s, 2H), 8.12 - 8.06 (m, 1H), 7.71 (t, 2H), 7.56 (d, 1H), 7.37 (dt, 7H), 7.30 (dd, 6H), 7.06 (t, 1H), 6.98 (d, 2H), 6.76 (d, 2H), 6.61 (d, 1H), 6.48 (d, 1H), 6.23 (d, 1H), 5.09 (dd, 2H), 4.70 (dd, 2H), 4.62 (d, 1H), 4.53 (s, 1H), 4.15 (dt, 3H), 3.89 (s, 1H), 3.75 - 3.54 (m, 3H), 3.51 - 3.42 (m, 3H), 3.14 - 3.08 (m, 3H), 3.08 - 2.97 (m, 1H), 2.61 (s, 4H), 2.51 - 2.39 (m, 6H), 2.36 - 2.08 (m, 10H), 1.77 - 1.66 (m, 1H), 1.57 - 1.28 (m, 15H), 1.04 (s, 9H), 0.98 (s, 6H).

**Example 32: Preparation of Compound 32**

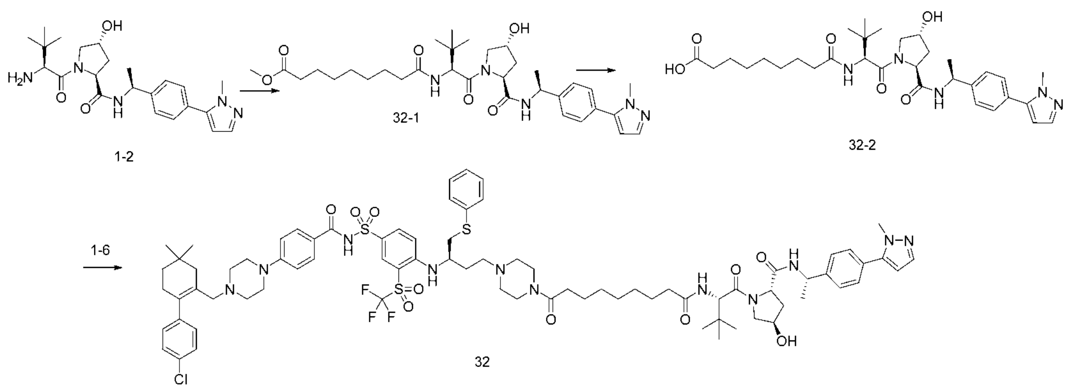


1) Preparation of intermediate 32-1

Intermediate **32-1** (117 mg) was obtained by following step 4 of Example 7, while replacing intermediate **7-3** with intermediate **1-2** and replacing monomethyl pimelate with monomethyl azelate. ESI-MS: m/z = 614.3 $[M+H]^+$.

2) Preparation of intermediate 32-2

Intermediate **32-2** (50 mg) was obtained by following step 5 of Example 7, while replacing intermediate **7-4** with intermediate **32-1.** ESI-MS: m/z = 600.3 $[M+H]^+$.

3) Preparation of compound 32

Compound **32** (20 mg) was obtained by following step 6 of Example 7, while replacing intermediate **7-5** with intermediate **32-2.** ESI-MS: m/z = 776.8 $[M+2H]^{2+}$.

$^1H$ NMR (500 MHz, $CDCl_3$) δ 8.35 (d, 1H), 8.15 (s, 2H), 8.12 - 8.06 (m, 1H), 7.71 (t, 2H), 7.56 (d, 1H), 7.38 (dt, 7H), 7.30 (dd, 6H), 7.06 (t, 1H), 6.99 (d, 2H), 6.76 (d, 2H), 6.60 (d, 1H), 6.48 (d, 1H), 6.23 (d, 1H), 5.09 (dd, 2H), 4.70 (dd, 2H), 4.62 (d, 1H), 4.53 (s, 1H), 4.15 (dt, 3H), 3.89 (s, 1H), 3.75 - 3.55 (m, 3H), 3.51 - 3.42 (m, 3H), 3.14 - 3.08 (m, 3H), 3.09 - 2.97 (m, 1H), 2.61 (s, 4H), 2.52 - 2.39 (m, 6H), 2.36 - 2.08 (m, 10H), 1.77 - 1.66 (m, 1H), 1.59 - 1.26 (m, 17H), 1.05 (s, 9H), 0.98 (s, 6H).

**Example 33: Preparation of Compound 33**

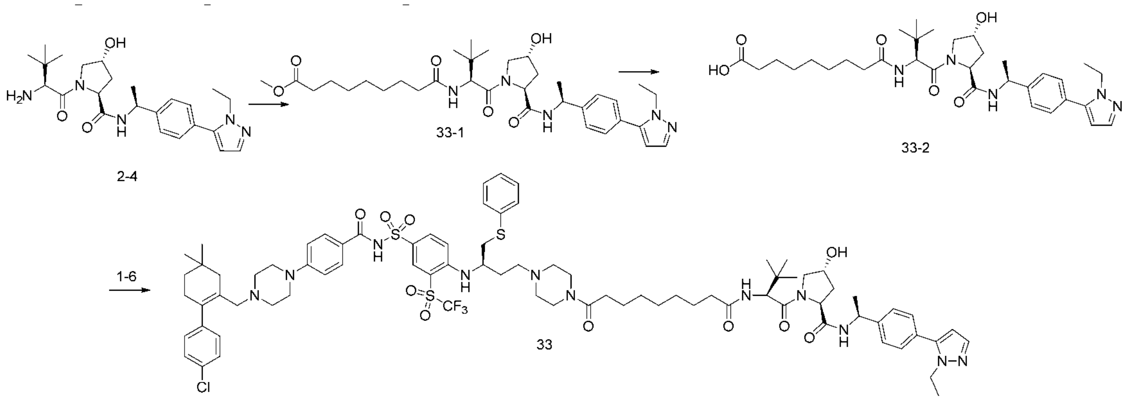


2-4 33-1 33-2 33

1) Preparation of intermediate 33-1

Intermediate **33-1** (150 mg) was obtained by following step 4 of Example 7, while replacing intermediate 7-3 with intermediate **2-4** and replacing monomethyl pimelate with monomethyl azelate. ESI-MS: m/z = 626.3 $[M+H]^+$.

2) Preparation of intermediate 33-2

Intermediate **33-2** (105 mg) was obtained by following step 5 of Example 7, while replacing intermediate 7-4 with intermediate **33-1.** ESI-MS: m/z = 612.3 $[M+H]^+$.

3) Preparation of compound 33

Compound **33** (30 mg) was obtained by following step 6 of Example 7, while replacing intermediate **7-5** with intermediate **33-2.** ESI-MS: m/z = 783.8 $[M+2H]^{2+}$.

$^1$H NMR (500 MHz, DMSO) δ 8.41 (s, 1H), 8.15 (s, 2H), 7.92 (s, 1H), 7.74 (m, 3H), 7.50 - 6.81 (m, 16H), 6.34 (s, 1H), 5.12 (s, 1H), 4.95 (s, 1H), 4.73 (s, 1H), 4.55 - 4.45 (m, 2H), 4.27 (s, 2H), 4.08 (s, 2H), 3.86 (s, 3H), 3.63 (s, 5H), 3.26 - 3.10 (s, 7H), 2.88 (s, 2H), 2.62 - 1.75 (m, 24H), 1.35 (m, 14H), 0.95 (s, 15H).

**Example 34: Preparation of Compound 34**

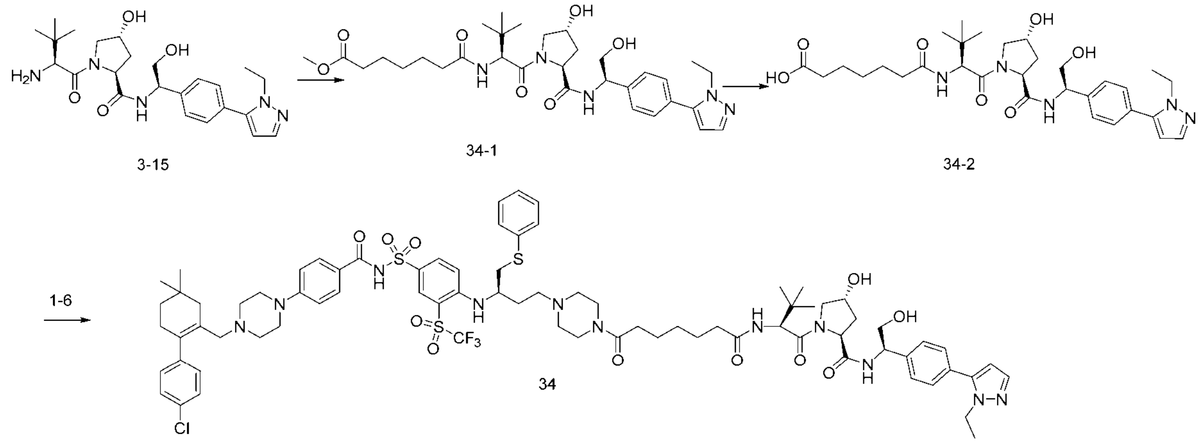


3-15 34-1 34-2 34

1) Preparation of intermediate 34-1

Intermediate **34-1** (93 mg) was obtained by following step 4 of Example 7, while replacing intermediate **7-3** with intermediate **3-15.** ESI-MS: m/z = 614.3 $[M+H]^+$.

2) Preparation of intermediate 34-2

Intermediate **34-2** (45 mg) was obtained by following step 5 of Example 7, while replacing intermediate **7-4** with intermediate **34-1.** ESI-MS: m/z = 600.3 $[M+H]^+$.

3) Preparation of compound 34

Compound **34** (5 mg) was obtained by following step 6 of Example 7, while replacing intermediate **7-5** with intermediate **34-2.** ESI-MS: m/z = 777.8 $[M+2H]^{2+}$.

$^1$H NMR (500 MHz, DMSO) δ 8.41 (s, 1H), 8.14 (s, 2H), 7.94 (s, 1H), 7.75 (m, 3H), 7.52 - 6.80 (m, 16H), 6.34 (s, 1H), 5.12 (s, 1H), 4.94 (s, 1H), 4.73 (s, 1H), 4.54 - 4.44 (m, 2H), 4.29 (s, 2H), 4.08 (s, 2H), 3.85 (s, 3H), 3.63 (s, 5H), 3.26 - 3.10 (s, 7H), 2.89 (s, 2H), 2.61 - 1.75 (m, 23H), 1.35 (m, 11H), 0.96 (s, 15H).

**Example 35: Preparation of Compound 35**

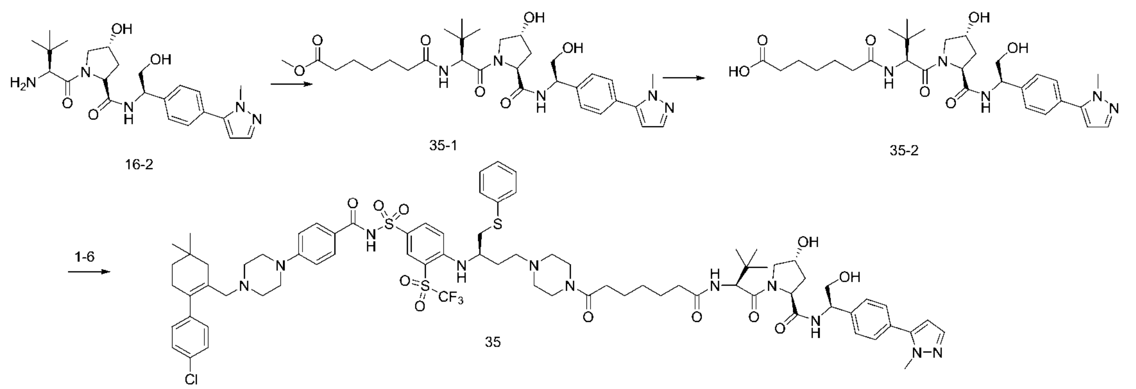


1) Preparation of intermediate 35-1

Intermediate **35-1** (120 mg) was obtained by following step 4 of Example 7, while replacing intermediate **7-3** with intermediate **16-2.** ESI-MS: m/z = 600.3 $[M+H]^+$.

2) Preparation of intermediate 35-2

Intermediate **35-2** (80 mg) was obtained by following step 5 of Example 7, while replacing intermediate **7-4** with intermediate **35-1.** ESI-MS: m/z = 586.3 $[M+H]^+$.

3) Preparation of compound 35

Compound **35** (15 mg) was obtained by following step 6 of Example 7, while replacing intermediate **7-5** with intermediate **35-2.** ESI-MS: m/z = 770.7 $[M+2H]^{2+}$.

$^1$H NMR (500 MHz, $CDCl_3$) δ 8.34 (d, 1H), 8.16 (s, 2H), 8.12 - 8.06 (m, 1H), 7.70 (t, 2H), 7.56 (d, 1H), 7.37 (dt, 7H), 7.31 (dd, 6H), 7.06 (t, 1H), 6.98 (d, 2H), 6.75 (d, 2H), 6.61 (d, 1H), 6.48 (d, 1H), 6.23 (d, 1H), 5.09 (dd, 2H), 4.70 (dd, 2H), 4.62 (d, 1H), 4.53 (s, 1H), 4.15 (dt, 3H), 3.89 (s, 1H), 3.75 - 3.54 (m, 3H), 3.51 - 3.42 (m, 3H), 3.14 - 3.08 (m, 3H), 3.08 - 2.97 (m, 1H), 2.61 (s, 4H), 2.51 - 2.38 (m, 6H), 2.36 - 2.08 (m, 10H), 1.77 - 1.65 (m, 1H), 1.57 - 1.28 (m, 13H), 1.05 (s, 9H), 0.97 (s, 6H).

**Example 36: Preparation of Compound 36**

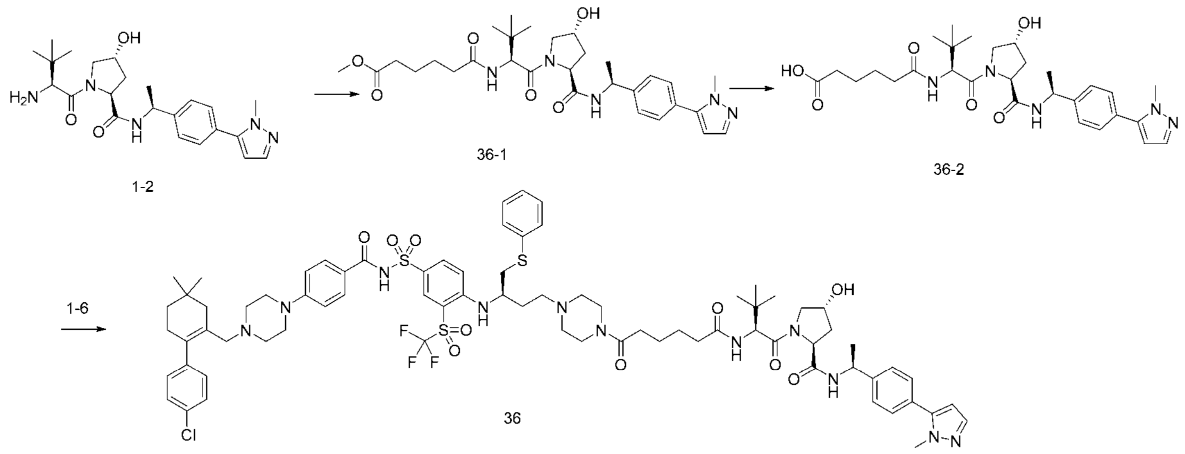


1-2 36-1 36-2 36

1) Preparation of intermediate 36-1

Intermediate **36-1** (110 mg) was obtained by following step 4 of Example 7, while replacing intermediate **7-3** with intermediate **1-2** and replacing monomethyl pimelate with monomethyl adipate. ESI-MS: m/z = 570.3 $[M+H]^+$.

2) Preparation of intermediate 36-2

Intermediate **36-2** (100 mg) was obtained by following step 5 of Example 7, while replacing intermediate **7-4** with intermediate **36-1.** ESI-MS: m/z = 556.3 $[M+H]^+$.

3) Preparation of compound 36

Compound **36** (25 mg) was obtained by following step 6 of Example 7, while replacing intermediate **7-5** with intermediate **36-2.** ESI-MS: m/z = 755.8 $[M+2H]^{2+}$.

$^1H$ NMR (500 MHz, $CDCl_3$) δ 8.35 (d, 1H), 8.15 (s, 2H), 8.12 - 8.06 (m, 1H), 7.71 (t, 2H), 7.56 (d, 1H), 7.38 (dt, 7H), 7.31 (dd, 6H), 7.07 (t, 1H), 6.99 (d, 2H), 6.76 (d, 2H), 6.60 (d, 1H), 6.48 (d, 1H), 6.24 (d, 1H), 5.09 (dd, 2H), 4.71 (dd, 2H), 4.62 (d, 1H), 4.53 (s, 1H), 4.14 (dt, 3H), 3.89 (s, 1H), 3.75 - 3.55 (m, 3H), 3.51 - 3.42 (m, 3H), 3.14 - 3.08 (m, 3H), 3.09 - 2.97 (m, 1H), 2.61 (s, 4H), 2.52 - 2.39 (m, 6H), 2.36 - 2.08 (m, 10H), 1.60 - 1.26 (m, 12H), 1.05 (s, 9H), 0.97 (s, 6H).

**Example 37: Preparation of Compound 37**

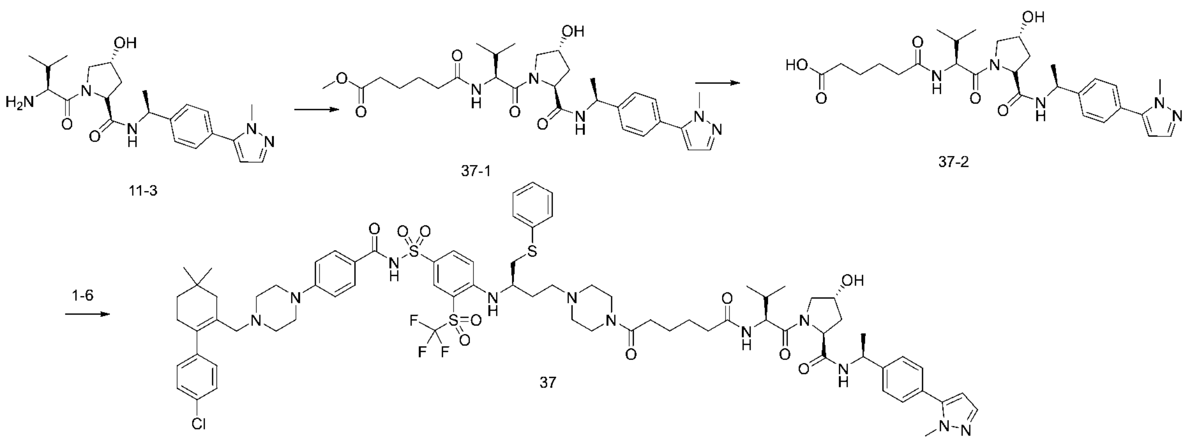


11-3 37-1 37-2 37

1) Preparation of intermediate 37-1

Intermediate **37-1** (160 mg) was obtained by following step 4 of Example 7, while replacing intermediate **7-3** with intermediate **11-3** and replacing monomethyl pimelate with monomethyl adipate. ESI-MS: m/z = 556.2 $[M+H]^+$.

2) Preparation of intermediate 37-2

Intermediate **37-2** (120 mg) was obtained by following step 5 of Example 7, while replacing intermediate **7-4** with intermediate **37-1.** ESI-MS: m/z = 542.3 $[M+H]^+$.

3) Preparation of compound 37

Compound **37** (20 mg) was obtained by following step 6 of Example 7, while replacing intermediate **7-5** with intermediate **37-2.** ESI-MS: m/z = 748.7 $[M+2H]^{2+}$.

$^1$H NMR (500 MHz, DMSO) δ 8.37 (s, 1H), 8.12 (s, 2H), 7.95 (s, 1H), 7.77 (m, 3H), 7.52 - 6.81 (m, 16H), 6.35 (s, 1H), 5.10 (s, 1H), 4.89 (s, 1H), 4.72 (s, 1H), 4.53 - 4.45 (m, 2H), 4.28 (s, 2H), 4.10 (s, 2H), 3.86 (s, 3H), 3.65 (s, 5H), 3.25 - 3.08 (s, 7H), 2.90 (s, 2H), 2.60 - 1.70 (m, 21H), 1.36 (m, 10H), 0.95 (s, 12H).

**Example 38: Preparation of Compound 38**

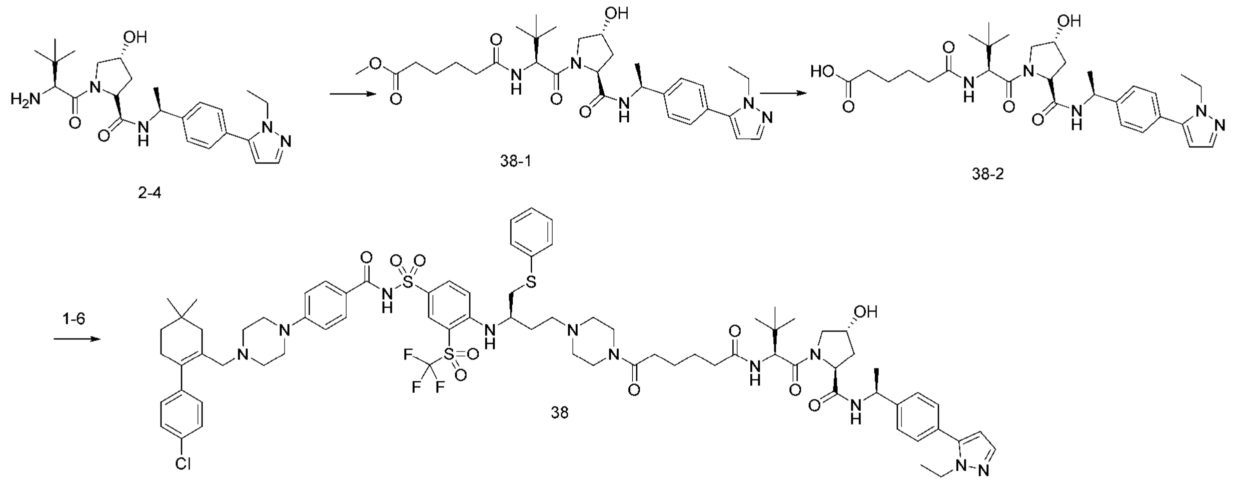


2-4 38-1 38-2 38

1) Preparation of intermediate 38-1

Intermediate **38-1** (100 mg) was obtained by following step 4 of Example 7, while replacing intermediate **7-3** with intermediate **2-4** and replacing monomethyl pimelate with monomethyl adipate. ESI-MS: m/z = 584.3 $[M+H]^+$.

2) Preparation of intermediate 38-2

Intermediate **38-2** (80 mg) was obtained by following step 5 of Example 7, while replacing intermediate **7-4** with intermediate **38-1.** ESI-MS: m/z = 570.3 $[M+H]^+$.

3) Preparation of compound 38

Compound **38** (20 mg) was obtained by following step 6 of Example 7, while replacing intermediate **7-5** with intermediate **38-2.** ESI-MS: m/z = 762.7 $[M+2H]^{2+}$.

$^1$H NMR (500 MHz, DMSO) δ 8.40 (s, 1H), 8.14 (s, 2H), 7.92 (s, 1H), 7.75 (m, 3H), 7.51 - 6.82 (m, 16H), 6.34 (s, 1H), 5.12 (s, 1H), 4.94 (s, 1H), 4.74 (s, 1H), 4.54 - 4.45 (m, 2H), 4.27 (s, 2H), 4.08 (s, 2H), 3.86 (s, 3H), 3.63 (s, 5H), 3.26 - 3.10 (s, 7H), 2.89 (s, 2H), 2.62 - 1.74 (m, 22H), 1.35 (m, 10H), 0.96 (s, 15H).

**Example 39: Preparation of Compound 39**

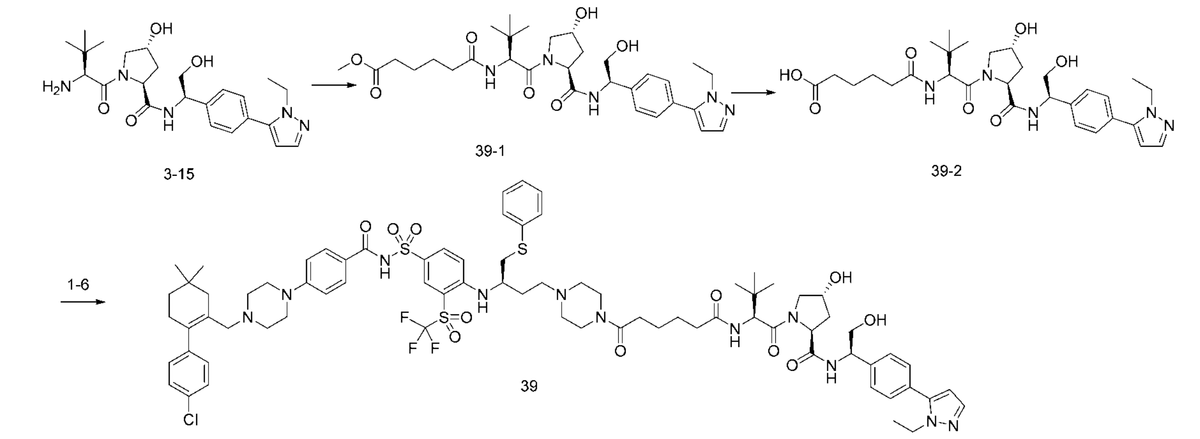


3-15 39-1 39-2 39

1) Preparation of intermediate 39-1

Intermediate **39-1** (80 mg) was obtained by following step 4 of Example 7, while replacing intermediate **7-3** with intermediate **3-15** and replacing monomethyl pimelate with monomethyl adipate. ESI-MS: m/z = 600.3 $[M+H]^+$.

2) Preparation of intermediate 39-2

Intermediate **39-2** (40 mg) was obtained by following step 5 of Example 7, while replacing intermediate **7-4** with intermediate **39-1.** ESI-MS: m/z = 586.3 $[M+H]^+$.

3) Preparation of compound 39

Compound **39** (3 mg) was obtained by following step 6 of Example 7, while replacing intermediate **7-5** with intermediate **39-2.**

ESI-MS: m/z = 770.8 $[M+2H]^{2+}$.

$^1$H NMR (500 MHz, DMSO) δ 8.36 (m, 1H), 8.14 (m, 1H), 7.96 (m, 1H), 7.81 (m, 1H), 7.72 (m, 2H), 7.49 (m, 1H), 7.40 (m, 7H), 7.32 (m, 2H), 7.26 (m, 2H), 7.17 (m, 2H), 7.12 (m, 2H), 7.03 (m, 1H), 6.93 - 6.85 (m, 3H), 6.31 (m, 1H), 5.10 (m, 1H), 4.90 (m, 1H), 4.74 (m, 1H), 4.56 - 4.43 (m, 2H), 4.29 (m, 1H), 4.11 (m, 4H), 3.67 - 3.55 (m, 5H), 2.36 (s, 5H), 2.30 - 2.22 (m, 8H), 2.12 (m, 2H), 2.00 (m, 6H), 1.83 (m, 2H), 1.51 - 1.41 (m, 8H), 1.34 - 1.22 (m, 9H), 0.95 (s, 15H).

**Example 40: Preparation of Compound 40**

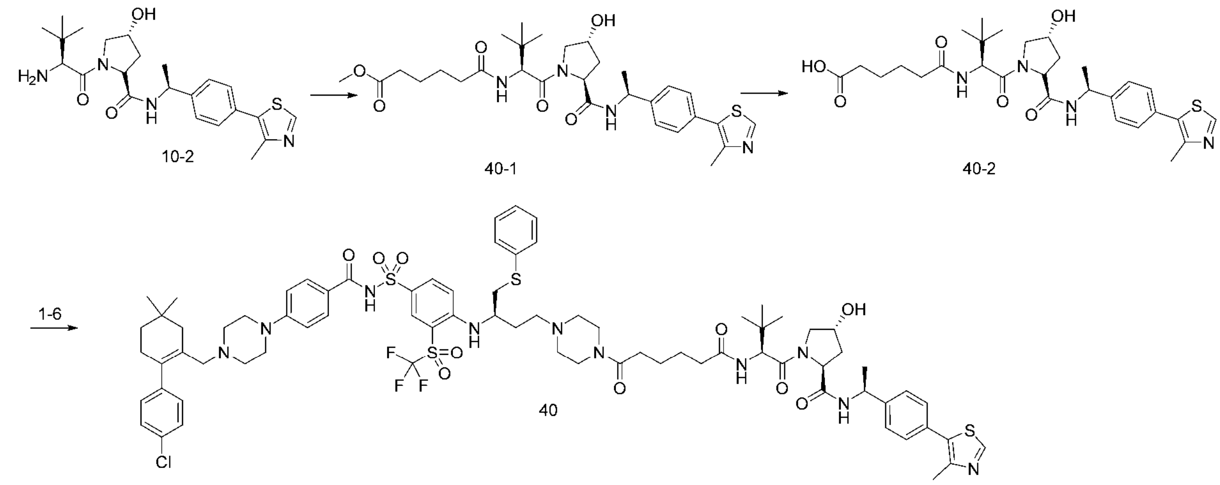


10-2 40-1 40-2 40

1) Preparation of intermediate 40-1

Intermediate **40-1** (135 mg) was obtained by following step 4 of Example 7, while replacing intermediate **7-3** with intermediate **10-2** and replacing monomethyl pimelate with monomethyl adipate. ESI-MS: m/z = 587.3 $[M+H]^+$.

2) Preparation of intermediate 40-2

Intermediate **40-2** (75 mg) was obtained by following step 5 of Example 7, while replacing intermediate **7-4** with intermediate **40-1.** ESI-MS: m/z = 573.3 $[M+H]^+$.

3) Preparation of compound 40

Compound **40** (20 mg) was obtained by following step 6 of Example 7, while replacing intermediate **7-5** with intermediate **40-2.** ESI-MS: m/z = 764.3 $[M+2H]^{2+}$.

**Example 41: Preparation of Compound 41**

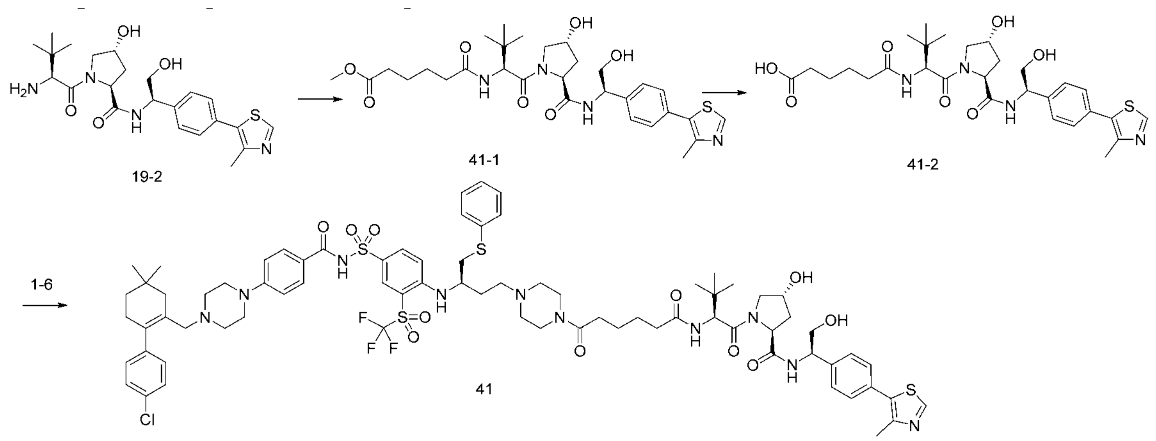


1) Preparation of intermediate 41-1

Intermediate **41-1** (160 mg) was obtained by following step 4 of Example 7, while replacing intermediate 7-3 with intermediate **19-2** and replacing monomethyl pimelate with monomethyl adipate. ESI-MS: m/z = 603.3 $[M+H]^+$.

2) Preparation of intermediate 41-2

Intermediate **41-2** (68 mg) was obtained by following step 5 of Example 7, while replacing intermediate **7-4** with intermediate **41-1.** ESI-MS: m/z = 589.3 $[M+H]^+$.

3) Preparation of compound 41

Compound **41** (15 mg) was obtained by following step 6 of Example 7, while replacing intermediate 7-5 with intermediate **41-2.** ESI-MS: m/z = 772.3 $[M+2H]^{2+}$.

**Example 42: Preparation of Compound 42**

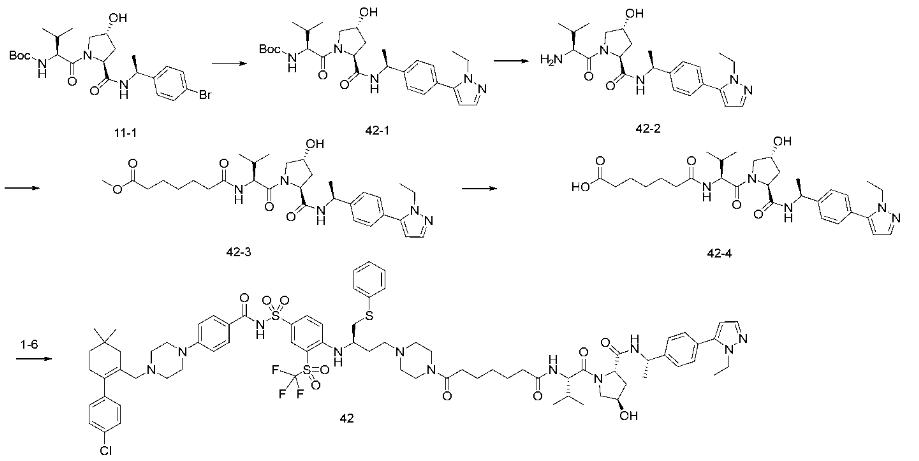


11-1 42-1 42-2 42-3 42-4 42

1) Preparation of intermediate 42-1

Intermediate **42-1** (1 g) was obtained by following step 2 of Example 11, while replacing 1-methyl-1*H*-pyrazole-5-boronic acid pinacol ester with 1-ethyl-5-(4,4,5,5-tetramethyl-1,3,2-dioxaborolan-2-yl)-1*H*-pyrazole. ESI-MS: m/z = 528.3 $[M+H]^+$.

2) Preparation of intermediate 42-2

Intermediate **42-2** (0.8 g) was obtained by following step 3 of Example 7, while replacing intermediate **7-2** with intermediate **42-1.** ESI-MS: m/z = 428.2 $[M+H]^+$.

3) Preparation of intermediate 42-3

Intermediate **42-3** (120 mg) was obtained by following step 4 of Example 7, while replacing intermediate **7-3** with intermediate **42-2.** ESI-MS: m/z = 584.3 $[M+H]^+$.

4) Preparation of intermediate 42-4

Intermediate **42-4** (70 mg) was obtained by following step 5 of Example 7, while replacing intermediate **7-4** with intermediate **42-3.** ESI-MS: m/z = 570.3 $[M+H]^+$.

5) Preparation of compound 42

Compound **42** (35 mg) was obtained by following step 6 of Example 7, while replacing intermediate **7-5** with intermediate **42-4.** ESI-MS: m/z = 762.7 $[M+2H]^{2+}$.

$^1H$ NMR (500 MHz, DMSO) δ 8.35 (s, 1H), 8.13 (s, 2H), 7.95 (s, 1H), 7.77 (m, 3H), 7.51 - 6.81 (m, 16H), 6.35 (s, 1H), 5.10 (s, 1H), 4.89 (s, 1H), 4.72 (s, 1H), 4.53 - 4.44 (m, 2H), 4.29 (s, 2H), 4.10 (s, 2H), 3.86 (s, 3H), 3.65 (s, 5H), 3.25 - 3.08 (s, 7H), 2.90 (s, 2H), 2.61 - 1.70 (m, 21H), 1.35 (m, 14H), 0.95 (s, 12H).

**Example 43: Preparation of Compound 43**

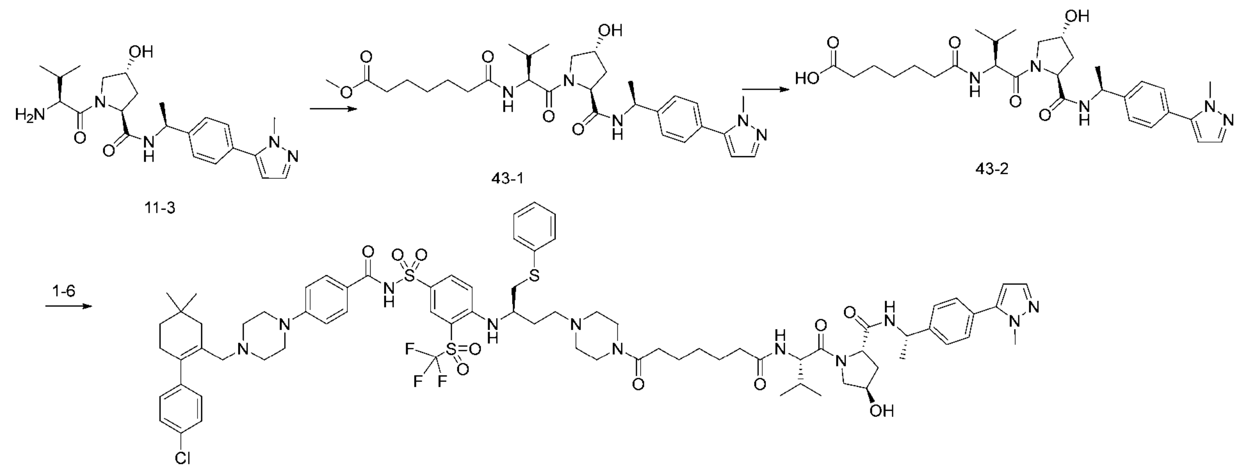


11-3 43-1 43-2

43

1) Preparation of intermediate 43-1

Intermediate **43-1** (150 mg) was obtained by following step 4 of Example 7, while replacing intermediate **7-3** with intermediate **11-3.** ESI-MS: m/z = 570.3 $[M+H]^+$.

2) Preparation of intermediate 43-2

Intermediate **43-2** (90 mg) was obtained by following step 5 of Example 7, while replacing intermediate **7-4** with intermediate **43-1.** ESI-MS: m/z = 556.3 $[M+H]^+$.

3) Preparation of compound 43

Compound **43** (30 mg) was obtained by following step 6 of Example 7, while replacing intermediate **7-5** with intermediate **43-2.** ESI-MS: m/z = 755.8 $[M+2H]^{2+}$.

$^1$H NMR (500 MHz, DMSO) δ 8.36 (s, 1H), 8.14 (s, 2H), 7.95 (s, 1H), 7.77 (m, 3H), 7.51 - 6.81 (m, 16H), 6.35 (s, 1H), 5.11 (s, 1H), 4.90 (s, 1H), 4.72 (s, 1H), 4.53 - 4.44 (m, 2H), 4.29 (s, 2H), 4.10 (s, 2H), 3.86 (s, 3H), 3.65 (s, 5H), 3.26 - 3.09 (s, 7H), 2.91 (s, 2H), 2.61 - 1.70 (m, 21H), 1.35 (m, 12H), 0.95 (s, 12H).

**Example 44: Preparation of Compound 44**

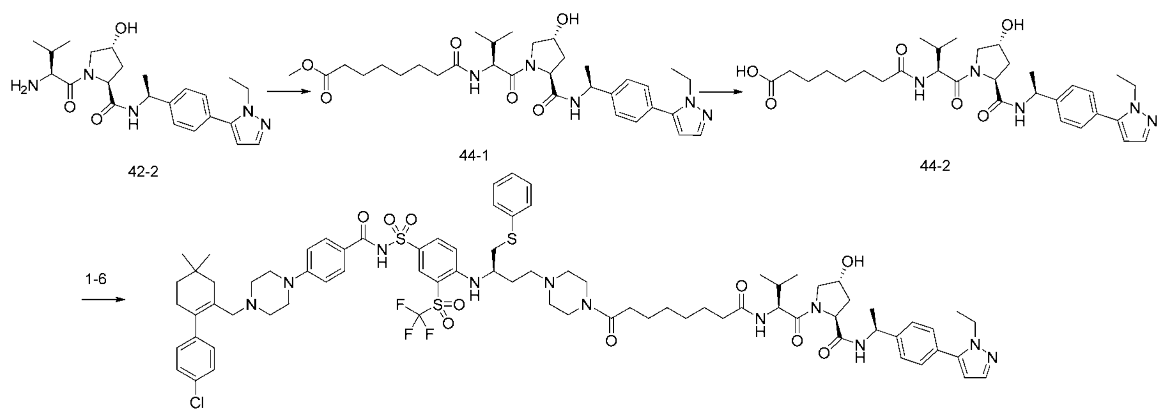


42-2 44-1 44-2

44

1) Preparation of intermediate 44-1

Intermediate **44-1** (131 mg) was obtained by following step 4 of Example 7, while replacing intermediate **7-3** with intermediate **42-2** and replacing monomethyl pimelate with monomethyl suberate. ESI-MS: m/z = 598.3 $[M+H]^+$.

2) Preparation of intermediate 44-2

Intermediate **44-2** (80 mg) was obtained by following step 5 of Example 7, while replacing intermediate **7-4** with intermediate **44-1.** ESI-MS: m/z = 584.3 $[M+H]^+$.

3) Preparation of compound 44

Compound **44** (25 mg) was obtained by following step 6 of Example 7, while replacing intermediate **7-5** with intermediate **44-2.** ESI-MS: m/z = 769.8 $[M+2H]^{2+}$.

$^1$H NMR (500 MHz, DMSO) δ 8.35 (s, 1H), 8.13 (s, 2H), 7.95 (s, 1H), 7.77 (m, 3H), 7.51 - 6.82 (m, 16H), 6.34 (s, 1H), 5.10 (s, 1H), 4.89 (s, 1H), 4.72 (s, 1H), 4.55 - 4.44 (m, 2H), 4.29 (s, 2H), 4.10 (s, 2H), 3.86 (s, 3H), 3.65 (s, 5H), 3.25 - 3.08 (s, 7H), 2.88 (s, 2H), 2.61 - 1.71 (m, 21H), 1.35 (m, 16H), 0.95 (s, 12H).

**Example 45: Preparation of Compound 45**

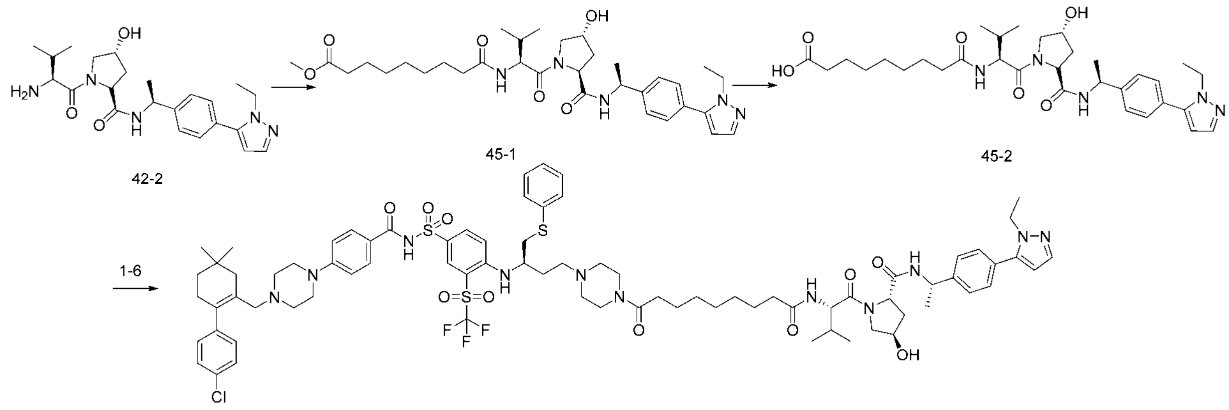


42-2 45-1 45-2 45

1) Preparation of intermediate 45-1

Intermediate **45-1** (108 mg) was obtained by following step 4 of Example 7, while replacing intermediate **7-3** with intermediate **42-2** and replacing monomethyl pimelate with monomethyl azelate. ESI-MS: m/z = 612.2 $[M+H]^+$.

2) Preparation of intermediate 45-2

Intermediate **45-2** (66 mg) was obtained by following step 5 of Example 7, while replacing intermediate **7-4** with intermediate **45-1.** ESI-MS: m/z = 598.3 $[M+H]^+$.

3) Preparation of compound 45

Compound **45** (8 mg) was obtained by following step 6 of Example 7, while replacing intermediate **7-5** with intermediate **45-2.** ESI-MS: m/z = 776.8 $[M+2H]^{2+}$.

$^1$H NMR (500 MHz, DMSO) δ 8.36 (s, 1H), 8.14 (s, 2H), 7.96 (s, 1H), 7.77 (m, 3H), 7.51 - 6.82 (m, 16H), 6.34 (s, 1H), 5.10 (s, 1H), 4.87 (s, 1H), 4.72 (s, 1H), 4.55 - 4.44 (m, 2H), 4.30 (s, 2H), 4.10 (s, 2H), 3.87 (s, 3H), 3.65 (s, 5H), 3.27 - 3.08 (s, 7H), 2.88 (s, 2H), 2.61 - 1.71 (m, 23H), 1.35 (m, 16H), 0.95 (s, 12H).

**Example 46: Preparation of Compound 46**

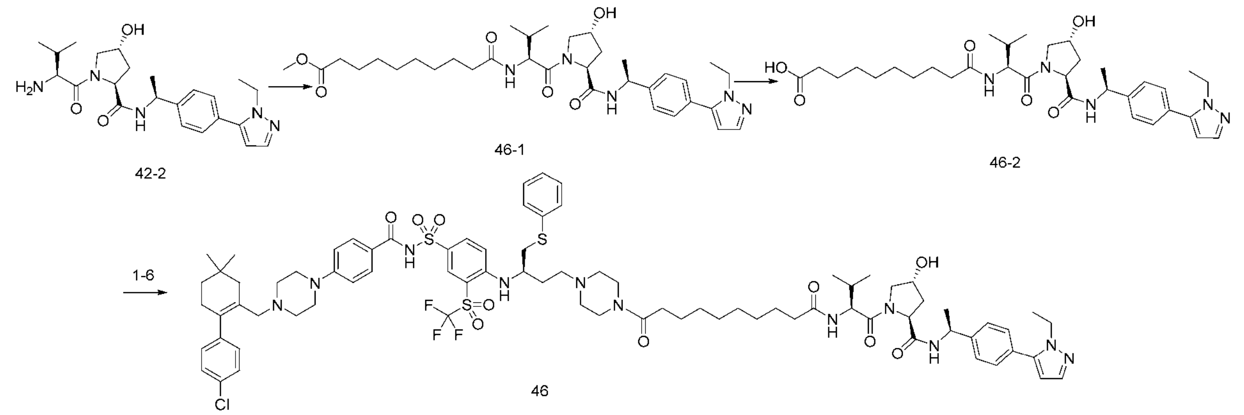


1) Preparation of intermediate 46-1

Intermediate **46-1** (100 mg) was obtained by following step 4 of Example 7, while replacing intermediate **7-3** with intermediate **42-2** and replacing monomethyl pimelate with monomethyl sebacate. ESI-MS: m/z = 626.3 $[M+H]^+$.

2) Preparation of intermediate 46-2

Intermediate **46-2** (70 mg) was obtained by following step 5 of Example 7, while replacing intermediate **7-4** with intermediate **46-1.** ESI-MS: m/z = 612.3 $[M+H]^+$.

3) Preparation of compound 46

Compound **46** (10 mg) was obtained by following step 6 of Example 7, while replacing intermediate **7-5** with intermediate **46-2.** ESI-MS: m/z = 783.7 $[M+2H]^{2+}$.

$^1$H NMR (500 MHz, DMSO) δ 8.35 (s, 1H), 8.14 (s, 2H), 7.95 (s, 1H), 7.77 (m, 3H), 7.51 - 6.82 (m, 16H), 6.34 (s, 1H), 5.10 (s, 1H), 4.87 (s, 1H), 4.73 (s, 1H), 4.55 - 4.45 (m, 2H), 4.30 (s, 2H), 4.10 (s, 2H), 3.87 (s, 3H), 3.65 (s, 5H), 3.27 - 3.08 (s, 7H), 2.88 (s, 2H), 2.61 - 1.71 (m, 23H), 1.35 (m, 18H), 0.95 (s, 12H).

**Example 47: Preparation of Compound 47**

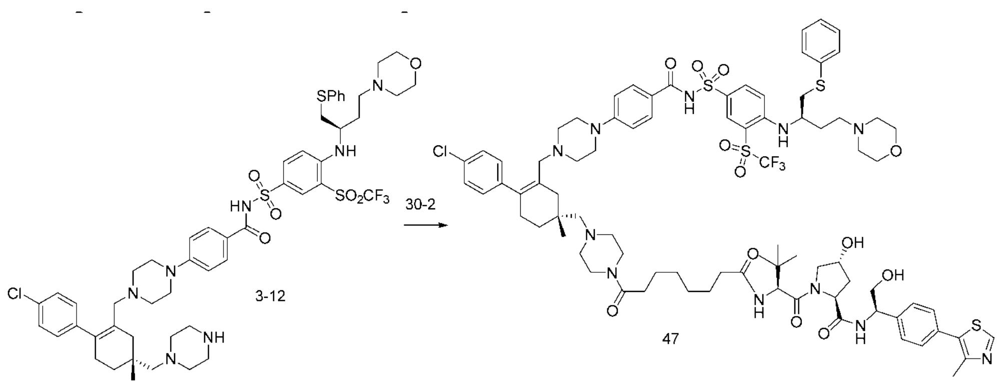


1) Preparation of compound 47

Intermediate **3-12** (121 mg) was dissolved in DMF, and intermediate **30-2** (70 mg), DIPEA (148 mg), and HATU (65 mg) were added. The mixture was reacted at room temperature for 3 h. The crude product from the reaction solution was purified by preparative liquid chromatography to give compound **47** (46 mg). ESI-MS: m/z = 828.1 $[M+2H]^{2+}$.

**Example 48: Preparation of Compound 48**

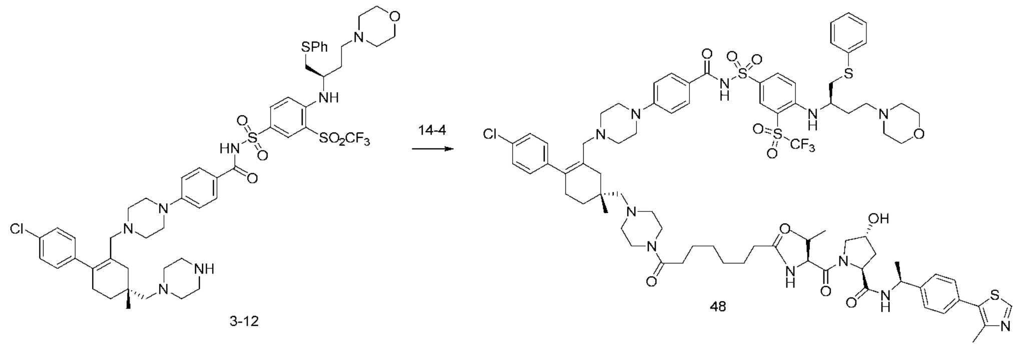


3-12

48

1) Preparation of compound 48

Compound **48** (8 mg) was obtained by following step 1 of Example 47, while replacing intermediate **30-2** with intermediate **14-4.**

ESI-MS: m/z = 813.8 $[M+2H]^{2+}$.

$^1$H NMR (500 MHz, DMSO) δ 11.95 (s, 1H), 8.98 (s, 1H), 8.35 (m, 1H), 8.12 (s, 1H), 7.92 (m, 2H), 7.71 (m, 2H), 7.43 (m, 2H), 7.39 - 7.32 (m, 5H), 7.27 (m, 2H), 7.15 (m, 3H), 6.98 (s, 1H), 6.85 (m, 2H), 5.07 (s, 1H), 4.90 (m, 1H), 4.49 - 4.21 (m, 3H), 4.07 (s, 1H), 3.61 (s, 3H), 3.53 (s, 2H), 3.22 (s, 6H), 2.45 (s, 4H), 2.25 (s, 11H), 1.99 (s, 6H), 1.91 (s, 6H), 1.79 (s, 3H), 1.57 (s, 1H), 1.46 (s, 9H), 1.37 (m, 2H), 1.24 (s, 8H), 0.95 (s, 4H), 0.91 - 0.81 (s, 9H).

**Example 49: Preparation of Compound 49**

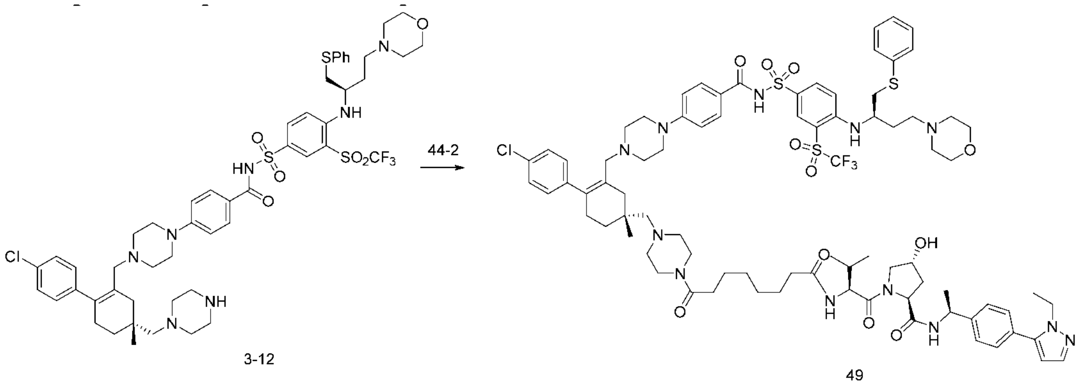


3-12

49

1) Preparation of compound 49

Compound **49** (10 mg) was obtained by following step 1 of Example 47, while replacing intermediate **30-2** with intermediate **44-2.**

ESI-MS: m/z = 812.2 $[M+2H]^{2+}$.

$^1$H NMR (500 MHz, DMSO) δ 11.92 (s, 1H), 8.36 (m, 1H), 8.12 (m, 1H), 7.97 - 7.88 (m, 2H), 7.72 (m, 2H), 7.48 (m, 1H), 7.39 (m, 4H), 7.33 (m, 2H), 7.27 (m, 2H), 7.18 (m, 1H), 7.12 (m, 2H), 6.97 (m, 1H), 6.85 (m, 2H), 6.31 (m, 1H), 5.08 (m, 1H), 4.93 (m, 1H), 4.42 (m, 1H), 4.38 - 4.26 (m, 2H), 4.11 (m, 3H), 3.61 (m, 2H), 3.53 (s, 4H), 3.43 (s, 4H), 3.21 (s, 4H), 2.47 (s, 3H), 2.35 (m, 4H), 2.25 (m, 5H), 2.20 - 2.06 (m, 5H), 2.04 - 1.93 (m, 5H), 1.91 (s, 6H), 1.79 (m, 2H), 1.61 - 1.53 (m, 1H), 1.47 (m, 7H), 1.38 (m, 3H), 1.30 (m, 4H), 1.27 - 1.20 (m, 7H), 0.95 (s, 3H), 0.86 (s, 6H).

**Example 50: Preparation of Compound 50**

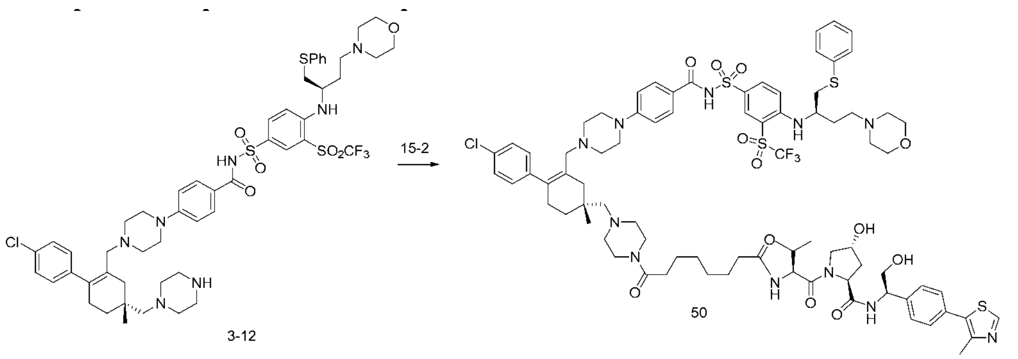


3-12

50

1) Preparation of compound 50

Compound **50** (5 mg) was obtained by following step 1 of Example 47, while replacing intermediate **30-2** with intermediate **15-2.** ESI-MS: m/z = 821.8 $[M+2H]^{2+}$.

**Experimental Example 1: Inhibitory Effect on Proliferation of RS4;11 Cells *In Vitro***

RS4; 11 cells (from Nanjing Cobioer) in logarithmic growth phase and good cell condition were collected into a centrifuge tube and centrifuged at 1000 rpm for 5 min in a low-speed benchtop centrifuge. The supernatant was discarded, and 3 mL of a plating medium (RPMI basic medium + 5% fetal bovine serum) was added using a pipette for resuspension. The cell suspension was loaded on a cell counter for counting and diluted with the plating medium to adjust the cell density to $1 \times 10^5$ cells/mL. The cells were seeded on a 96-well plate at 100 μL/well using a multi-channel pipette and cultured in a cell incubator at 37 °C with 5% $CO_2$ and saturated humidity. After 24 h of culture, compounds were loaded using a nanoliter pipettor such that the final concentrations of the compounds were 2000 nM-0.91 nM; the wells were set in duplicate, and meanwhile, a control was set. After another 72 h of culture in the cell incubator, an assay reagent CCK-8 (manufacturer: Dojindo Laboratories, Japan) was added at 10 μL/well. After 4 h of incubation in the cell incubator, the absorbance was measured at 450 nm on an Envision microplate reader, and the inhibition rate was calculated: inhibition rate (%) = (mean value of negative control group - mean value of experimental group)/(mean value of negative control group - mean value of blank group) × 100%. A dose-response curve was fitted by four-parameter analysis, with the logarithm of compound concentration serving as abscissa and inhibition rate serving as ordinate, so that $IC_{50}$ was calculated.

The experimental results show that the compounds of the present disclosure had an inhibitory effect on the proliferation of RS4;11 cells.

**Experimental Example 2: Inhibitory Effect on Proliferation of MOLT-4 Cells *In Vitro***

A dish of MOLT-4 cells in logarithmic growth phase and good cell condition was collected into a centrifuge tube and centrifuged at 1500 rpm for 3 min in a low-speed benchtop centrifuge. The supernatant was discarded, and 5 mL of a complete medium (RPMI basic medium + 10% FBS) was added using a pipette for cell resuspension. The cell suspension was loaded on a cell counter for counting and diluted with the complete medium to adjust the cell density to $1.6 \times 10^5$ cells/mL, and then an equivalent amount of the RPMI basic medium was added to adjust the serum concentration to 5% and the cell density to $8 \times 10^4$ cells/mL for plate seeding. The cells were seeded on a 96-well plate at 100 μL/well using a multi-channel pipette and cultured in a cell incubator at 37 °C with 5% $CO_2$ and saturated humidity. After 24 h of culture, compounds were loaded using a nanoliter pipettor such that the final concentrations of the compounds were 1000 nM-0.46 nM; 2 replicate wells were set for each concentration, and cells without compound addition were used as the negative control. After 72 h, CCK-8 (Dojindo Laboratories, Japan) was added at 10 μL/well for incubation for 3.5 h, then the absorbance was measured at 450 nm on an Envision microplate reader, and the inhibition rate was calculated: inhibition rate (%) = (mean value of negative control group - experimental group)/(mean value of negative control group - mean value of blank group) × 100%. A dose-response curve was fitted by four-parameter analysis, with the logarithm of compound concentration serving as abscissa and inhibition rate serving as ordinate, so that $IC_{50}$ was calculated. The results are shown in Table 1.

Table 1. Test results for inhibitory activity of compounds ($IC_{50}$, nM)

| **Compound No.** | Inhibitory activity against proliferation of MOLT-4 cells | **Compound No.** | Inhibitory activity against proliferation of MOLT-4 cells |
|---|---|---|---|
| **1** | ≤10 | **20** | ≤10 |
| **2** | ≤10 | **21** | ≤10 |
| **3** | ≤10 | **22** | ≤10 |
| **4** | ≤10 | **23** | ≤10 |
| **5** | ≤10 | **24** | ≤10 |
| **6** | ≤10 | **28** | ≤10 |
| **11** | ≤10 | **30** | ≤10 |
| **14** | ≤10 | **32** | ≤10 |
| **15** | ≤10 | **47** | ≤10 |
| **16** | ≤10 | **48** | ≤10 |
| **17** | ≤10 | **49** | ≤10 |
| **19** | ≤10 | **50** | ≤10 |

The experimental results show that the compounds of the present disclosure had an inhibitory effect on the proliferation of RS4;11 cells and MOLT-4 cells *in vitro.*

**Experimental Example 3: Assay on Degradation of BCL-XL Protein in MOLT-4 Cells *In Vitro***

A dish of MOLT-4 cells in logarithmic growth phase and good cell condition was collected into a centrifuge tube and centrifuged at 1500 rpm for 3 min in a low-speed benchtop centrifuge. The supernatant was discarded, and 5 mL of a complete medium (RPMI basic medium + 10% FBS) was added using a pipette for cell resuspension. The cell suspension was loaded on a cell counter for counting and diluted with the complete medium to adjust the cell density to $1 \times 10^7$ cells/mL. The cells were seeded on a 96-well plate at 100 μL/well using a multi-channel pipette. Compounds were loaded using a nanoliter pipettor such that the final concentrations of the compounds were 1000 nM-1 nM; 2 replicate wells were set for each concentration. The cells were cultured in a cell incubator at 37 °C with 5% $CO_2$ and saturated humidity.

After 24 h of culture, the cells were collected for flow cytometry. After the cells were washed with PBS containing 2% BSA, they were firstly fixed with 80% methanol, then permeabilized with PBS containing 0.1% Tween 20, subsequently blocked with PBS containing 10% BSA, and finally subjected to antibody labeling. The cells were incubated with a primary antibody BCL-XL (54H6) Rabbit mAb (CST, 2764S) at room temperature for 0.5 h, washed with PBS containing 2% BSA, and then incubated with Anti-rabbit IgG (H+L), F(ab')2 Fragment (Alexa Fluor® 488 Conjugate) (CST, 4412S) at room temperature for 0.5 h. After the incubation was completed, the cells were washed and resuspended with PBS containing 2% BSA, and then the cell suspension was loaded on IQue3 (Sartorius) for assay.

The group labeled only with the secondary antibody was used as a background group, and cells without compound addition were used as a negative control group. The expression of BCL-XL protein was measured using the mean fluorescence intensity (MFI) index to calculate the inhibition rate. Degradation rate (%) = (MFI mean value of negative control group - MFI of compound group)/(MFI mean value of negative control group - MFI mean value of background group) $\times$ 100%. A dose-response curve was fitted by four-parameter analysis, with the logarithm of compound concentration serving as abscissa and degradation rate serving as ordinate, so that $DC_{50}$ (half maximal degradation concentration) was calculated.

The experimental results show that the compounds of the present disclosure had the effect of degrading BCL-XL protein in MOLT-4 cells *in vitro.*

**Experimental Example 4: Evaluation of Inhibitory Activity Against BCL-XL/BAK Binding**

A Tag1-BCL-XL protein stock solution and a Tag2-BAK protein stock solution were diluted to 8 nM and 20 nM respectively with a dilution buffer in a kit (cisbio, 63ADK000CB04PEG). The Tag1-BCL-XL protein dilution was added to a 384-well plate at 5 μL/well. Compounds were loaded using a nanoliter pipettor such that the final concentrations of the compounds were 1000 nM-0.24 nM; the wells were set in duplicate, and meanwhile, a control was set. The Tag2-BAK protein dilution was added at 5 μL/well. The mixture was well mixed by centrifugation and incubated at room temperature

for 15 min. An Anti-Tag1-$Eu^{3+}$ antibody and an Anti-Tag2-XL665 antibody were diluted to 1X concentration with an assay buffer in a kit (cisbio, 63ADK000CB04PEG). The Anti-Tag1-$Eu^{3+}$ and Anti-Tag2-XL665 antibody dilutions were well mixed in a 1:1 volume ratio, and the resulting antibody mixed solution was added at 10 μL/well. The resulting mixture was mixed well by centrifugation and incubated at room temperature for 2 h. Fluorescence values were measured at 665 nm/620 nm on an Envision microplate reader. A dose-response curve was fitted by four-parameter analysis, so that $IC_{50}$ was calculated.

The experimental results show that the compounds of the present disclosure had an inhibitory activity against BCL-XL/BAK binding *in vitro.*

**Experimental Example 5: *In Vitro* Stability in Liver Microsomes**

Liver microsome incubation samples (species: human, rat, mouse, and monkey) were each prepared by mixing a PBS buffer (pH 7.4), a liver microsome solution (0.5 mg/mL), a test compound, and an NADPH + $MgCl_2$ solution and incubated at 37 °C and 300 rpm for 1 h. Zero-hour samples were prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted, and then assayed by LC/MS/MS.

The experimental results show that the compounds of the present disclosure exhibited *in vitro* metabolic stability in liver microsomes. The results are shown in Tables 2 and 3.

Table 2

| Compound No. | Remaining amount% (T = 60 min) (0.5 mg/mL) | Compound No. | Remaining amount% (T = 60 min) (0.5 mg/mL) |
|---|---|---|---|
| | Human liver microsome | | Human liver microsome |
| 1 | ≥85% | 28 | ≥85% |
| 2 | ≥85% | 31 | ≥85% |
| 4 | ≥85% | 32 | ≥85% |
| 5 | ≥85% | | |

Table 3

| Compound No. | Remaining amount% (T = 60 min) (0.5 mg/mL) | Compound No. | Remaining amount% (T = 60 min) (0.5 mg/mL) |
|---|---|---|---|
| | Mouse liver microsome | | Mouse liver microsome |
| **25** | ≥85% | **32** | ≥85% |
| **27** | ≥85% | **44** | ≥85% |
| **28** | ≥85% | **45** | ≥85% |
| **30** | ≥85% | | |

**Experimental Example 6: *In Vivo* Pharmacokinetics in Mice**

ICR mice weighing 18-22 g were randomized into groups of 9 after 3-5 days of acclimatization and then intravenously injected with a test compound solution at a dose of 1 mg/kg. Plasma samples to be tested were prepared by taking blood from the orbit at time points of 5 min, 15 min, 30 min, 1 h, 2 h, 3 h, 4 h, 6 h, 8 h, 10 h, and 24 h. Twenty microliters of each plasma sample to be tested and a standard curve sample were taken, and an acetonitrile solution containing an internal standard was added. Supernatants were obtained by protein precipitation, diluted, and then assayed by LC/MS/MS. Pharmacokinetic parameters were fitted using a non-compartmental model.

The experimental results show that the compounds of the present disclosure had good pharmacokinetic parameters *in vivo.*

**Experimental Example** 7: *In* ***Vivo*** **Pharmacokinetics in Rats**

SD rats weighing 210-230 g were randomized into groups of 3 after 3-5 days of acclimatization and then intravenously injected with an example solution at a dose of 0.5 mg/kg.

Plasma samples to be tested were prepared by taking blood from the orbit at time points of 0 min, 15 min, 30 min, 1 h, 2 h, 4 h, 6 h, 8 h, 10 h, 24 h, and 32 h.

Fifty microliters of each plasma sample to be tested and a standard curve sample were taken, and an acetonitrile solution containing an internal standard was added. Supernatants were obtained by protein precipitation, diluted, and then assayed by LC/MS/MS.

Data were fitted using a non-compartmental model.

The experimental results show that the compounds of the present disclosure had good pharmacokinetic parameters in rats.

**Experimental Example 8: *In Vivo* Pharmacokinetics in Dogs**

Beagle dogs weighing 10-12 kg were randomized into groups of 3 after 3-5 days of acclimatization and then intravenously injected with an example solution at a dose of 0.2 mg/kg.

Plasma samples to be tested were prepared by taking blood from the forelimb vein at time points of 0 min, 15 min, 30 min, 1 h, 2 h, 4 h, 6 h, 8 h, 10 h, 24 h, 32 h, and 48 h.

Fifty microliters of each plasma sample to be tested and a standard curve sample were taken, and an acetonitrile solution containing an internal standard was added. Supernatants were obtained by protein precipitation, diluted, and then assayed by LC/MS/MS.

Data were fitted using a non-compartmental model.

The experimental results show that the compounds of the present disclosure had good pharmacokinetic parameters in dogs.

**Experimental Example 9: Assay on Kinetics of BCL-XL Protein Degradation**

A dish of MOLT-4 cells in logarithmic growth phase and good cell condition was collected into a centrifuge tube and centrifuged at 1500 rpm for 3 min in a low-speed benchtop centrifuge. The supernatant was discarded, and 5 mL of a complete medium (RPMI basic medium + 10% FBS) was added using a pipette for cell resuspension. The cell suspension was loaded on a cell counter for counting and diluted with the complete medium to adjust the cell density to $2 \times 10^6$ cells/mL. The cells were seeded on a 96-well V-bottom plate at 100 μL/well using a multi-channel pipette. Compounds were loaded using a nanoliter pipettor such that the final concentrations of the compounds were 100 nM; 2 replicate wells were set for this concentration. The cells were cultured in a cell incubator at 37 °C with 5% $CO_2$ and saturated humidity.

After 4 h of culture, the cells were collected by centrifugation at 300 g for 5 min and then washed with ice-cold PBS. After the washing, 100 μL of a lysis buffer was added to each well. The mixture was well mixed by using a pipette to ensure complete cell lysis; there should be no visible cell pellet after complete lysis. Lysis was then performed on ice for 30 min. After the lysis, the lysate was stored in a freezer at -80 °C for later detection.

The cell lysate was thawed on ice, then centrifuged at 500 g for 10 min, and assayed using a BCLXL ELISA kit (R&D, DYC894-5). Firstly, a high-binding 96-well plate was coated with a BCLXL capture antibody overnight and then blocked with PBS containing 1% BSA. Subsequently, the sample was incubated for 2 h. After the incubation, a detection antibody was added for incubation for 2 h. Finally, streptavidin-HRP was added for incubation for 20 min, TMB was added for color development for 15 min, and then an assay was performed. The absorbance was measured at 450/570 nm using an Envision microplate reader. The absorbance at 570 nm and the blank background value were subtracted, and the degradation rate was calculated: degradation rate (%) = 100% × (mean value of negative control group - experimental group)/mean value of negative control group. The results are shown in Table 4.

Table 4

| Compound No. | Degradation rate (%) | Compound No. | Degradation rate (%) |
|---|---|---|---|
| 1 | ≥40% | 5 | ≥40% |
| 2 | ≥40% | 48 | ≥40% |
| 3 | ≥40% | 49 | ≥40% |
| 4 | ≥40% | | |

The experimental results show that the compounds of the present disclosure exhibited good BCL-XL protein degradation kinetics.

**Experimental Example 10: Evaluation of Toxicity in Dog Platelets**

Nine milliliters of dog whole blood was extracted using a 10 mL sodium citrate vacuum anticoagulation tube, and the mixture was well mixed by turning the tube upside down and then centrifuged at 100 g and room temperature for 10 min. The supernatant (i.e., plasma) was collected and gently transferred into a 50 mL centrifuge tube, and then 5 mL of an acid citrate buffer working solution was added. The resulting mixture was well mixed gently, and 200 μL of the mixture was taken for counting, then centrifuged at 1200 g and room temperature for another 10 min.

(Note: Both acceleration and deceleration speeds of the centrifuge were set to 5). The supernatant was gently discarded, and then 2 mL of a Tyrode's Solution working solution containing 1 μM prostaglandin E1 (PGE1) and 0.2 units/mL apyrase was added for gentle washing. The supernatant was discarded, and the residue was gently resuspended and well mixed in a Tyrode's Solution working solution containing 1 μM prostaglandin E1 (PGE1) and 0.2 units/mL apyrase such that the final density was $1 \times 10^8$ platelets/mL. The suspension was then seeded on a 96-well U-bottom plate at 90 μL/well. After plating, an additional 10 μL of FBS was added to each well.

Compounds were loaded using a nanoliter pipettor such that the final concentrations of the compounds were 2000 nM-8.2 nM; 2 replicate wells were set for each concentration. The plate was then sealed with a plate-sealing film, placed on a constant-temperature microplate shaker, and incubated at room temperature (20 °C) with shaking at 300 rpm. After 72 h, an assay reagent CCK-8 (manufacturer: Dojindo Laboratories, Beijing; 10 μL/well) was added. After 1 h of incubation in a cell incubator, 50 μL of the mixture was transferred to a 96-well flat-bottom plate using a multi-channel pipette and well mixed by shaking, and then the absorbance was measured at 450 nm on a PerkinElmer Envision microplate reader. A dose-response curve was fitted by four-parameter analysis, so that the $IC_{50}$ values were calculated. The results are shown in Table 5.

Table 5

| Compound No. | Dog platelet $IC_{50}$ (nM) | Compound No. | Dog platelet $IC_{50}$ (nM) |
|---|---|---|---|
| 1 | ≥300 | 27 | ≥300 |
| 2 | ≥300 | 32 | ≥300 |
| 3 | ≥300 | 48 | ≥300 |
| 4 | ≥300 | 49 | ≥300 |
| 5 | ≥300 | | |

The experimental results show that the compounds of the present disclosure had relatively low toxicity in dog platelets.

**Experimental Example 11: Evaluation of Pharmacodynamics in CB17-SCID Mouse Subcutaneous Xenograft Tumor Model of MOLT-4 Human Acute Lymphoblastic Leukemia Cells**

SPF-grade female CB17-SCID mice (source: Shanghai Lingchang Biotech Co., Ltd.) were subcutaneously inoculated at the right axilla with MOLT-4 cells at $1 \times 10^7$ cells/mouse. When the mean tumor volume reached about 200 $mm^3$, the animals were grouped.

The day of grouping was defined as day 0. Administration was performed once by tail vein injection on day 0. The tumor volume was measured twice to thrice every week, and meanwhile, the mice were weighed and the data were recorded. The general behavior of the mice was observed and recorded every day. After the experiment was completed, the tumors were harvested, weighed, and photographed.

The detection indexes and the calculation formulas are as follows:

$$\text{Tumor volume TV } (mm^3) = 1/2 \times (a \times b^2),$$

where a represents the long diameter of the tumor, and b represents the short diameter of the tumor.

$$\text{Relative tumor volume RTV} = TV_t/TV_0,$$

where $TV_0$ represents the tumor volume on day 0, and $TV_t$ represents the tumor volume at each measurement.

$$\text{Relative tumor proliferation rate T/C (\%)} = (T_{RTV}/C_{RTV}) \times 100\%,$$

where $T_{RTV}$ represents RTV of the treatment group, and $C_{RTV}$ represents RTV of the vehicle control group.

$$\text{Tumor growth inhibition rate TGI (\%)} = (1 - TW/TW_0) \times 100\%,$$

where TW represents the tumor weight of the treatment group, and $TW_0$ represents the tumor weight of the vehicle control group.

$$\text{Weight change rate WCR (\%)} = (Wt_t - Wt_0)/Wt_0 \times 100\%,$$

where $Wt_0$ represents the body weight of the mouse on day 0, and $Wt_t$ represents the body weight of the mouse at each measurement.

The experimental results show that the compounds of the present disclosure had a good inhibitory effect on tumor growth *in vivo* (e.g., on day 14, the tumor volume inhibition rate was > 50% and the tumor weight inhibition rate was > 50%).

**Claims**

1. A compound of formula I, a stereoisomer thereof, or a pharmaceutically acceptable salt thereof:

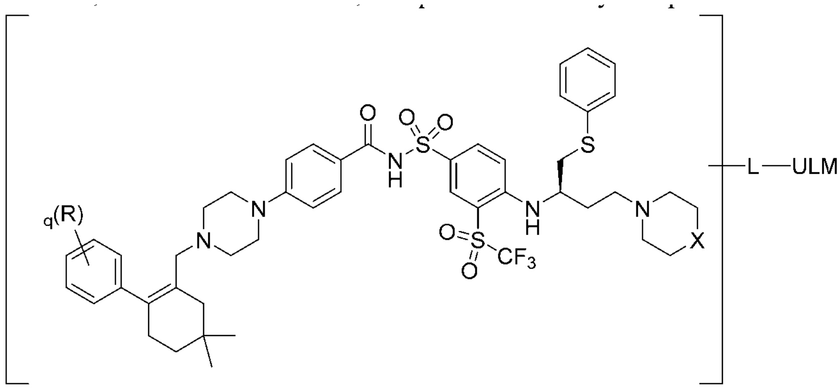

formula I

wherein

R is selected from the group consisting of OH, $NH_2$, CN, halogen, and $C_{1-6}$ alkyl optionally substituted with one or more OH, $NH_2$, CN, or halogens;
X is selected from the group consisting of $CH_2$, NH, and O;
L is a connecting group;
ULM is

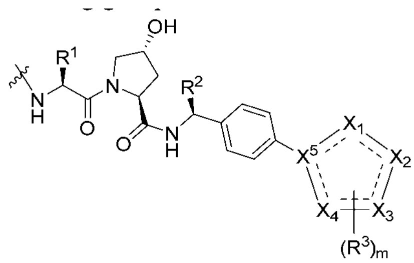

;

wherein $R^1$ is selected from $C_{1-6}$ alkyl optionally substituted with one or more halogens;
$R^2$ is selected from $C_{1-6}$ alkyl optionally substituted with one or more OH, $NH_2$, CN, or halogens;
$X^1$, $X^2$, $X^3$, $X^4$, and $X^5$ are each independently selected from the group consisting of CH, C, N, NH, O, and S; each $R^3$ is independently selected from the group consisting of OH, $NH_2$, CN, halogen, $C_{1-6}$ alkyl, and $C_{1-6}$ alkoxy, wherein the $NH_2$, $C_{1-6}$ alkyl, or $C_{1-6}$ alkoxy is optionally substituted with one or more OH, $NH_2$, CN, or halogens; m and q are each independently selected from the group consisting of 0, 1, 2, and 3;

the proviso is that ULM is not

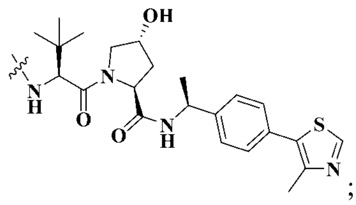

;

each R, X, L, $R^1$, $R^2$, $X^1$, $X^2$, $X^3$, $X^4$, or $X^5$ is independently optionally substituted with one or more substituents.

**2.** The compound, the stereoisomer thereof, or the pharmaceutically acceptable salt thereof according to claim 1, wherein

R is selected from the group consisting of halogen and $C_{1-6}$ alkyl optionally substituted with one or more OH, $NH_2$, CN, or halogens; or
R is selected from the group consisting of F, Cl, Br, and $C_{1-3}$ alkyl optionally substituted with one or more OH, $NH_2$, CN, or halogens; or
R is selected from the group consisting of F, Cl, Br, and $C_{1-3}$ alkyl optionally substituted with one or more F or Cl; or
R is selected from the group consisting of F, Cl, Br, and trifluoromethyl; or
R is selected from Cl; or
q is selected from the group consisting of 0, 1, and 2; or
q is selected from the group consisting of 0 and 1; or
the structural unit

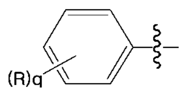

is selected from

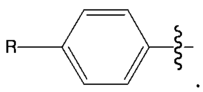

.

**3.** The compound, the stereoisomer thereof, or the pharmaceutically acceptable salt thereof according to claim 1 or 2, wherein X is selected from the group consisting of NH and O.

**4.** The compound, the stereoisomer thereof, or the pharmaceutically acceptable salt thereof according to any one of claims 1-3, wherein L is selected from the group consisting of a bond, $-C_{1-20}$ alkyl-, $-C_{2-20}$ alkenyl-, and $-C_{2-20}$ alkynyl-, wherein one or more $-CH_2-$ in the $-C_{1-20}$ alkyl-, $-C_{2-20}$ alkenyl-, or $-C_{2-20}$ alkynyl- are each independently optionally replaced by $R^x$; $R^x$ is selected from the group consisting of -O-, $-NR^a-$, $-S(O)_2-$, $-S(O)_2NR^a-$, -S(O)-, $-S(O)NR^a-$, -C(O)-, -C(O)O-, $-C(O)NR^a-$, $-C(O)N(R^a)O-$, -OC(O)-, $-OC(O)NR^a-$, $-N(R^a)C(O)O-$, $-N(R^a)C(O)-$, $-N(R^a)S(O)_2-$, 5- to 12-membered heteroaryl, phenyl, $C_{3-10}$ cycloalkyl, 3- to 10-membered heterocycloalkyl, and -S-, wherein $R^a$ is selected from the group consisting of hydrogen and $C_{1-6}$ alkyl; each $R^x$ or $R^a$ is independently optionally substituted with one or more substituents; or

$R^a$ is selected from the group consisting of hydrogen and $C_{1-4}$ alkyl; or $R^a$ is selected from the group consisting of hydrogen and $C_{1-3}$ alkyl; or $R^a$ is selected from the group consisting of hydrogen and $C_{1-2}$ alkyl; or $R^a$ is selected from the group consisting of hydrogen and methyl; or
$R^x$ is selected from the group consisting of -O-, -C(O)-, 5- to 12-membered heteroaryl, phenyl, $C_{3-10}$ cycloalkyl, 3- to 10-membered heterocycloalkyl, -NH-, $-N(C_{1-6}$ alkyl)-, and -S-; or
$R^x$ is selected from the group consisting of -O-, -C(O)-, 5- to 6-membered heteroaryl, phenyl, $C_{3-6}$ cycloalkyl, 3- to 6-membered heterocycloalkyl, -NH-, $-N(C_{1-6}$ alkyl)-, and -S-; or
$R^x$ is selected from the group consisting of -O-, -C(O)-, phenyl, $C_{5-6}$ cycloalkyl, 5- to 6-membered heterocycloalkyl, -NH-, $-N(C_{1-3}$ alkyl)-, and -S-; or
$R^x$ is selected from the group consisting of -O-, -C(O)-, phenyl, piperidinyl, piperazinyl, -NH-, $-N(C_{1-3}$ alkyl)-, and

-S-; or

$R^x$ is selected from the group consisting of -C(O)- and piperazinyl; or

$R^x$ is selected from the group consisting of -C(O)- and

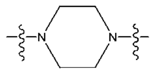

; or

L is selected from the group consisting of a bond, $-C_{1-12}$ alkyl-, $-C_{2-12}$ alkenyl-, and $-C_{2-12}$ alkynyl-, wherein one or more $-CH_2-$ in the $-C_{1-12}$ alkyl-, $-C_{2-12}$ alkenyl-, or $-C_{2-12}$ alkynyl- are each independently optionally replaced by $R^x$; $R^x$ is as defined above; or

L is selected from the group consisting of a bond, $-C_{3-12}$ alkyl-, $-C_{3-12}$ alkenyl-, and $-C_{3-12}$ alkynyl-, wherein one or more $-CH_2-$ in the $-C_{3-12}$ alkyl-, $-C_{3-12}$ alkenyl-, or $-C_{3-12}$ alkynyl- are each independently optionally replaced by $R^x$; $R^x$ is as defined above; or

L is selected from the group consisting of a bond, $-C_{6-12}$ alkyl-, $-C_{6-12}$ alkenyl-, and $-C_{6-12}$ alkynyl-, wherein one or more $-CH_2-$ in the $-C_{6-12}$ alkyl-, $-C_{6-12}$ alkenyl-, or $-C_{6-12}$ alkynyl- are each independently optionally replaced by $R^x$; $R^x$ is as defined above; or

L is selected from the group consisting of a bond, $-C_{8-9}$ alkyl-, $-C_{8-9}$ alkenyl-, and $-C_{8-9}$ alkynyl-, wherein one or more $-CH_2-$ in the $-C_{8-9}$ alkyl-, $-C_{8-9}$ alkenyl-, or $-C_{8-9}$ alkynyl- are each independently optionally replaced by $R^x$; $R^x$ is as defined above; or

L is selected from the group consisting of a bond, $-C_{6-12}$ alkyl-, $-C_{6-12}$ alkenyl-, and $-C_{6-12}$ alkynyl-, wherein one or more $-CH_2-$ in the $-C_{6-12}$ alkyl-, $-C_{6-12}$ alkenyl-, or $-C_{6-12}$ alkynyl- are each independently optionally replaced by $R^x$; $R^x$ is selected from the group consisting of -O-, -C(O)-, phenyl, piperidinyl, piperazinyl, -NH-, $-N(C_{1-3}$ alkyl)-, and -S-; or

L is selected from $-C_{3-12}$ alkyl-, wherein one or more $-CH_2-$ in the $-C_{3-12}$ alkyl- are each independently optionally replaced by $R^x$ $R^x$ is selected from the group consisting of -C(O)- and piperazinyl; or

L is selected from $-C_{6-12}$ alkyl-, wherein one or more $-CH_2-$ in the $-C_{6-12}$ alkyl- are each independently optionally replaced by $R^x$ $R^x$ is selected from the group consisting of -C(O)- and piperazinyl; or

L is selected from $-C_{6-10}$ alkyl-, wherein one or more $-CH_2-$ in the $-C_{6-10}$ alkyl- are each independently optionally replaced by $R^x$; $R^x$ is selected from the group consisting of -C(O)- and piperazinyl; or

L is selected from $-C_{6-10}$ alkyl-, wherein one or more $-CH_2-$ in the $-C_{6-10}$ alkyl- are each independently optionally replaced by $R^x$; $R^x$ is selected from the group consisting of -C(O)- and

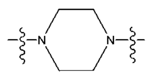

; or

L is selected from $-C_{8-10}$ alkyl-, wherein one or more $-CH_2-$ in the $-C_{8-10}$ alkyl- are each independently optionally replaced by -C(O)-; or

L is selected from the group consisting of

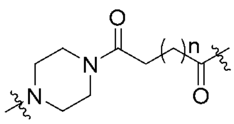, 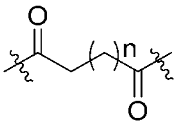, 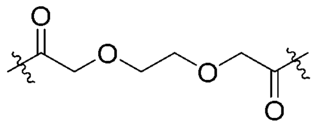,

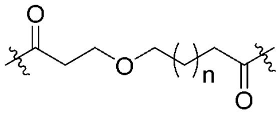, 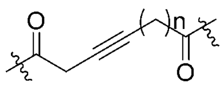, 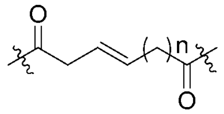, 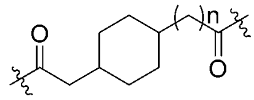,

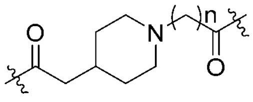 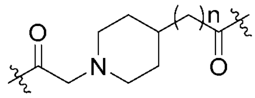 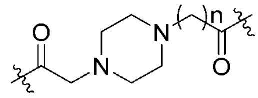 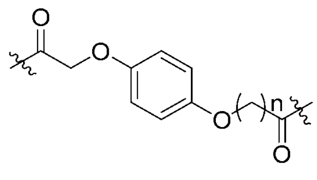

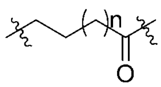, 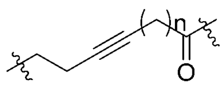, 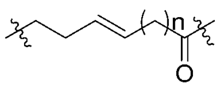, 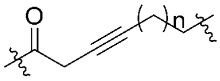, 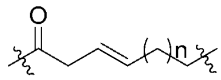,

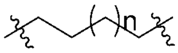, 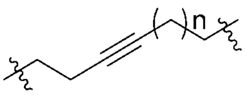, and 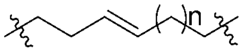,

wherein n is selected from the group consisting of 0-10, or n is selected from the group consisting of 1-9, or n is selected from the group consisting of 1-7; or

L is selected from the group consisting of

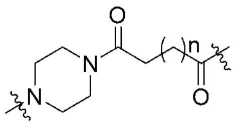, 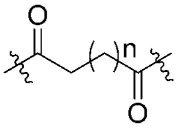, 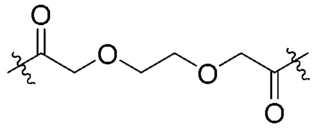,

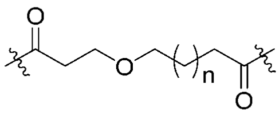, 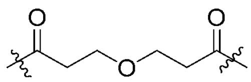, 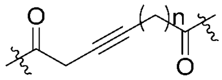, 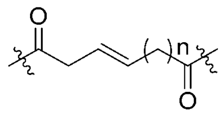,

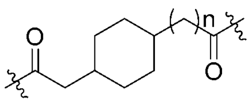, 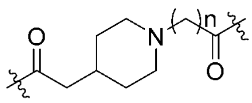, 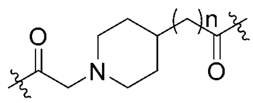, 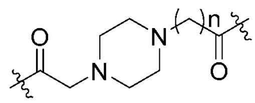,

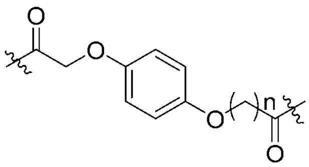, 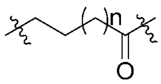, 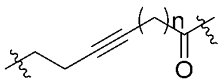, 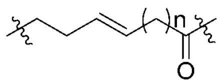, 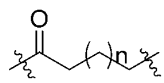,

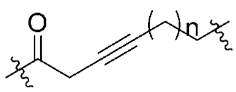, 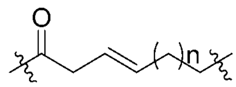, 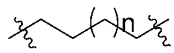, 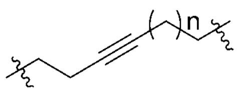,

and

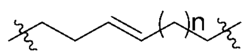,

, wherein n is selected from the group consisting of 0-10, or n is selected from the group consisting of 1-9, or n is selected from the group consisting of 1-7; or

L is selected from the group consisting of

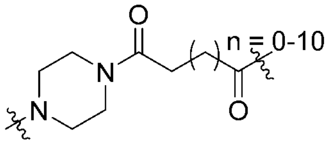, 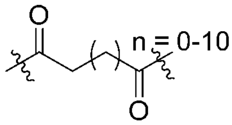,

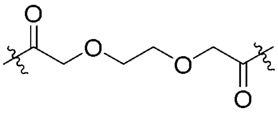, 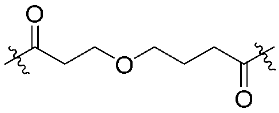, 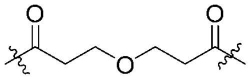, 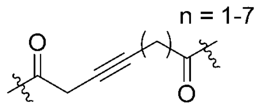,

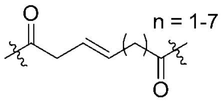, 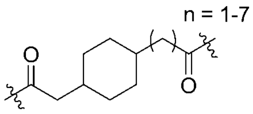, 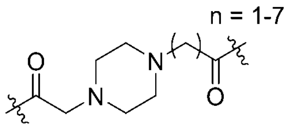, 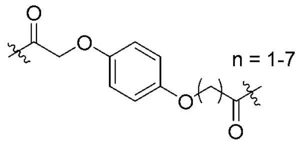,

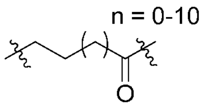, 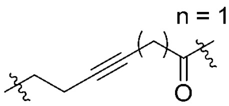, 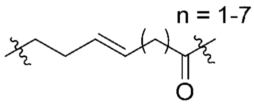, 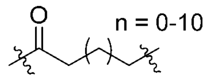,

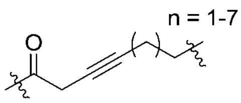, 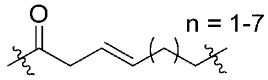, 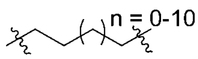, 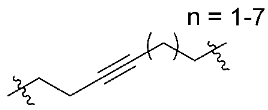,

and

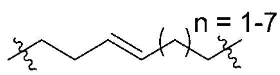;

or
L is selected from the group consisting of

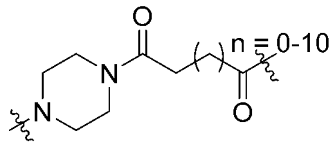 and 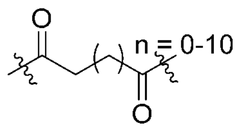;

or
L is selected from the group consisting of

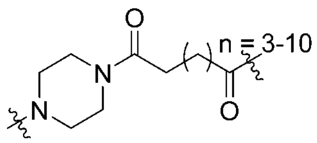 and 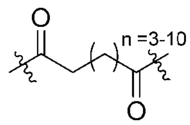;

or
L is selected from the group consisting of

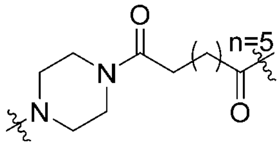, 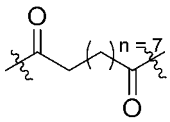, 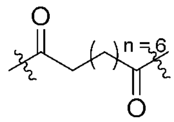,

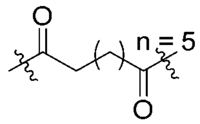, 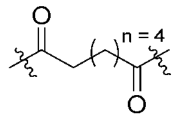, and 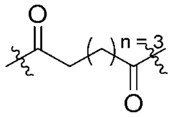;

or
L is selected from the group consisting of

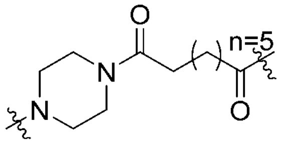, 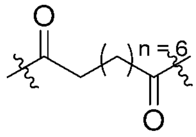, and 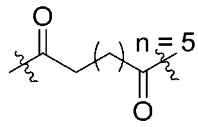;

or
L is selected from the group consisting of

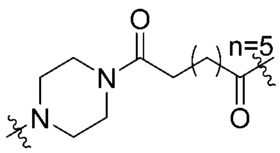 and 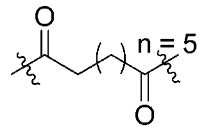.

5. The compound, the stereoisomer thereof, or the pharmaceutically acceptable salt thereof according to any one of claims 1-4, wherein one optional end of L is connected to ULM; or the right end of L is connected to ULM; or the left end of L is connected to ULM; or L is selected from the group consisting of

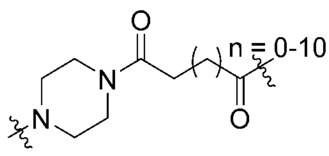

* and

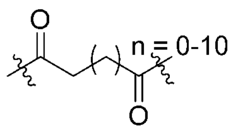

*, wherein * indicates that the end is connected to ULM.

6. The compound, the stereoisomer thereof, or the pharmaceutically acceptable salt thereof according to any one of claims 1-5, wherein $X^1$, $X^2$, $X^3$, $X^4$, and $X^5$ are each independently selected from the group consisting of CH, C, N, NH, O, and S; or

$X^1$, $X^2$, $X^3$, $X^4$, and $X^5$ are each independently selected from the group consisting of CH, C, N, NH, and S; or
$X^1$, $X^2$, $X^3$, $X^4$, and $X^5$ are each independently selected from the group consisting of CH, C, NH, and N; or
$X^1$, $X^2$, $X^3$, $X^4$, and $X^5$ are each independently selected from the group consisting of CH, C, N, NH, O, and S, and at least one of them is N; or
$X^1$, $X^2$, $X^3$, $X^4$, and $X^5$ are each independently selected from the group consisting of CH, C, N, NH, O, and S, and at least two of them are heteroatoms; or
$X^1$, $X^2$, $X^3$, $X^4$, and $X^5$ are each independently selected from the group consisting of CH, C, N, NH, O, and S, and at least two of them are selected from the group consisting of N and S; or
the ring formed by $X^1$, $X^2$, $X^3$, $X^4$, and $X^5$ is aromatic; or
the ring formed by $X^1$, $X^2$, $X^3$, $X^4$, and $X^5$ is a five-membered heteroaromatic ring; or

$X^1$ and $X^2$ are each independently selected from the group consisting of N and NH, $X^3$ and $X^4$ are each independently selected from CH, and $X^5$ is selected from C; or
$X^1$, $X^2$, and $X^4$ are selected from CH, and $X^3$ and $X^5$ are selected from the group consisting of N and NH; or
$X^1$, $X^2$, and $X^4$ are selected from CH, $X^3$ is selected from the group consisting of N and NH, and $X^5$ is selected from N; or
$X^1$ is selected from S, $X^2$ and $X^4$ are selected from CH, $X^3$ is selected from the group consisting of N and NH, and $X^5$ is selected from C; or
the structural unit

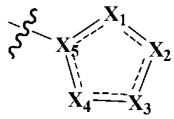

is selected from the group consisting of

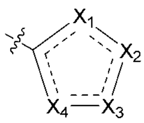 and 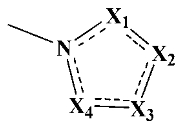 ;

or the structural unit

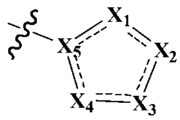

is selected from the group consisting of

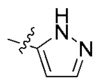 , 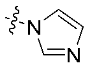 , and 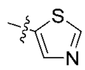 .

**7.** The compound, the stereoisomer thereof, or the pharmaceutically acceptable salt thereof according to any one of claims 1-6, wherein $R^1$ is selected from $C_{1-4}$ alkyl optionally substituted with one or more halogens; or $R^1$ is selected from $C_{3-4}$ alkyl optionally substituted with one or more halogens; or $R^1$ is selected from the group consisting of isopropyl and tert-butyl optionally substituted with one or more halogens; or $R^1$ is selected from the group consisting of isopropyl and tert-butyl; and/or
$R^2$ is selected from $C_{1-4}$ alkyl optionally substituted with one or more OH, $NH_2$, CN, or halogens; or $R^2$ is selected from methyl optionally substituted with one or more OH, $NH_2$, CN, or halogens; or $R^2$ is selected from methyl optionally substituted with one or more OH; or $R^2$ is selected from the group consisting of hydroxymethyl and methyl.

**8.** The compound, the stereoisomer thereof, or the pharmaceutically acceptable salt thereof according to any one of claims 1-7, wherein each $R^3$ is independently selected from the group consisting of OH, $NH_2$, CN, halogen, $C_{1-3}$ alkyl, and $C_{1-3}$ alkoxy, wherein the $NH_2$, $C_{1-3}$ alkyl, or $C_{1-3}$ alkoxy is optionally substituted with one or more OH, $NH_2$, CN, or halogens; or each $R^3$ is independently selected from the group consisting of $C_{1-3}$ alkyl and $C_{1-3}$ alkoxy; or each $R^3$ is independently selected from the group consisting of OH, $NH_2$, CN, halogen, methyl, and ethyl; or each $R^3$ is independently selected from the group consisting of methyl and ethyl; or

the structural unit

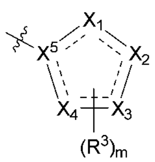

is selected from the group consisting of

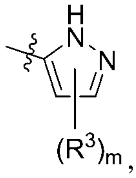, 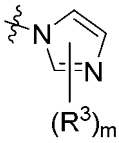, and 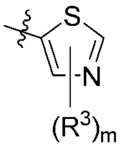;

or
the structural unit

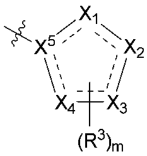

is selected from the group consisting of

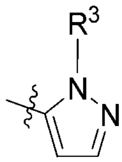, 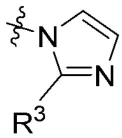, and 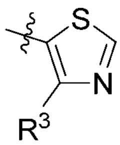;

or
the structural unit

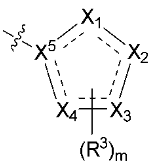

is selected from the group consisting of

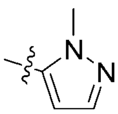, 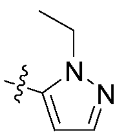, 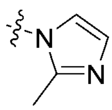,

and

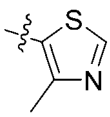;

or
the structural unit

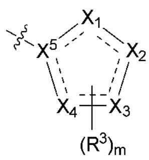

is selected from the group consisting of

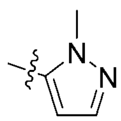 and 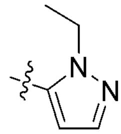 .

**9.** The comnound, the stereoisomer thereof, or the pharmaceutically acceptable salt thereof according to any one of claims 1-8, wherein ULM is

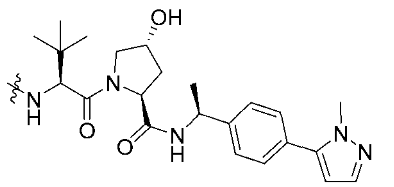 , 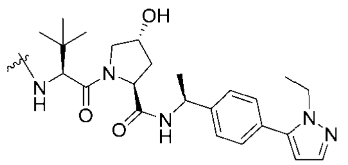 ,

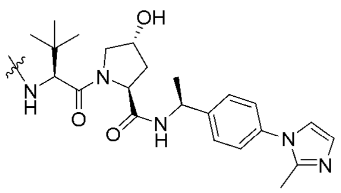 , 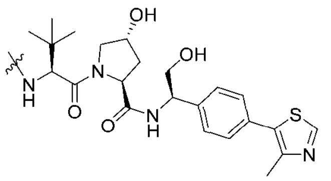 , 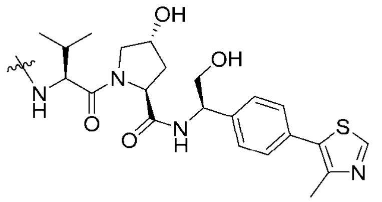 ,

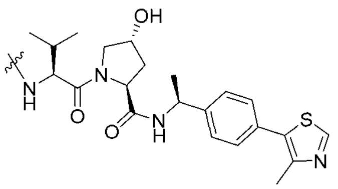 , 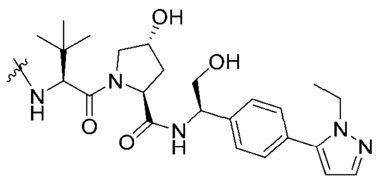 , 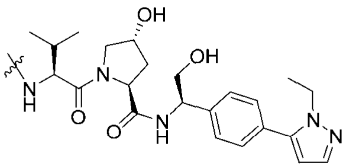 ,

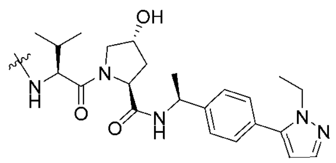 , 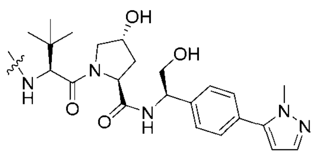 , 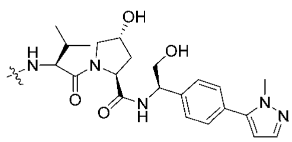 ,

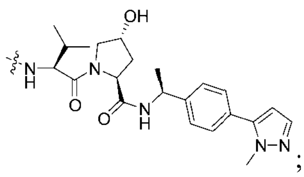 ;

or
ULM is

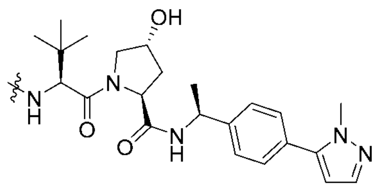,
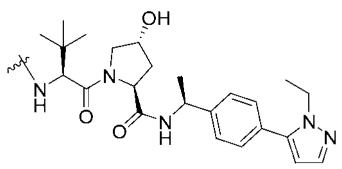,
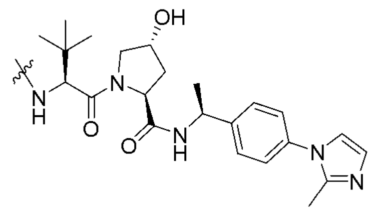,

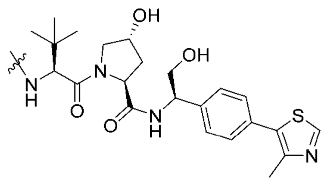,
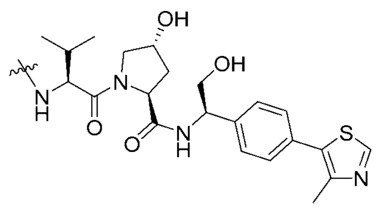,
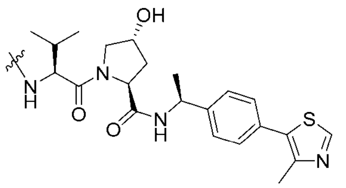,

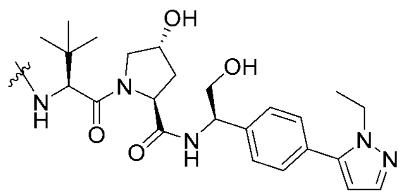,
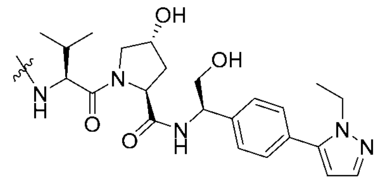, or
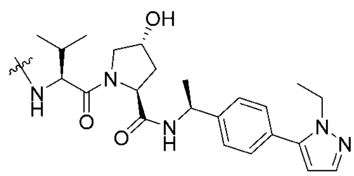.

**10.** The compound, the stereoisomer thereof, or the pharmaceutically acceptable salt thereof according to any one of claims 1-9, wherein m and q are each independently selected from 1.

**11.** The compound, the stereoisomer thereof, or the pharmaceutically acceptable salt thereof according to claim 1, which is selected from the group consisting of compounds of formula II, formula III, formula II-A, formula III-A, formula III-B, formula IV, formula V, formula VI and formula VII, stereoisomers thereof, or pharmaceutically acceptable salts thereof:

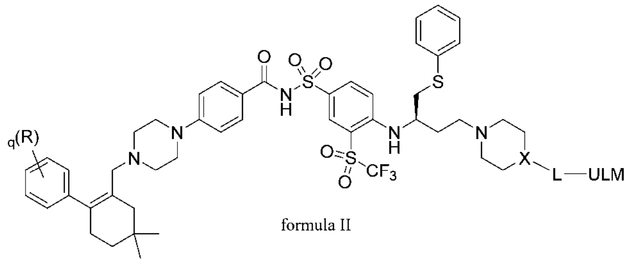
formula II

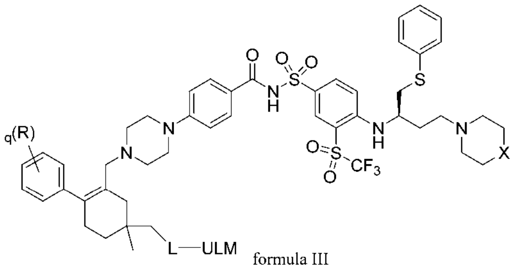
formula III

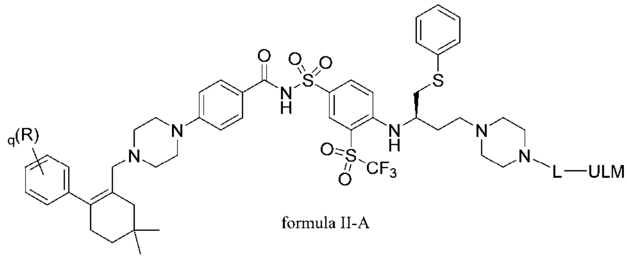
formula II-A

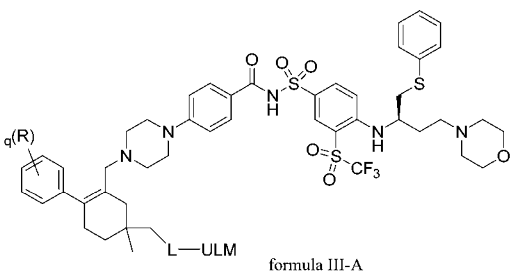
formula III-A

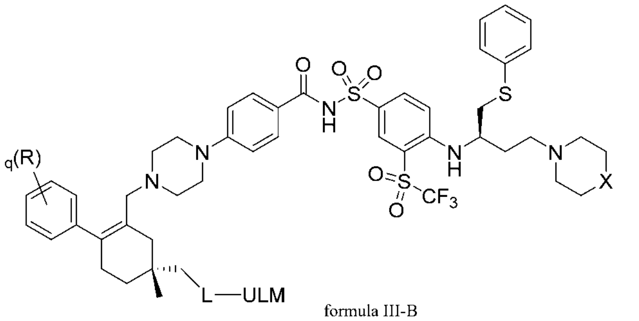

formula III-B

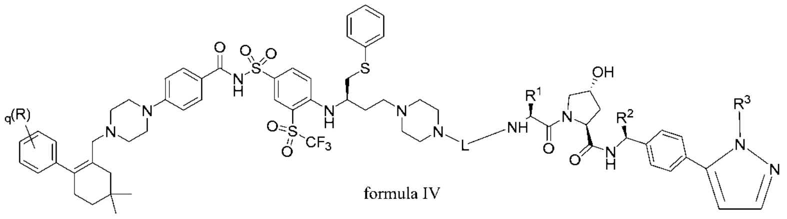

formula IV

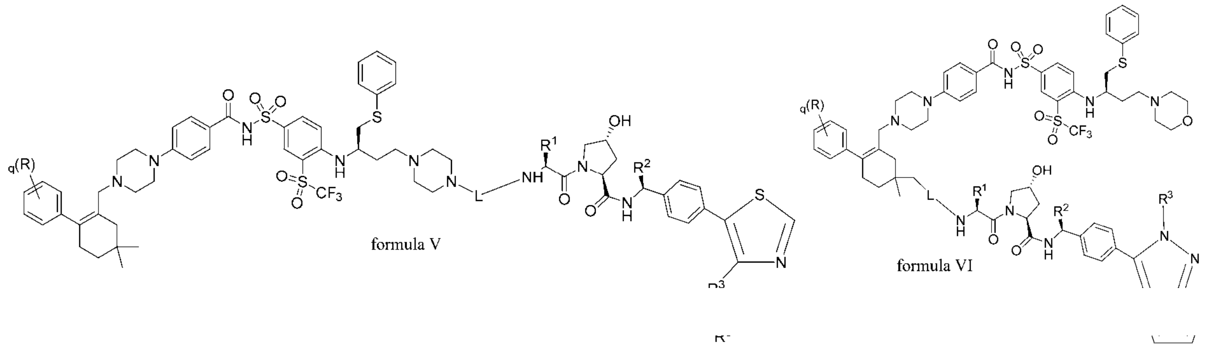

formula V

formula VI

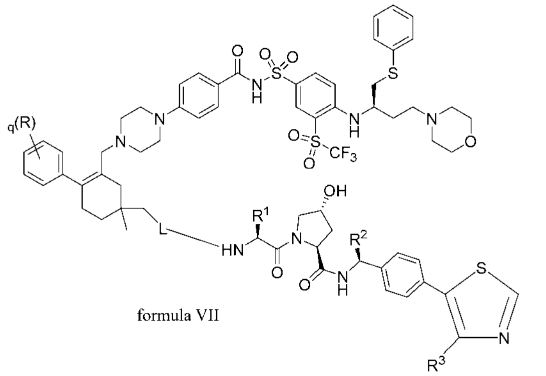

formula VII

wherein R, q, L, ULM, X, $R^1$, $R^2$, or $R^3$ is as defined in any one of claims 1-10.

**12.** A compound of a formula selected from the group consisting of the following formulas, a stereoisomer thereof, or a pharmaceutically acceptable salt thereof:

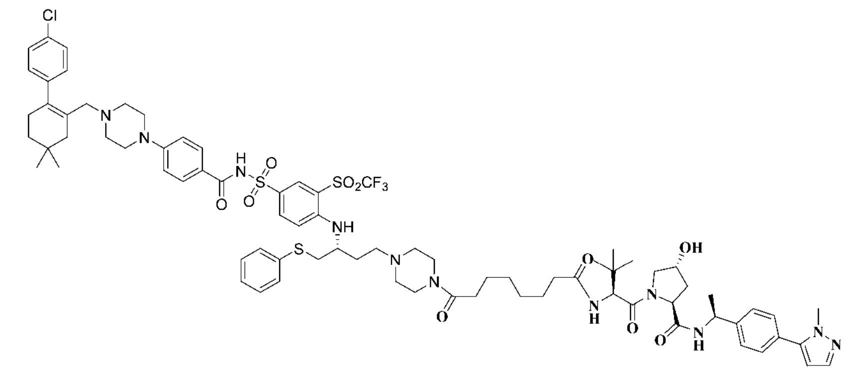

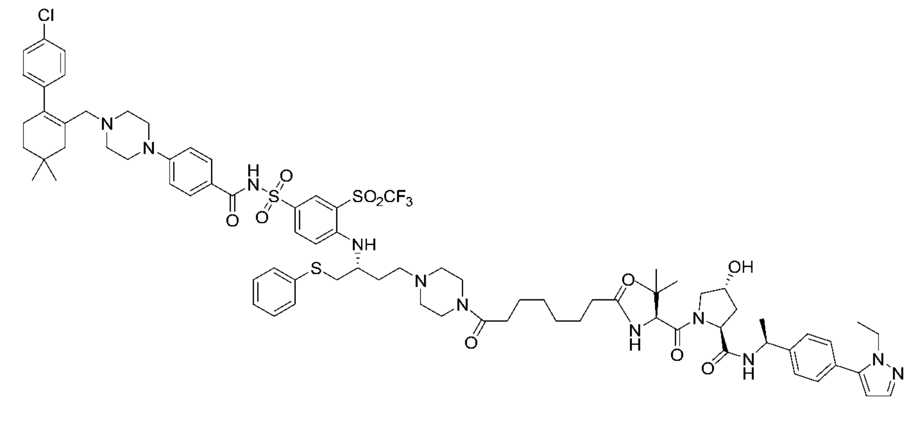

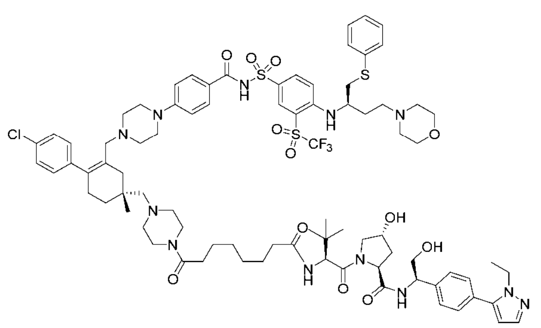

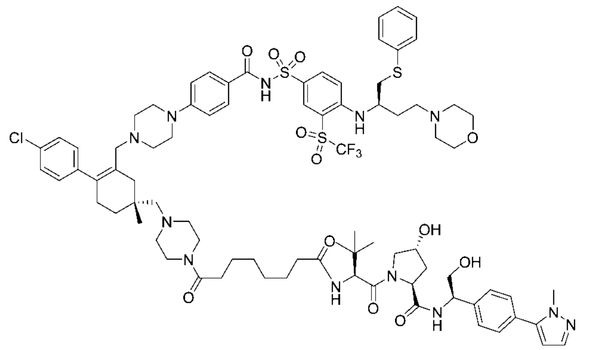

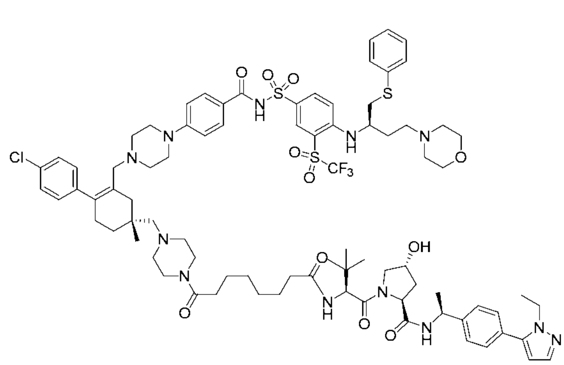

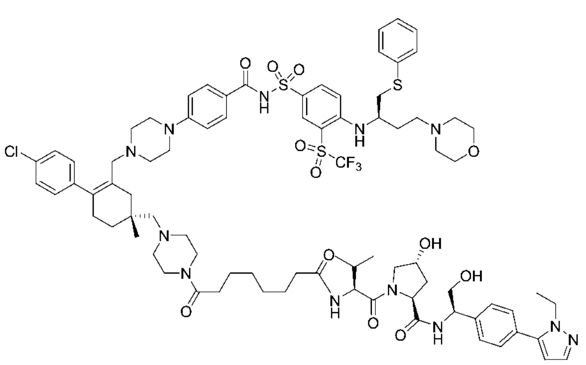

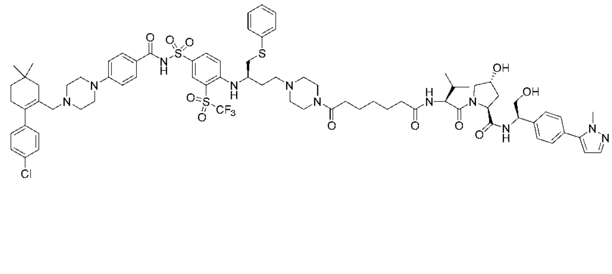

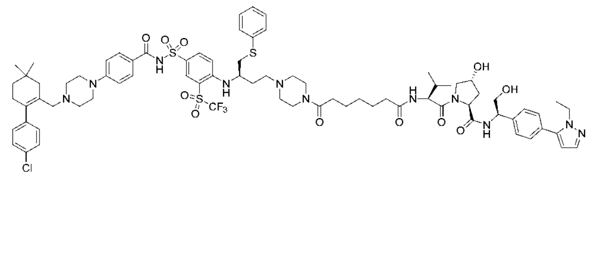

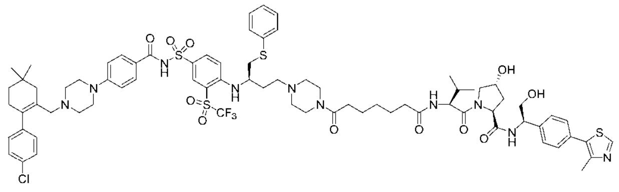

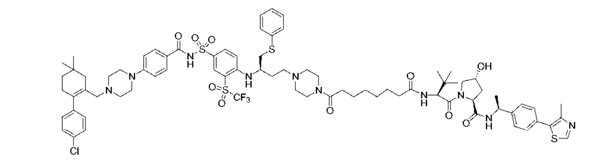

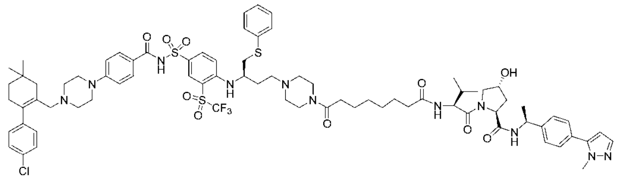

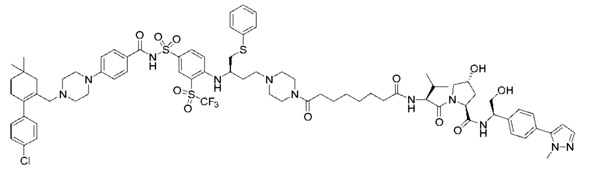

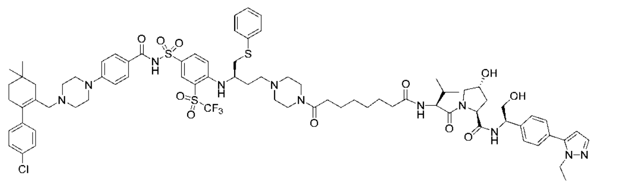

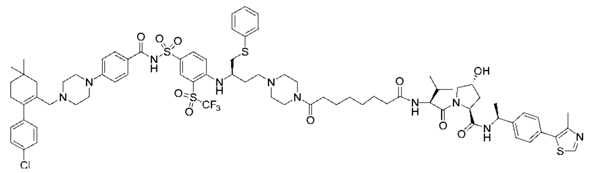

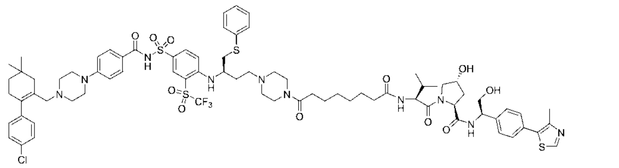

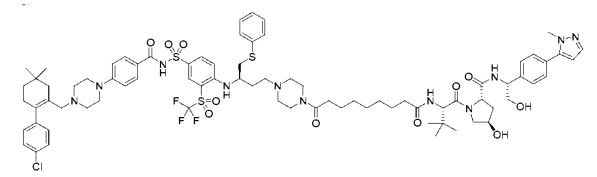

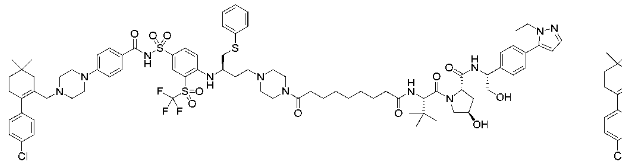

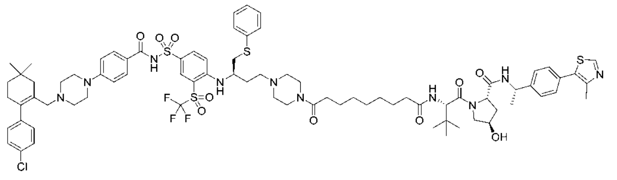

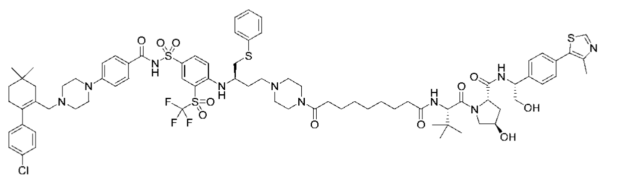

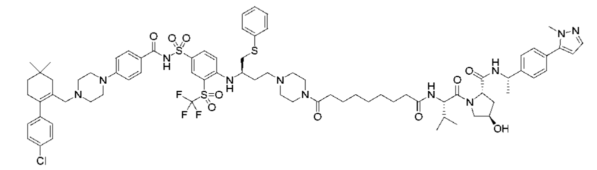

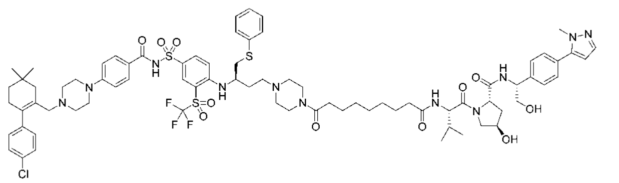

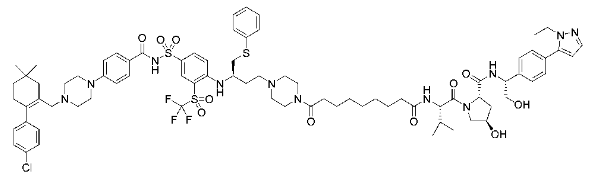

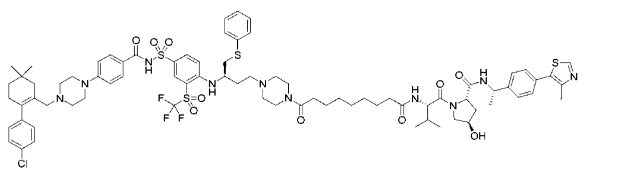

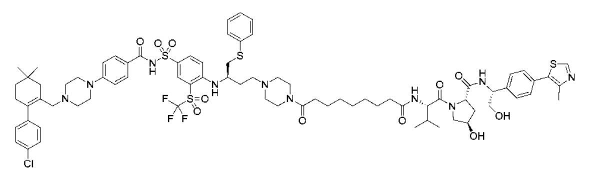

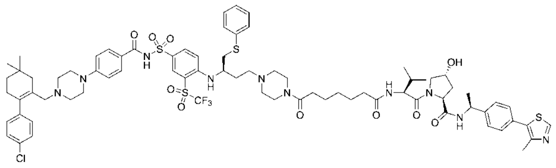

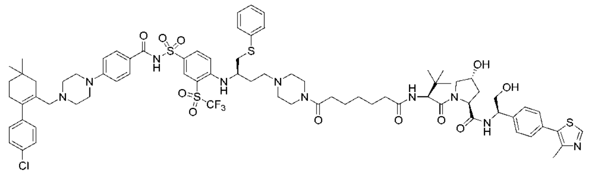

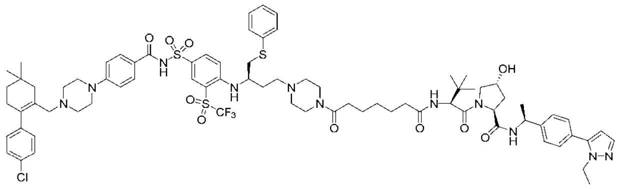

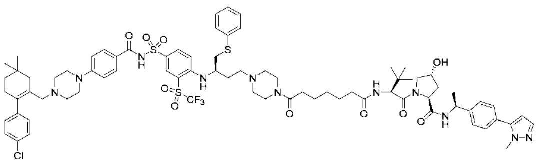

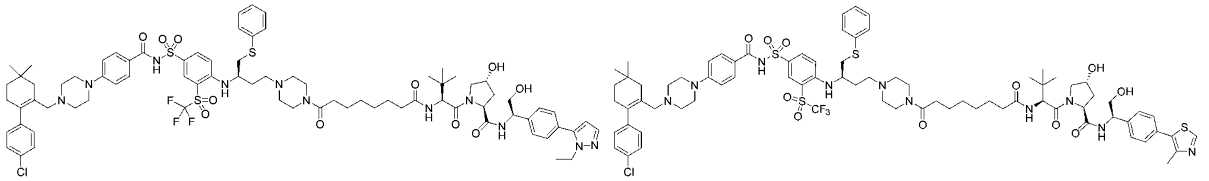

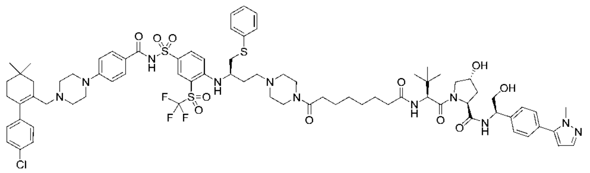

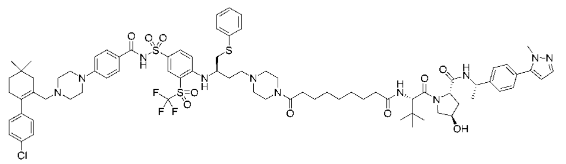

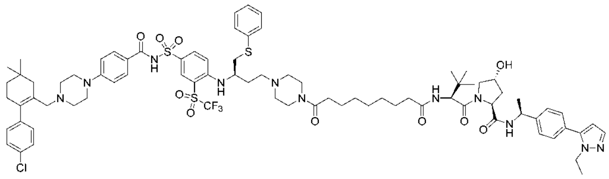

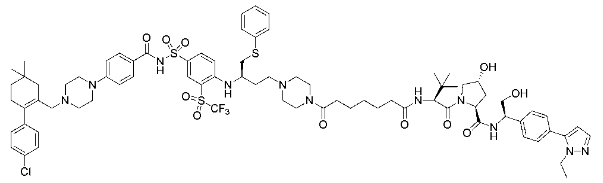

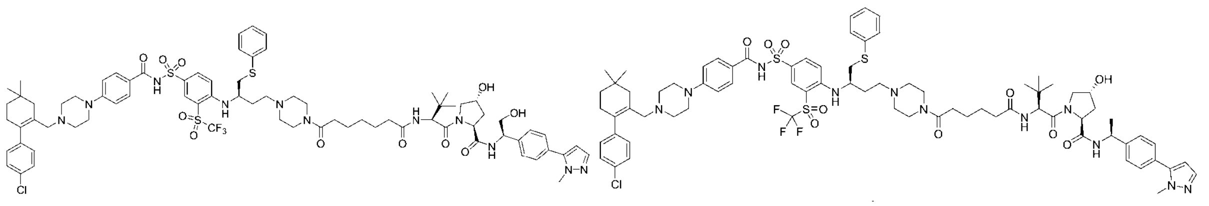

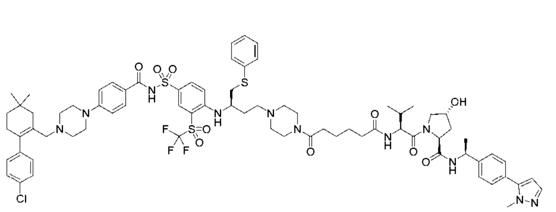

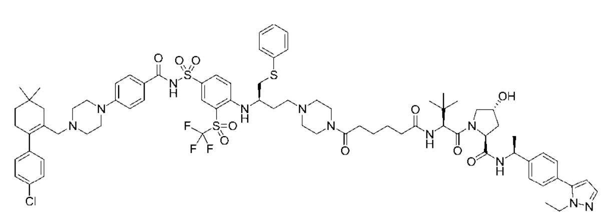

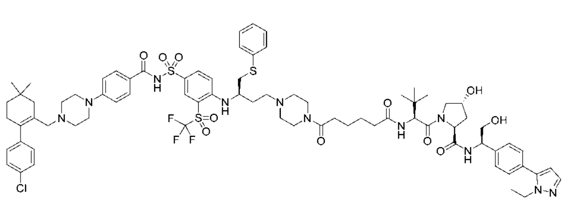

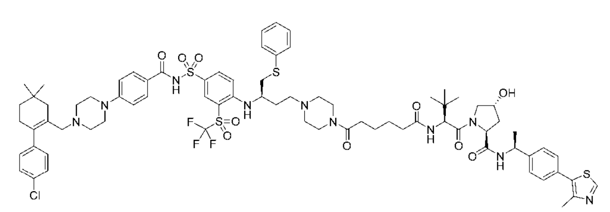

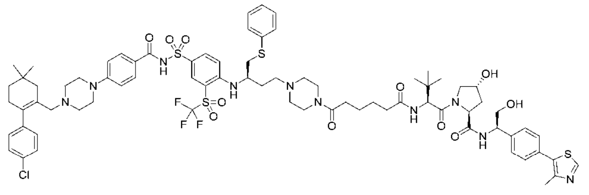

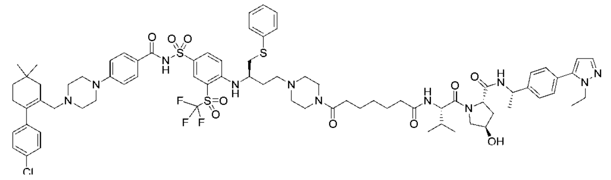

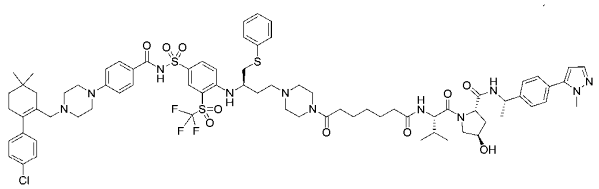

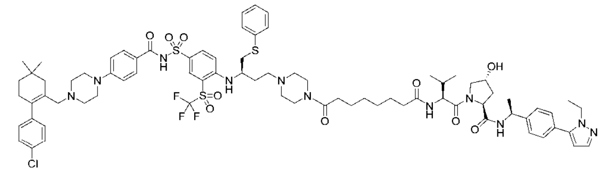

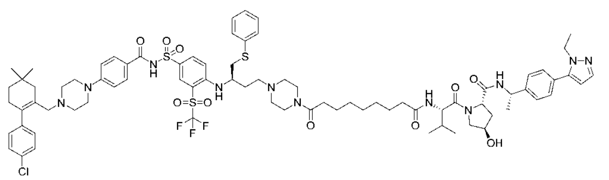

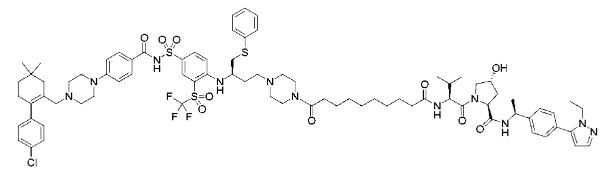

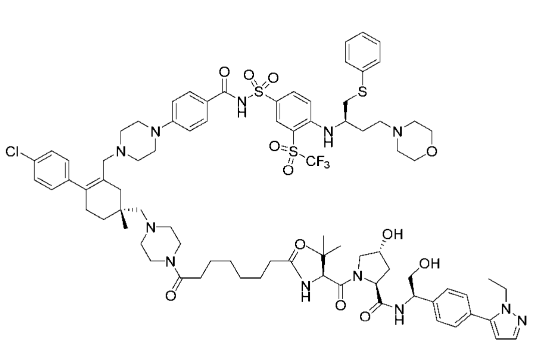

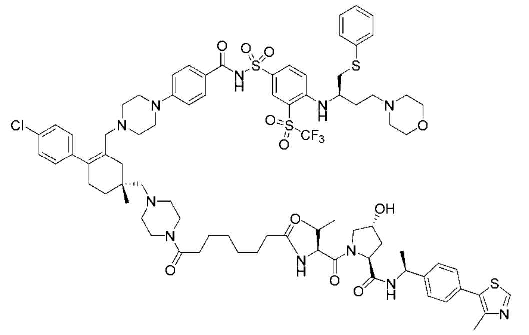

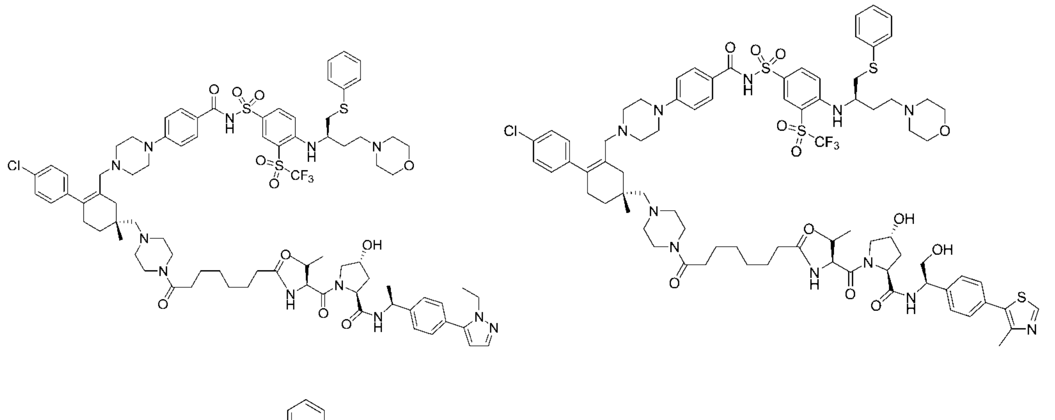

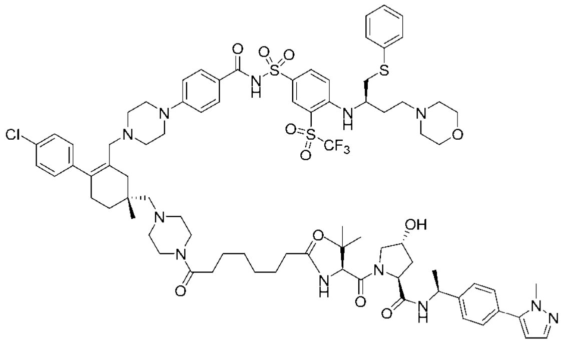

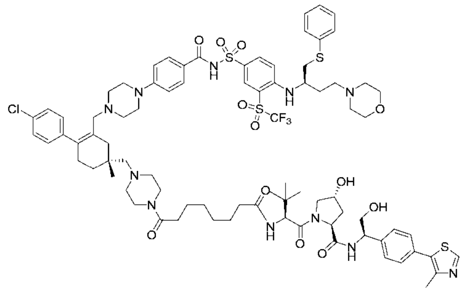 and 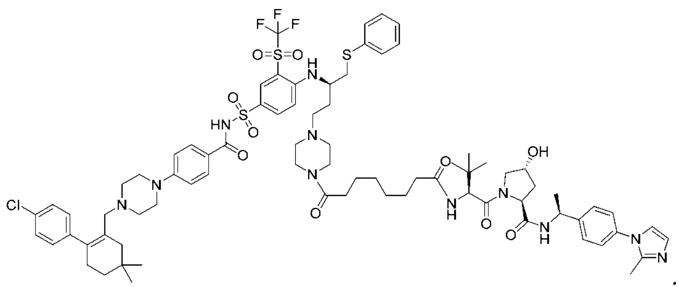.

**13.** A pharmaceutical composition, comprising the compound, the stereoisomer thereof, or the pharmaceutically acceptable salt thereof according to any one of claims 1-12, and optionally further comprising a pharmaceutically acceptable excipient.

**14.** Use of the compound, the stereoisomer thereof, or the pharmaceutically acceptable salt thereof according to any one of claims 1-12 or the pharmaceutical composition according to claim 13 for preventing or treating a disorder treated by degrading a target protein that binds to a targeting ligand.

**15.** Use of the compound, the stereoisomer thereof, or the pharmaceutically acceptable salt thereof according to any one of claims 1-12 or the pharmaceutical composition according to claim 13 for preventing or treating a disorder treated by binding to cereblon protein, wherein
preferably, the disorder treated by binding to cereblon protein is selected from a cancer.

**INTERNATIONAL SEARCH REPORT**

International application No. **PCT/CN2024/077255**

**A. CLASSIFICATION OF SUBJECT MATTER**

C07D487/08(2006.01)i; C07D491/08(2006.01)i; A61K31/395(2006.01)i; A61P35/00 (2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

CNTXT, WPABSC, DWPI, CNKI, ISI Web of Knowledge, STN(REGISTRY, CAPLUS): 正大天晴, 三氟甲磺酰基, 癌症, 肿瘤, B细胞淋巴瘤2蛋白, trifluoromethyl, cancer, tumor, Bcl-2, 结构式检索, structural formula search

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| PX | WO 2024015529 A2 (JAZZ PHARMACEUTICALS IRELAND LTD. et al.) 18 January 2024 (2024-01-18)<br>figure 22C, compound DT2216 | 1-15 |
| PX | WO 2023220425 A1 (KYMERA THERAPEUTICS, INC.) 16 November 2023 (2023-11-16)<br>description, table 1, compounds I-42, I-192, I-193, I-205, I-206, I-207, I-208 and I-210, and claim 22 | 1-15 |
| PX | CN 116490169 A (DIALECTICAL THERAPEUTICS, INC.) 25 July 2023 (2023-07-25)<br>claim 1 | 1-15 |
| X | CN 109152933 A (BIOVENTURES, LLC) 04 January 2019 (2019-01-04)<br>claims 1, 15 and 24, and abstract | 1-15 |
| X | CN 112105360 A (BIOVENTURES, LLC) 18 December 2020 (2020-12-18)<br>abstract, and claims 1, 3 and 15-25 | 1-15 |
| X | CN 113660937 A (UNIVERSITY OF FLORIDA RESEARCH FOUNDATION, INC.) 16 November 2021 (2021-11-16)<br>description, page 70, compound 17, and claims 38-61 | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 April 2024** | **30 April 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No. **PCT/CN2024/077255**

**C. DOCUMENTS CONSIDERED TO BE RELEVANT**

| Category* | Citation of document, with indication, where appropriate, of the relevant passages | Relevant to claim No. |
|---|---|---|
| X | CN 112707900 A (SHANGHAITECH UNIVERSITY et al.) 27 April 2021 (2021-04-27) claims 1 and 35-39, and description, paragraph [0030], and description, page 117, compounds SIAIS363046 and SIAIS364019 | 1-15 |
| X | WO 2017184995 A1 (BIOVENTURES, LLC) 26 October 2017 (2017-10-26) description, pages 3-15 and 33-135 | 1-15 |

Form PCT/ISA/210 (second sheet) (July 2022)

**INTERNATIONAL SEARCH REPORT**
**Information on patent family members**

International application No.
**PCT/CN2024/077255**

| Patent document cited in search report | Publication date (day/month/year) | Patent family member(s) | Publication date (day/month/year) |
|---|---|---|---|
| WO 2024015529 A2 | 18 January 2024 | WO 2024015529 A3 | 07 March 2024 |
| WO 2023220425 A1 | 16 November 2023 | None | |
| CN 116490169 A | 25 July 2023 | KR 20230107314 A | 14 July 2023 |
| | | JP 2023548981 A | 21 November 2023 |
| | | WO 2022103881 A1 | 19 May 2022 |
| | | CA 3196536 A1 | 19 May 2022 |
| | | US 2024009122 A1 | 11 January 2024 |
| | | EP 4243775 A1 | 20 September 2023 |
| | | AU 2021377235 A1 | 08 June 2023 |
| CN 109152933 A | 04 January 2019 | WO 2020081880 A1 | 23 April 2020 |
| | | WO 2017184995 A1 | 26 October 2017 |
| | | KR 20180134908 A | 19 December 2018 |
| | | KR 102447884 B1 | 27 September 2022 |
| | | JP 2019514877 A | 06 June 2019 |
| | | JP 6936498 B2 | 15 September 2021 |
| | | EP 3445452 A1 | 27 February 2019 |
| | | EP 3445452 A4 | 30 October 2019 |
| | | US 2019135801 A1 | 09 May 2019 |
| | | US 10807977 B2 | 20 October 2020 |
| | | AU 2017254687 A1 | 18 October 2018 |
| | | AU 2017254687 B2 | 30 September 2021 |
| | | CA 3018991 A1 | 26 October 2017 |
| | | US 2020331905 A1 | 22 October 2020 |
| | | US 11319316 B2 | 03 May 2022 |
| CN 112105360 A | 18 December 2020 | EP 3743069 A1 | 02 December 2020 |
| | | EP 3743069 A4 | 09 June 2021 |
| | | JP 2021511342 A | 06 May 2021 |
| | | JP 7385284 B2 | 22 November 2023 |
| | | WO 2019144117 A1 | 25 July 2019 |
| | | CA 3088253 A1 | 25 July 2019 |
| CN 113660937 A | 16 November 2021 | WO 2020163823 A2 | 13 August 2020 |
| | | WO 2020163823 A8 | 01 October 2020 |
| | | WO 2020163823 A3 | 29 October 2020 |
| | | KR 20210137025 A | 17 November 2021 |
| | | CA 3127501 A1 | 13 August 2020 |
| | | AU 2020218367 A1 | 12 August 2021 |
| | | JP 2022520061 A | 28 March 2022 |
| | | EP 3920923 A2 | 15 December 2021 |
| | | EP 3920923 A4 | 26 October 2022 |
| | | US 2022169628 A1 | 02 June 2022 |
| CN 112707900 A | 27 April 2021 | WO 2021078301 A1 | 29 April 2021 |
| WO 2017184995 A1 | 26 October 2017 | US 2019135801 A1 | 19 May 2019 |
| | | US 10807977 B2 | 20 October 2020 |
| | | US 2020331905 A1 | 22 October 2020 |
| | | US 11319316 B2 | 03 May 2022 |
| | | WO 2020081880 A1 | 23 April 2020 |
| | | EP 3445452 A1 | 27 February 2019 |
| | | EP 3445452 A4 | 30 October 2019 |
| | | CA 3018991 A1 | 26 October 2017 |

Form PCT/ISA/210 (patent family annex) (July 2022)

**INTERNATIONAL SEARCH REPORT**
**Information on patent family members**

International application No.
**PCT/CN2024/077255**

| Patent document cited in search report | Publication date (day/month/year) | Patent family member(s) | Publication date (day/month/year) |
|---|---|---|---|
| | | AU 2017254687 A1 | 18 October 2018 |
| | | AU 2017254687 B2 | 30 September 2021 |
| | | JP 2019514877 A | 06 June 2019 |
| | | JP 6936498 B2 | 15 September 2021 |
| | | KR 20180134908 A | 19 December 2018 |
| | | KR 102447884 B1 | 27 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310134195
- CN 202410136766


### Non-patent literature cited in the description


- Greene's Protective Groups in Organic Synthesis. John Wiley & Sons, Inc